(19) Europäisches Patentamt
European Patent Office
Office européen des brevets

(11) EP 0 471 612 B1

(12) **FASCICULE DE BREVET EUROPEEN**

(45) Date de publication et mention de la délivrance du brevet:
**28.01.1998 Bulletin 1998/05**

(51) Int. Cl.$^6$: **C07J 41/00**, A61K 31/56, C07J 43/00, C07J 63/00

(21) Numéro de dépôt: **91402214.0**

(22) Date de dépôt: **09.08.1991**

(54) **Nouveaux 19-Nor stéroides ayant en position 11béta une chaîne carbonée comportant une fonction amide, leur préparation, leur application comme médicaments et les compositions pharmaceutiques les renfermant**

Neue 19-Nor Steroide, die eine Amid tragende Gruppe in der 11-Beta-Stelle haben, ihre Herstellung, ihre Verwendung als Medikamente und pharmazeutische Präparate davon

New steroids having an amide substituted group in the 11-beta position, their preparation, their use as medicines and pharmaceutical composition thereof

(84) Etats contractants désignés:
**AT BE CH DE DK ES FR GB GR IT LI LU NL SE**

(30) Priorité: **14.08.1990 FR 9010323**

(43) Date de publication de la demande:
**19.02.1992 Bulletin 1992/08**

(73) Titulaire: **ROUSSEL UCLAF**
**93230 Romainville (FR)**

(72) Inventeurs:
- **Claussner, André**
  **F-93250 Villemomble (FR)**
- **Nique, François**
  **F-93320 Pavillons Sous Bois (FR)**
- **Teutsch, Jean-Georges**
  **F-93500 Pantin (FR)**
- **van de Velde, Patrick**
  **F-75019 Paris (FR)**

(74) Mandataire:
**Vieillefosse, Jean-Claude et al**
**Hoechst Marion Roussel**
**Département des Brevets**
**102, Route de Noisy**
**93235 Romainville Cédex (FR)**

(56) Documents cités:
**EP-A- 384 842**
**EP-A- 0 190 759**
**EP-A- 0 308 345**
**DE-A- 3 621 024**
**FR-A- 2 235 949**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 471 612 B1

Printed by Xerox (UK) Business Services
2.15.8/3.4

## Description

La présente invention concerne de nouveaux 19-Nor stéroïdes ayant en libéta une chaîne carbonée comportant une fonction amide ou thioamide, leur procédé de préparation et les intermédiaires de ce procédé, leur application comme médicaments et les nouvelles compositions pharmaceutiques les renfermant.

La demande de brevet français FR 2 235 949 décrit des dérivés de l'oestrone, ayant éventuellement une chaîne en position 11-béta terminée par une fonction carboxylique, la demande de brevet allemand DE 3621024 concerne des 11béta-phényl-estrène ou -estradiène et la demande de brevet européen EP 0190759 des 11béta-phényl gonanes.

Par ailleurs la demande de brevet européen EP 0308345 décrit des stéroïdes comportant un cycle spirannique en position 17 et la demande de brevet européen EP 0384842 décrit des 19-norstéroïdes ayant en 11béta une chaîne carbonée comportant une fonction amide ou carbamate.

L'invention a pour objet les composés de formule (I) :

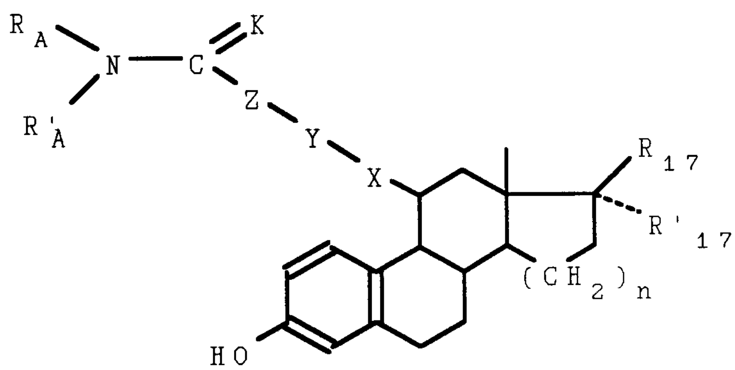

(I)

dans laquelle :
<u>soit</u>

n = 1, K représente un atome d'oxygène,
$R_{17}$ représente un radical hydroxyle ou un radical de formule

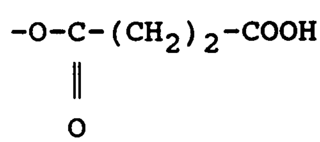

éventuellement salifié,
$R'_{17}$ représente un atome d'hydrogène ou un radical éthynyle,
$R_A$ représente un radical méthyle,
$R'_A$ représente un radical isopropyle ou butyle linéaire,
X représente un radical méthylène, phénylène ou phényloxy lié au stéroïde par un atome de carbone,
Y représente un des radicaux choisis dans le groupe constitué par $-(CH_2)_7$ ; $-(CH_2)_8-$ ; $-(CH_2)_5-C{\equiv}C-$ ; $-(CH_2)q-O-CH_2-$ avec q = 5 a 7 et $-(CH_2)_5-S-CH_2-$ dans lequel l'atome de soufre est éventuellement oxydé sous forme de sulfoxyde ou de sulfone,
dont les noms suivent :

- le monobutanedioate de 11béta-(4-((7-(butylméthylamino) carbonyl) heptyl) oxy) phényl)-3-hydroxy-estra-1,3,5(10)-trien-17béta-yle,
- le butanedioate de 11béta-(4-((7-((butylméthylamino) carbonyl) heptyl) oxy) phényl)-hydroxy-estra-1,3,5(10)-trien-17béta-yle et de sodium,
- le N-butyl-2-(6-(4-(3,17béta-dihydroxy-estra-1,3,5(10)-trien-11béta-yl)phénoxy)hexyloxy-N-méthyl-acétamide,
- le N-butyl-8-(4-(3,17béta-dihydroxy-estra-1,3,5(10)-trien-11béta-yl)phénoxy)-N-méthyl-2-octynamide,
- le N-butyl-2-((5-(4-(3,17béta-dihydroxy-estra-1,3,5(10)-trien-11béta-yl) phényl) pentyl) thio) N-méthyl acétamide,
- le N-butyl-4-(3,17béta-dihydroxy-estra-1,3,5(10)-trien-11béta-yl)-N-méthyl-benzenenonamide,

- le N-butyl-2-((5-(4(3,17béta-dihydroxy-estra-1,3,5(10)-trien-11béta-yl) phenyl) pentyl) oxy)-N-méthyl-acétamide,
- le 2-((8-(3,17béta-dihydroxy 19-nor-17alpha-pregna-1,3,5(10)-trien-20-yn-11béta-yl) octyl) oxy)-N-méthyl-N-(1-méthyléthyl)-acétamide.

Z représente une simple liaison,

soit

n = 1 ou 2,
K représente un atome d'oxygène ou un atome de soufre, $R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O, arylènoxy ou arylènethio lié au stéroïde par un atome de carbone,
- Y représente une simple liaison ou une chaine aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison étant entendu que lorsque Y et Z sont une simple liaison, X ne peut-être un radical méthylène ou $CH_2$-O,

$R_A$ ou $R_{A'}$ identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié ou $R_A$ et $R_{A'}$ forment avec l'atome d'azote auquel ils sont liés un hétérocycle à 5 ou 6 chainons, saturé ou non, renfermant éventuellement un ou plusieurs hétéroatomes choisis parmi le groupe constitué par les atomes d'oxygène, d'azote et de soufre et éventuellement substitué par un radical alkyle ayant de 1 à 4 atomes de carbone, un radical aryle ou aralkyle ayant de 6 à 10 atomes de carbone, étant entendu que l'un au moins des radicaux $R_A$ ou $R_{A'}$ n'est pas un atome d'hydrogène et qu'au moins l'une quelconque des conditions suivantes est remplie :

- soit n = 2,
- soit K est un atome de soufre,
- soit X est un radical arylénethio lié au stéroïde par un atome de carbone,
- soit $R_A$ et $R_{A'}$ forment avec l'atome de carbone auquel ils sont liés un hétérocycle à 5 ou 6 chainons substitué par un radical aryle ou aralkyle.

Parmi les composés de l'invention, on peut citer particulièrement les produits de formule (I) dont la préparation est donnée plus loin dans la partie expérimentale et notamment ceux dont les noms suivent :

- le monobutanedioate de 11béta-(4-((7-(butylméthylamino) carbonyl) heptyl) oxy) phényl)-3-hydroxy-estra-1,3,5(10)-trien-17béta-yle,
- le butanedioate de 11béta-(4-((7-((butylméthylamino) carbonyl) heptyl) oxy) phényl)-3-hydroxy-estra-1,3,5(10)-trien-17béta-yle et de sodium,
- le N-butyl-2-(6-(4-(3,17béta-dihydroxy-estra-1,3,5(10)-trien-11béta-yl)phénoxy)hexyloxy-N-méthyl-acétamide,
- le N-butyl-8-(4-(3,17béta-dihydroxy-estra-1,3,5(10)-trien-11béta-yl) phénoxy)-N-méthyl-2-octynamide,
- le N-butyl-2-((5-(4-(3,17béta-dihydroxy-estra-1,3,5(10)-trièn-11béta-yl) phényl) pentyl) thio) N-méthyl acétamide,
- le N-butyl-4-(3,17béta-dihydroxy-estra-1,3,5(10)-trien-11béta-yl)-N-méthyl-benzenenonamide,
- le N-butyl-2-((5-(4-(3,17béta-dihydroxy-estra-1,3,5(10)-trien-11béta-yl) phényl) pentyl) oxy)-N-méthyl-acétamide,
- le 2-((8-(3,17béta-dihydroxy 19-nor 17alpha-pregna 1,3,5(10)(trièn-20-yn-11béta-yl) octyl) oxy) N-méthyl N-(1-méthyléthyl) acétamide.

Parmi les composés de l'invention, on peut également citer particulièrement les produits répondant à la formule ($I_A$) correspondant à la formule (I) telle que définie ci-dessus dans laquelle :

n = 1 ou 2,
K représente un atome d'oxygène ou un atome de soufre, $R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O, arylènoxy ou arylènethio lié au stéroïde par un atome de carbone,
- Y représente une simple liaison ou une chaine aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison étant entendu que lorsque Y et Z sont une simple liaison, X ne peut-être un radical méthylène ou $CH_2$-O,

$R_A$ ou $R_{A'}$ identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié ou $R_A$ et $R_{A'}$ forment avec l'atome d'azote auquel ils sont liés un hétérocycle à 5 ou 6 chainons, saturé ou non, renfermant éventuellement un ou plusieurs hétéroatomes choisis parmi le groupe constitué par les atomes d'oxygène, d'azote et de soufre et éventuellement substitué par un radical alkyle ayant de 1 à 4 atomes de carbone, un radical aryle ou aralkyle ayant de 6 à 10 atomes de carbone, étant entendu que l'un au moins des radicaux $R_A$ ou $R_{A'}$ n'est pas un atome d'hydrogène et qu'au moins l'une quelconque des conditions suivantes est remplie :

- soit n = 2,
- soit K est un atome de soufre,
- soit X est un radical arylénethio lié au stéroïde par un atome de carbone,
- soit $R_A$ et $R_{A'}$ forment avec l'atome de carbone auquel ils sont liés un hétérocycle à 5 ou 6 chainons substitué par un radical aryle ou aralkyle.

Lorsque $R_{17}$ est un radical acyloxy il peut s'agir notamnotamment du dérivé d'un acide aliphatique ou cycloaliphatique saturé ou insaturé et notamment d'un acide alcanoïque tel que par exemple l'acide acétique, propionique, butyrique ou isobutyrique, valérique ou undécylique, d'un acide hydroxyalcanoïque tel que par exemple l'acide hydroxyacétique ; d'un acide cycloalcoylcarboxylique ou (cycloalcoyle) alcanoïque tel que par exemple l'acide cyclopropyl, cyclopentyl ou cyclohexylcarboxylique, cyclopentyle ou cyclohexyle acétique ou propionique, d'un acide benzoïque, d'un acide salicylique ou d'un acide phénylalcanoïque tel que l'acide phényle acétique ou phényle propionique, d'un amino acide tel que l'acide diéthylamino acétique ou aspartique ou de l'acide formique. Il s'agit de préférence du dérivé de l'acide acétique, propionique, butyrique ou butanedioïque.

Lorsque $R'_{17}$ représente un radical alkyle, il peut s'agir du radical méthyle, éthyle, propyle, isopropyle, butyle, isobutyle, n-pentyle, n-hexyle, 2-méthyl pentyle, 2,3-diméthyl butyle, n-heptyle, 2-méthylexyle, 2,2-diméthylpentyle, 3,3-diméthyl pentyle, 3-éthylpentyle, n-octyle, 2,2-diméthylhexyle, 3,3-diméthylhexyle, 3-méthyl 3-éthylpentyle.

Il s'agit de préférence du radical méthyle.

Lorsque $R'_{17}$ représente un radical alcényle, il peut s'agir d'un radical vinyle, propényle, isopropényle, allyle, 2-méthylallyle, butényle ou isobutényle. Il s'agit de préférence du radical vinyle ou propényle.

Lorsque $R'_{17}$ représente un radical alcynyle, il peut s'agir du radical éthynyle, propynyle, propargyle, butynyle ou isobutynyle. Il s'agit de préférence du radical éthynyle ou propynyle.

L'expression éventuellement substitué appliquée aux radicaux alkyle, alcényle ou alcynyle comprend un ou plusieurs radicaux identiques ou différents choisis de préférence parmi les radicaux :

- halogène tel que fluor, chlore, brome, iode,
- alkoxy tel que méthoxy, éthoxy, propyloxy, isopropyloxy, butyloxy,
- alkylthio tel que méthylthio, éthylthio, propylthio, isopropylthio, butylthio,
- amino, alkylamino tel que méthylamino ou éthylamino, dialkylamino tel que diméthylamino, diéthylamino ou méthyl

éthylamino, chacun des radicaux dialkylamino étant éventuellement sous forme oxydée,

- amino alkyle tel que aminométhyle ou aminoéthyle,
- dialkylaminoalkyle tel que diméthylamino méthyle ou éthyle,
- dialkylaminoalkyloxy tel que diméthylamino éthyloxy,
- hydroxyle éventuellement acylé, par exemple acétoxy ou un radical de formule

$$-O-\underset{\underset{O}{\|}}{C}-(CH_2)_n\ CO_2H$$

dans lequel n = 2 à 5 notamment,

- acyle tel que acétyle, propionyle, butyryle, benzoyle,
- carboxy libre ou estérifié tel que alkoxy carbonyle par exemple méthoxy carbonyle ou éthoxy carbonyle,
- cyano,
- trifluorométhyle,
- aryle tel que phényle, furyle, thiényle ou aralkyle tel que benzyle, ces radicaux étant eux-mêmes éventuellement substitués par des radicaux alkyle, tel que méthyle, éthyle, propyle, isopropyle, butyle, isobutyle, tert-butyle ou par des radicaux, alkoxy, alkylthio, alkylamino ou di-alkylamino indiqués ci-dessus.

Lorsque X représente un groupement arylène, il s'agit de préférence du radical phénylène.

Lorsque X représente un groupement arylènoxy, il s'agit de préférence du radical phénylènoxy.

Lorsque X représente un radical arylènethio, il s'agit de préférence du radical phénylènethio.

Lorsque Y représente une chaîne aliphatique linéaire ou ramifiée, saturée ou insaturée, il peut s'agir du radical méthylène, éthylène, propylène, isopropylène, butylène, isobutylène, ou tert-butylène, n-pentylène, n-hexylène, 2-méthyl pentylène, 2,3-diméthyl butylène, n-heptylène, 2-méthylhexylène, 2,2-diméthylpentylène, 3,3-diméthylpentylène, 3-éthylpentylène, n-octylène, 2,2-diméthylhexylène, 3,3-diméthylhexylène, 3-méthyl 3-éthylpentylène, nonylène, 2,4-diméthyl heptylène, n-décylène, n-undécylène, n-dodécylène, n-tridécylène, n-tétradécylène, n-penta décylène, n-hexadécylène, n-heptadécylène ou n-octadécylène, de préférence n-nonylène ou n-décylène. Il peut s'agir également des radicaux vinylène, isopropénylène, allylène, 2-méthylallylène ou isobuténylène, et lorsque la chaîne est interrompue ou terminée par un ou plusieurs radicaux arylènes, il s'agit de préférence du radical phénylène, étant entendu que terminée concerne l'une quelconque des deux extrémités de Y.

Lorsque RA ou RA'représente un radical alkyle, il peut s'agir du radical méthyle, éthyle, propyle, isopropyle, butyle, isobutyle ou tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl 3-éthylpentyle.

Les radicaux précités peuvent être substitués par un ou plusieurs radicaux aryles tel que phényle, furyle, thiènyle, de préférence un radical phényle, par un ou plusieurs radicaux alkylamino ou dialkylamino tel que diméthylamino ou par un ou plusieurs carboxyles estérifiés par exemple par un méthoxycarbonyle ou un éthoxycarbonyle, par un ou plusieurs atomes d'halogène par exemple le fluor, le chlore ou le brome.

On peut citer notamment le radical 2,2,3,3,4,4,4 heptafluorobutyle.

Lorsque RA et RA' forment avec l'atome d'azote auquel ils sont liés un hétérocycle à 5 ou 6 chainons, il s'agit d'un hétérocycle saturé, de préférence une pyrrolidine ou une pipéridine éventuellement substitué par un radical alkyle tel que méthyle, éthyle, propyle ou isopropyle, de préférence méthyle ou éthyle, par un radical aryle tel que phényle ou tolyle ou par un radical alkylaryle tel que benzyle ou phényléthyle, de préférence benzyle ou d'un hétérocycle insaturé, de préférence un pyrrole ou une pyridine éventuellement substitué par un radical alkyle tel que méthyle, renfermant éventuellement un autre hétéroatome, de préférence une morpholine, une pipérazine ou une pyrimidine, éventuellement substitué par un radical alkyle, de préférence méthyle ou éthyle.

La présente demande concerne plus particulièrement les produits de formule ($I_A$) pour lesquels n est égal à 1.

Parmi les composés préférés de l'invention on peut citer spécialement les produits de formule ($I_A$) pour lesquels K est un atome de soufre, X est un radical arylènoxy et Y représente une chaine linéaire saturée renfermant de 5 à 10 atomes de carbone, éventuellement interrompue par un atome d'oxygène, ceux pour lesquels X est un radical arylènethio et Y représente une chaîne linéaire saturée renfermant de 5 à 10 atomes de carbone et ceux pour lesquels X est un radical arylène, Y représente une chaine linéaire insaturée renfermant de 5 à 10 atomes de carbone, $R_A$ et $R_{A'}$ forment avec l'atome auquel ils sont liés une pipéridine N-substituée par un radical aralkyle.

Parmi les composés préférés de l'invention on peut donc naturellement citer les produits de formule (I) dont la préparation est donnée plus loin dans la partie expérimentale et plus particulièrement :

- le N-butyl-5-(4-(3,17béta-dihydroxy-estra-1,3,5(10)-trien-11béta-yl) phénoxy) pentyloxy)-N-méthyl-éthanethioamide,

- le N-butyl-8-(4-(3,17béta-dihydroxy-estra-1,3,5(10)-trien-11béta-yl) phénoxy)-N-méthyl-octanethioamide.

La présente demande a également pour objet un procédé de préparation des produits de formule (I) telle que définie ci-dessus, caractérisé en ce que l'on soumet un composé de formule (II) :

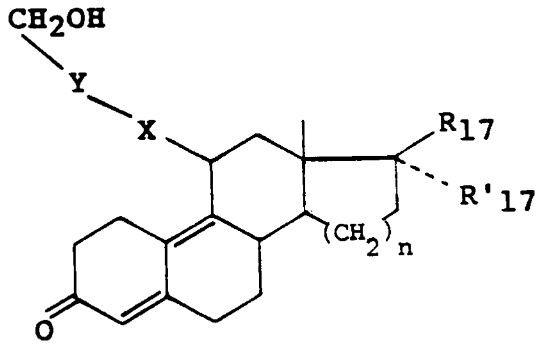

(II)

dans laquelle X, Y, n, $R_{17}$ et $R'_{17}$ ont la même signification que précédemment, étant entendu que lorsque $R_{17}$ représente un radical hydroxyle, ce radical est protégé, à l'action d'un agent d'oxydation pour obtenir le produit de formule (III) :

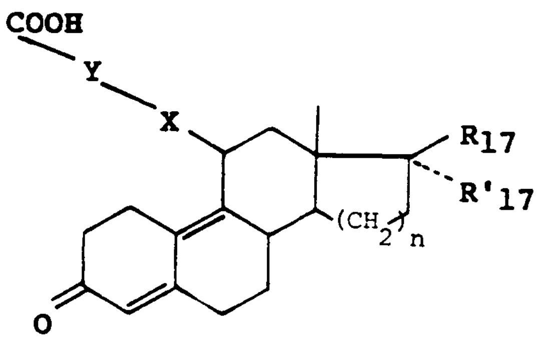

(III)

que l'on soumet à l'action d'un agent permettant d'activer la fonction carboxylique, puis à l'action d'un composé de formule (IV) :

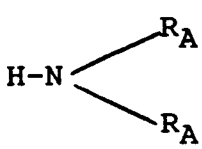

(IV)

dans laquelle $R_A$ et $R_A'$ ont la même signification que précédemment, pour obtenir le produit de formule (V) :

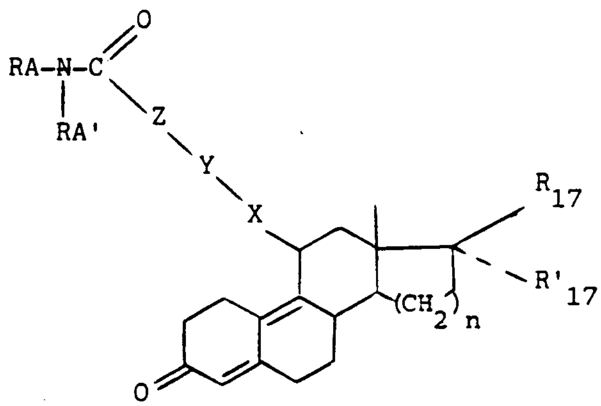

(V)

dans laquelle Z est une simple liaison, produits (V) que, si nécessaire, l'on soumet à une réaction de déprotection de la fonction hydroxy et, si désiré,

- à un agent de réduction lorsque $R_{17}$ et $R'_{17}$ forment ensemble une fonction cétone, puis le cas échéant soumet à un agent d'acylation le dérivé hydroxylé en 17 ainsi obtenu,
- ou à un agent de saponification lorsque $R_{17}$ représente une fonction acyloxy,

composé de formule (V) que l'on soumet à un agent d'aromatisation du cycle A, puis à un agent de saponification ménagée pour obtenir les produits de formule (I) que, si désiré, l'on soumet à une ou à plusieurs des réactions suivantes :

soit, lorsque $R_{17}$ et $R'_{17}$ forment ensemble une fonction cétone, l'on soumet à un agent de réduction ou à un complexe métallique de formule (VI) :

$$M\text{-}R'_{17} \quad \text{(VI)}$$

dans laquelle M représente un atome métallique et $R'_{17}$ a la même signification que précédemment, à l'exception d'hydrogène,
soit, lorsque $R_{17}$ est un radical hydroxyle, l'on soumet à un agent d'acylation sélective en position 17,
soit, lorsque $R_A$ ou $R_A'$ est un atome d'hydrogène, on soumet à un agent d'alkylation approprié,
soit, lorsque Y représente une chaine aliphatique insaturée, l'on soumet à un agent d'hydrogénation,
pour obtenir les produits de formule (I) dans laquelle K est un atome d'oxygène que, si désiré, l'on soumet à un agent de sulfuration pour obtenir les produits de formule (I) dans laquelle K est un atome de soufre.

Les composés de formule (V) sont obtenus en faisant réagir un composé de formule (III) activé par exemple sous forme d'anhydride mixte par action d'un agent tel qu'un chloroformiate, par exemple le chloroformiate d'isobutyle, en présence d'une base telle qu'une amine tertiaire, par exemple la N-méthylmorpholine, dans un solvant anhydre tel qu'un solvant chloré, par exemple le chlorure de méthylène, avec l'amine de formule (IV).

Le produit de formule (III) est obtenu à l'aide d'un agent d'oxydation tel que, par exemple, le mélange $CrO_3$ - acide sulfurique dans un solvant neutre tel que par exemple, l'acétone.

Dans un mode de réalisation préféré de l'invention, les composés de formule (II) comprennent une chaîne en 11béta terminée par une fonction alcool choisie dans le tableau suivant :

| X | Y | |
|---|---|---|
| $\Phi$ | $-(CH_2)_7$ | $-CH_2OH$ |
| $\Phi$ | $-(CH_2)_5-S-CH_2$ | $-CH_2OH$ |
| $\Phi$ | $-C\equiv C-(CH_2)_5$ | $-CH_2OH$ |
| $\Phi$ O | $-(CH_2)_7$ | $-CH_2OH$ |
| $\Phi$ O | $-(CH_2)_5-O-CH_2$ | $-CH_2OH$ |
| $\Phi$ O | $-(CH_2)_5-SO-CH_2$ | $-CH_2OH$ |

Comme illustré dans les exemples ci-après, les composés de formule (III) comprennent une chaîne en 11béta, terminée par une fonction carboxylique, correspondant au produit d'oxydation d'une chaîne choisie parmi les chaînes en 11béta terminée par une des fonctions alcool citées ci-dessus, le composé de formule (IV) est choisi parmi les amines telles que par exemple la butylamine, la méthylbutylamine, la méthylisopropylamine ou la benzylpipéridine qui sont des produits connus, ou la méthyl hepta-fluorobutylamine dont la préparation est donnée plus loin.

Lorsque le composé de formule (V) a une fonction cétone en 17, on obtient le stéroïde hydroxylé en 17béta correspondant, par action d'un agent de réduction tel que le borohydrure de sodium dans un solvant neutre tel que le méthanol ou l'hydrure de triterbutoxylithium aluminium dans le tétrahydrofuranne.

Lorsque le composé de formule (V) a une fonction hydroxy en 17, on obtient le stéroïde acyloxylé en 17béta correspondant, par action d'un agent d'acylation, par exemple d'acétylation tel que l'anhydride acétique dans la pyridine, en présence éventuellement de 4-diméthylaminopyridine.

Lorsque le composé de formule (V) a une fonction acyloxy en 17, on obtient le stéroïde hydroxylé en 17béta correspondant, par action d'un agent de saponification, tel que la potasse en milieu alcoolique.

Les composés de formule (I) qui sont des stéroïdes dérivés de l'oestradiol ayant une chaîne en 11béta comportant une fonction amide substituée sont obtenus à partir des composés de formule (V) par action d'un agent d'aromatisation tel que l'hydroxyde de palladium sur magnésie au sein du méthanol ou bien le mélange bromure d'acétyle-anhydride acétique, à une température ne dépassant pas la température ambiante, suivie d'une réaction de saponification ménagée avec par exemple de la potasse dans le méthanol, du bicarbonate de sodium ou bien du méthanol en présence d'acide chlorhydrique.

Lorsque le composé de formule (I) a une fonction cétone en 17, on obtient :

- le stéroïde 17béta hydroxylé correspondant, dans les conditions décrites ci-dessus, pour le composé de formule (V) par exemple par action d'un agent de réduction tel que le borohydrure de sodium dans un solvant neutre tel que le méthanol,
- le composé de formule (I) correspondant comportant un radical $R'_{17}$ que représente un radical alkyle, alcényle ou alcynyle, éventuellement substitué, en utilisant comme complexe, par exemple, un complexe du lithium, selon le procédé décrit dans la demande de brevet EP 57115.

Lorsque le composé de formule (I) a une fonction hydroxy en 17, on obtient le stéroïde 17béta acyloxylé correspondant, par action d'un agent d'acylation sélective par exemple l'anhydride acétique dans la pyridine.

Lorsque $R_A$ ou $R_{A'}$ est un atome d'hydrogène, on obtient le produit alkylé correspondant, par action d'un halogénure d'alkyle, par exemple l'iodure de méthyle ou d'éthyle, le bromure de méthyle ou d'éthyle dans un solvant tel que le tétrahydrofuranne.

Il est bien entendu que si $R'_{17}$ comprend un radical alkyle, alcényle ou alcynyle substitué par une fonction réactive, celle-ci peut être provisoirement protégée par les méthodes usuelles.

Lorsque Y est une chaîne aliphatique insaturée on obtient le produit de formule (I) de réduction de la triple liaison correspondant soit à l'aide d'hydrogène en présence de palladium sur charbon actif, sulfate de baryum et éventuellement une base telle que la pyridine ou la quinoléine, dans le cas d'une réduction partielle, soit à l'aide d'hydroxyde de palladium seul ou de chlorotris (triphényl-phosphine) rhodium dans le cas d'une réduction totale.

Les produits de formule (I) dans laquelle Y représente une chaîne aliphatique ramifiée peuvent être préparés par alkylation des produits de formule (I) dans laquelle Y représente une chaîne aliphatique linéaire après avoir, le cas échéant, bloqué les fonctions réactives en 3 et en 17. L'alkylation est effectuée par exemple à l'aide d'un halogénure d'alkyle tel que l'iodure de méthyle, en présence de lithium diisopropylamide.

Lorsque K est un atome d'oxygène, on obtient le produit de formule (I) correspondant dans lequel K est un atome

de soufre, par action d'un agent de sulfuration, par exemple le [2,4-bis(4-méthyoxyphényl)-1,3-dithia-2,4-diphosphénate-2,4-disulfure] (réactif de Lawesson), après blocage éventuel des fonctions réactives en 3 et 17.

L'invention concerne également un procédé de préparation des produits de formule (I') correspondant aux produits de formule (I) dans laquelle X représente un radical arylène et Y représente une chaîne aliphatique éventuellement liée au groupe arylène par une double ou une triple liaison et comportant au moins 3 atomes de carbone ou liée au groupe arylène par un atome d'oxygène ou un atome de soufre, caractérisé en ce que l'on soumet un composé de formule (VII) :

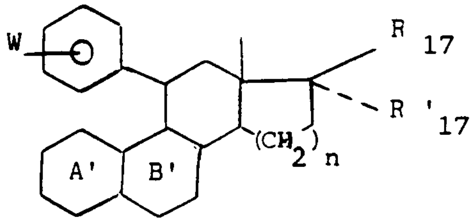

(VII)

dans laquelle W représente soit un radical -OH, soit un radiradical mercapto éventuellement activé, soit un radical -C≡CH, n, $R_{17}$ et R′ ayant la même signification que celle indiquée précédemment, étant entendu que lorsque $R_{17}$ représente un radical -OH, il est éventuellement protégé, les cycles A′ et B′ sont :

- soit

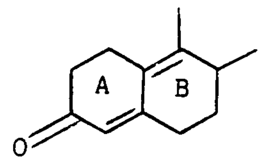

- soit

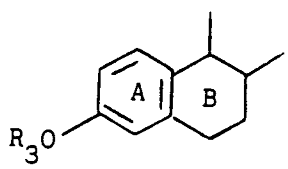

où $R_3$ représente un atome d'hydrogène ou un groupement protecteur de la fonction hydroxyle,

**ou bien**, dans le cas où W représente un radical C≡CH à l'action d'un dérivé halogéné de formule (VIII) :

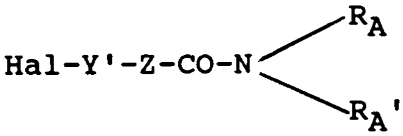

(VIII)

dans laquelle Hal est un atome d'halogène, Z, $R_A$ et $R_A$′ ont la même signification que celle indiquée précédemment et Y′ représente la chaine aliphatique Y ci-dessus comportant 2 atomes de carbone en moins, en présence d'une base forte et soumet le cas échéant à l'action d'un agent de déprotection en 3 et/ou en 17, pour obtenir le produit de formule ($IX_A$) :

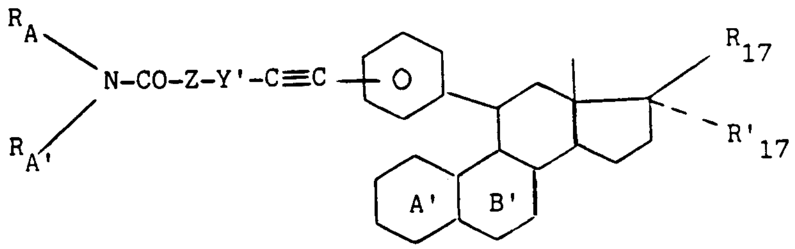

(IXA)

produit que, si désiré, l'on soumet à un agent de réduction partielle ou totale de la triple liaison pour obtenir le produit de formule ($IX_B$) :

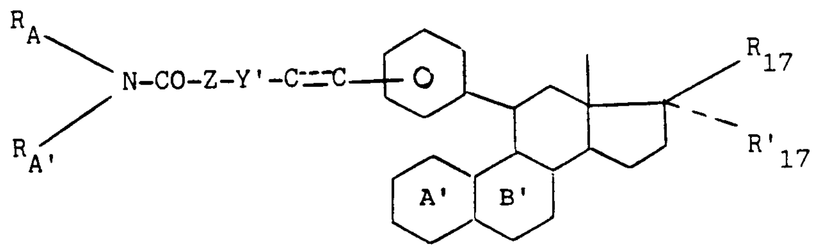

(IXB)

**ou bien**, dans le cas où W représente un radical -OH, un radical mercapto ou un radical mercapto activé, à l'action d'un dérivé halogéné de formule (X) :

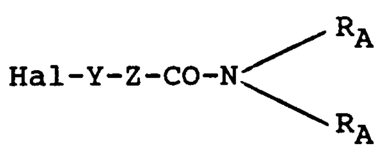

(X)

dans laquelle Hal, Y, Z, $R_A$ et $R_A'$ ont la même signification que celle indiquée précédemment, en présence d'un agent alcalin, puis le cas échéant, à l'action d'un agent de déprotection pour obtenir un produit de formule ($IX_C$) :

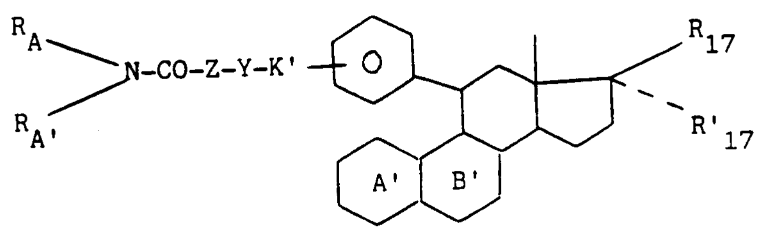

(IXC)

dans laquelle K' représente soit un atome d'oxygène, soit un atome de soufre, produits de formule ($IX_A$), ($IX_B$) ou ($IX_C$) que le cas échéant l'on traite comme indiqué précédemment pour les produits de formule (V), pour obtenir les produits de formule (I') que le cas échéant, l'on traite comme indiqué précédemment pour les produits de formule (I).

Lorsque W représente un dérivé activé du radical mercapto, il peut s'agir d'un thiolate de métal, par exemple l'argent.

Selon un mode préféré du procédé de l'invention décrit ci-dessus :

- les groupements de protection éventuels des fonctions hydroxyles en 3 et en 17 sont choisis parmi les groupements usuels tels que tétrahydropyrannyle et tert-butyle,

- l'atome d'halogène que représente Hal est par exemple un atome de brome, de chlore ou d'iode,
- la base forte utilisée est par exemple le butyllithium ou l'hydrure de sodium,
- la réduction éventuelle de la triple liaison est effectuée soit à l'aide d'hydrogène en présence de palladium sur charbon actif, sulfate de baryum et éventuellement une base telle que la pyridine ou la quinoléine, dans le cas d'une réduction partielle, soit à l'aide d'hydroxyde de palladium seul ou de chlorotris (triphénylphosphine) rhodium dans le cas d'une réduction totale,
- l'activation éventuelle du radical mercapto est effectuée par action d'un sel métallique, par exemple le nitrate d'argent, sur un mercapto protégé lequel peut être par exemple un thioéther tel qu'un dérivé alkylthio, par exemple tert-butylthio, triphényl-méthylthio, isobutyloxyméthylthio ou un dérivé d'un cycle à 5 ou 6 chaînons contenant éventuellement un hétéroatome appartenant au groupe de l'azote, l'oxygène ou le soufre, de préférence l'oxygène. Comme exemple de radical mercapto protégé, on peut citer notamment le radical tétrahydropyrranylthio.
- l'agent alcalin utilisé est un hydroxyde alcalin par exemple la soude,
- le déblocage des fonctions protégées est effectué à l'aide d'un agent d'hydrolyse usuel tel que l'acide chlorhydrique,
- dans les composés de formule (VII) utilisé au départ, le radical W est en position para.

Les composés de formule (I) présentent d'intéressantes propriétés pharmacologiques. L'étude des produits sur les récepteurs hormonaux a permis de mettre en évidence qu'ils possèdent en particulier une remarquable activité anti-oestrogène et des propriétés anti-prolifératives, comme le montrent les résultats des tests donnés plus loin.

Ces propriétés rendent les composés de formule (I) utilisables dans le traitement de carcinomes hormono-dépendants, comme par exemple les carcinomes mammaires et ses métastases et dans le traitement des tumeurs bénignes du sein.

L'invention a donc pour objet les produits de formule (I) à titre de médicaments.

Parmi les médicaments de l'invention on peut citer particulièrement les composés décrits dans la partie expérimentale et tout particulièrement les produits des exemples 1, 2, 3, 4, 6, 7, 9, 10, 11 et 14.

La posologie utile varie en fonction de l'affection à traiter et de la voie d'administration ; elle peut varier par exemple de 1 à 100 mg par jour chez l'adulte par voie orale.

L'invention s'étend aux compositions pharmaceutiques renfermant comme principe actif au moins l'un des médicaments tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains des intermédiaires de formule (II) ou (III) sont des produits nouveaux et l'invention a donc également pour objet les produits de formule (II) et (III) telles que définies précédemment, dans laquelle :

- soit n = 2.

Les composés nouveaux de formule (II) tels que définis précédemment sont préparés selon un mode opératoire dont un exemple est donné plus loin. D'une manière générale, les composés de formule (II) peuvent être préparés selon le procédé suivant :

On fait réagir en présence d'un sel de cuivre, le dérivé magnésien d'un alcool halogéné de formule (XI) :

$$hal\text{-}X\text{-}Y\text{-}CH_2OR \qquad (XI)$$

dans laquelle X et Y ont la signification donnée précédemment, hal est un atome d'halogène, de préférence un atome de chlore ou de brome et R est soit un atome d'hydrogène soit un groupement protecteur de la fonction alcool par exemple un groupement :

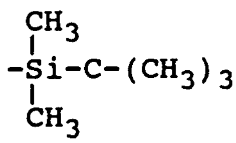

sur un composé de formule (XII) :

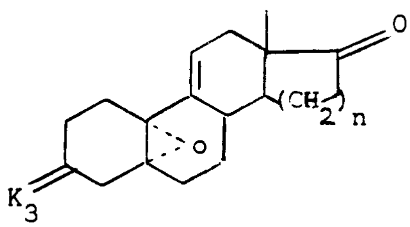

(XII)

dans laquelle n a la signification donnée précédemment, $K_3$ est un groupement protecteur de la fonction cétone en 3 tel qu'un cétal cyclique, pour obtenir le produit de formule (XIII) :

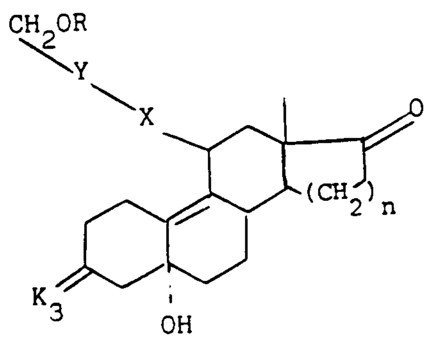

(XIII)

que l'on soumet, si désiré,

- soit au dérivé lithien du produit de formule (XIV) :

$$H\text{-}R'_{17} \qquad (XIV)$$

  dans laquelle $R'_{17}$ a la signification donnée précédemment, étant entendu qu'il ne s'agit pas d'un atome d'hydrogène
- soit à un agent de réduction, puis éventuellement à un agent d'acylation, et que l'on soumet à un agent de déshydratation et d'hydrolyse susceptible de libérer la fonction 3-céto delta-4 et la fonction alcool pour obtenir le composé de formule (II).

Les produits de formule (XII) nécessaires à la mise en oeuvre du procédé sont des produits connus. Leur préparation est décrite par exemple dans le brevet EP 0057115 lorsque n = 1 et dans la demande européen EP 0116974 lorsque n = 2.

Des produits de formule (VII) nécessaires à la mise en oeuvre du procédé, dans lesquels n = 1 et W est différent d'un radical mercapto, sont décrits notamment dans la demande de brevet européen EP 0245170. Les produits dans lesquels n = 2 et W est différent d'un radical mercapto sont obtenus par le même procédé en partant du produit (XII) dans lequel n = 2.

Les produits de formule (VII) dans lesquels W est un radical mercapto, éventuellement activé, sont des produits nouveaux et l'invention a donc également pour objet les produits de formule (VII) telle que définie précédemment, dans laquelle W représente un radical mercapto éventuellement activé à titre de produits intermédiaires nouveaux. Les produits nouveaux (VII) définis ci-dessus peuvent être préparés par action d'un magnésien contenant un groupement mercapto sur un produit de formule (XII). Des exemples spécifiques de préparations de tels produits nouveaux (VII) sont

décrits ci-après dans les exemples.

La demande de brevet européen EP 0384842 (n/cas 2235) déposée le 22 février 1990 a décrit de nouveaux 19-Nor stéroïdes ayant en 11béta une chaîne carbonée comportant une fonction amide ou carbamate, répondant à la formule générale :

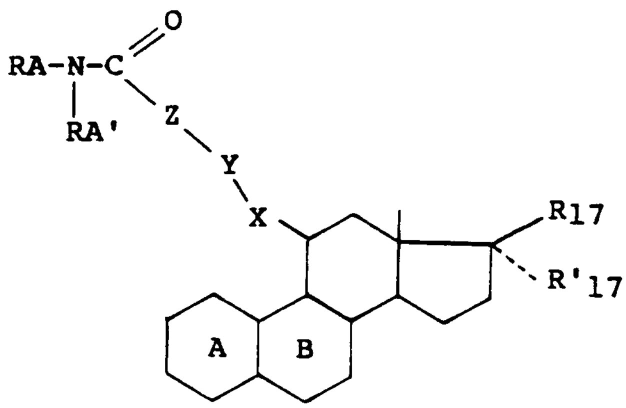

dans laquelle les cycles A et B ont l'une des structures suivantes :

a) soit A et B représentent le groupement :

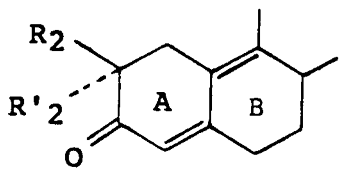

dans lequel $R_2$ et $R'_2$ identiques ou différents représentent un atome d'hydrogène ou un radical alkyle ayant de 1 à 4 atomes de carbone ;

b) soit A et B représentent le groupement :

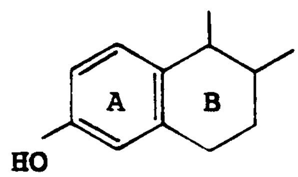

dans lequel $R_3$ représente un atome d'hydrogène ou un radical alkyle renfermant de 1 à 4 atomes de carbone ou un radical acyle,

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène ou oxygène et éventuellement terminée par un radical arylène,

- Z représente une simple liaison ou un radical $CH_2$-O lié au radical Y par l'atome de carbone,

étant entendu que lorsque Y et Z sont une simple liaison, X ne peut être un radical méthylène ou $CH_2$-O,
RA et RA', identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène.

Les composés de formule (I) de la présente demande constituent pour certains des composés répondant à la formule générale ci-dessus mais non décrits nommément dans cette demande et pour d'autres des composés apparentés.

Des exemples de préparation des produits (VIII) et (X) figurent ci-après dans la partie expérimentale.

__Préparation 1 :__ **chlorhydrate de N-hepta-fluorobutyl N-méthylamine.**

On refroidit à 0°C 100 $cm^3$ d'éther anhydre, 100 $cm^3$ de tétrahydrofuranne anhydre puis fait barboter, pendant 10 minutes, de la méthylamine. On introduit en une demi-heure 44,98 g d'anhydride heptafluorobutyrique en maintenant un faible barbottage de méthylamine. On agite pendant 2 heures en laissant revenir à température ambiante. On distille à faible volume sous pression réduite, reprend par 200 $cm^3$ de tétrahydrofuranne anhydre et introduit lentement 30 $cm^3$ de complexe diborane-diméthyl sulfure. On porte au reflux pendant 16 heures. On refroidit à température ambiante puis introduit lentement 200 $cm^3$ de méthanol. On fait ensuite barboter de l'acide chlorhydrique gazeux pendant 15 minutes. On porte à reflux pendant 1 heure puis distille les solvants sous pression réduite. On reprend le résidu par 200 $cm^3$ de méthanol. On fait de nouveaux barboter pendant 10 minutes de l'acide chlorhydrique gazeux puis porte à reflux pendant 2 heures. On distille le solvant, agite 10 minutes dans 100 $cm^3$ d'acide chlorhydrique 6N glacé. On essore, lave à l'acide chlorhydrique 2N, sèche et obtient 22,699 g de produit attendu. Le chlorhydrate obtenu ci-dessus est purifié par cristallisation dans 140 $cm^3$ d'éthanol. On ajoute alors 140 $cm^3$ d'éther, agite une demi-heure, essore, lave à l'éther et sèche sous pression réduite. On obtient 21,7 g de produit recherché (sublime à environ 200°C).

| Analyse : $C_5H_6F_7N$, HCl : 249,56 | | | | | |
|---|---|---|---|---|---|
| Calculé | C% 24,06 | H% 2,83 | Cl% 14,20 | F% 53,29 | N% 5,61 |
| Trouvé | 24,0 | 2,8 | 14,4 | 52,3-52,1 | 5,6 |

__Préparation 2 :__ **[(5-chloro pentyl) oxy] diméthyl (1,1-diméthyl)-silane.**

A une solution de 10,2 g de 5-chloropentanol dans 80 $cm^3$ de chlorure de méthylène, on ajoute 13,8 $cm^3$ de triéthylamine puis 200 mg de 4-diméthyl aminopyridine sous atmosphère d'azote, on refroidit à -10°C et ajoute 13,75 g de terbutyl chloro diméthyl-silane. On agite pendant 15 minutes à -10°C et laisse réchauffer à température ambiante et dilue avec une solution saturée de bicarbonate de sodium. On sépare le dérivé brut huileux et chromatographie sur silice (éluant : cyclohexane-acétate d'éthyle 95-5) et obtient 18 g de produit attendu.

Spectre IR : ($CHCl_3$)

| | |
|---|---|
| OH | absence |
| O-Si+ | présence |

**Préparation 3 : [(3-bromopropyl oxy)]-diméthyl-(1,1-diméthyl éthyl)-silane.**

Stade A : 3-[[diméthyl (1,1-diméthyl éthyl) silyl] oxy] propanol.

Dans un ballon placé sous atmosphère d'argon on introduit 9,46 g d'hydrure de sodium à 50 % dans l'huile que l'on élimine par lavage avec de l'hexane, on ajoute 380 ml de tétrahydrofuranne, 15 g de 1,3-propanediol et agite pendant 1 heure à température ambiante. On refroidit à +5°C, ajoute 29,7 g de terbutylchlorodiméthyl silane, agite pendant 1 heure à température ambiante puis verse dans 3,5 litres d'éther éthylique. On lave avec 1 litre de solution aqueuse de carbonate de potassium à 10 %, puis avec 1 litre de solution aqueuse saturée de chlorure de sodium. On sèche sur sulfate de sodium et évapore à sec sous pression réduite. On chromatographie le résidu (35,5 g) sur silice (éluant : acétate d'éthylecyclohexane 4-6). On obtient 30,6 g de produit attendu.

Spectre IR : ($CHCl_3$)

| | |
|---|---|
| OH | 3625, 3500 $cm^{-1}$ |
| -O-Si+ | 1258, 838 $cm^{-1}$ |

Stade B : [(3-bromopropyl) oxy]-diméthyl-(1,1-diméthyl éthyl)silane.

A une solution de 2,5 g de produit obtenu au stade A ci-dessus dans 27 ml de chlorure de méthylène refroidie à -25°C on ajoute 5,44 g de tétrabromométhane et 4,30 g de triphénylphosphine. On agite 1 h 30 à 0°C puis chromatographie la solution réactionnelle sur silice (éluant : acétate d'éthylecyclohexane 1-9). On isole 2,885 g de produit attendu.

| Analyse : $C_9H_{21}BrOSi$ | | | |
|---|---|---|---|
| | % C | % H | % Br |
| Calculé | 42,68 | 8,35 | 31,55 |
| Trouvé | 40,0 | 7,9 | 36,6 |

Spectre IR : ($CHCl_3$)

-O-Si+ 1258, 837 $cm^{-1}$

**Préparation 4 : 4-triméthyisilyléthynyl-bromobenzène.**

On introduit 150 g de bromo iodo benzène à 97 %, 500 $cm^3$ de diméthylformamide anhydre, 100 $cm^3$ de triéthylamine, 50 g de triméthylsilyl acétylène, 2,1 g d'iodure de cuivre et 2,22 g de bis (triphénylphosphine) palladium (II) dichlorure. On agite pendant 2 heures puis ajoute 500 $cm^3$ d'eau glacée et extrait 3 fois par 500 $cm^3$ d'acétate d'éthyle. La phase organique est lavée à l'eau salée puis séchée sur sulfate de soude. Les solvants sont évaporés sous pression

réduite. On obtient 136,54 g d'huile brune. On purifie par distillation sous pression réduite et obtient 106,97 g de produit attendu.
Eb : 75-82°C sous 0,2 mbar. F = 62°C.

Spectre IR : ($CHCl_3$)

absence de C=CH
C=C 2160 $cm^{-1}$

**Préparation 5 : [(8-bromo octyl) oxy] diméthyl (1,1-diméthyl éthyl) silane.**

A une solution de 3,97 g de 8-bromo octanol dans 19 $cm^3$ de diméthylformamide, on ajoute 1,55 g d'imidazole, et en 10 minutes, 3,32 g de terbutyl chloro diméthyl silane en solution dans 4,7 $cm^3$ de tétrahydrofuranne. On agite pendant 1 heure à température ambiante, filtre l'insoluble et évapore à sec sous pression réduite. On chromatographie sur silice (éluant : cyclohexane-toluène 8-2) et on obtient 5,4 g de produit attendu.

**Préparation A de l'exemple 1 : 11béta-(4-hydroxyphényl) estra-4,9-dièn-3,17-dione.**

Stade A : 3-(1,2-éthanediyl) acétal cyclique de 11béta-(4-hydroxyphényl) 5-alpha-hydroxy estra-9-èn 3,17-dione.

a) Préparation du magnésien

A une suspension de 7,1 g de magnésium en tournures dans 14,5 $cm^3$ de tétrahydrofuranne on ajoute goutte à goutte au reflux 50 g de triméthylsilyloxy 4-bromo benzène dans 100 $cm^3$ de tétrahydrofuranne. On agite pendant 30 minutes au reflux et obtient une solution d'environ 0,95 M de magnésien.

b) Condensation

On agite, pendant 10 minutes à température ambiante 10 g de 3-(1,2-éthanediyl) acétalcyclique de 5alpha, 10alpha-époxy estr-9,11-èn-3,17-dione obtenu selon EP 0057 115 (exemple 7) en solution dans 200 $cm^3$ de tétrahydrofuranne et 0,45 g de chlorure de cuivre. On ajoute en 20 minutes 110 $cm^3$ de la solution du magnésien obtenu ci-dessus, sans dépasser 27°C. Après 1 h 30 d'agitation, on verse dans 1300 $cm^3$ de solution glacée saturée de chlorure d'ammonium. La phase aqueuse est extraite 3 fois avec de l'acétate d'éthyle, on lave avec une solution saturée de chlorure de sodium déshydrate et évapore à sec sous pression réduite et obtient 34,9 g de produit brut.

c) Désilylation

Le produit brut obtenu ci-dessus est dissous dans 150 $cm^3$ de tétrahydrofuranne, on ajoute 130 $cm^3$ d'une solution 1M de fluorure de tétrabutylammonium. On agite 15 minutes à température ambiante, verse dans l'eau, extrait avec de l'acétate d'éthyle, lave à l'eau, sèche et évapore à sec sous pression réduite. On obtient 26,9 g de produit brut que l'on empâte à 40°C pendant 30 minutes dans 100 $cm^3$ d'un mélange acétate d'éthyle-chlorure de méthylène (1-1). On filtre l'insoluble et obtient 5,77 g de produit recherché. Par chromatographie sur silice des liqueurs mères (éluant : acétate d'éthyle-chlorure de méthylène 1-1), on recueille 5,7 g supplémentaires de produit recherché. On réunit les 2 lots de produits obtenus (11,47 g) que l'on recristallise dans l'éthanol. On obtient 8 g du produit attendu. F = 255°C.

Spectre IR : ($CHCl_3$)

Région OH 3464, 3280 $cm^{-1}$
C=O 1720 $cm^{-1}$
aromatique 1613, 1592, 1511 $cm^{-1}$

Stade B : 11béta-(4-hydroxyphényl) estra 4,9-dièn-3,17-dione.

On agite pendant 1 h 30, 5 g du produit obtenu au stade A, 110 $cm^3$ d'éthanol et 28 $cm^3$ d'acide chlorhydrique 2N. On alcalinise à pH environ 9 avec de l'ammoniaque concentrée. On évapore à sec et reprend avec de l'acétate d'éthyle, lave à l'eau, avec une solution saturée de chlorure de sodium, sèche et évapore à sec sous pression réduite. On chromatographie le résidu (4,8 g) sur silice (éluant : chlorure de méthylène-acétate d'éthyle 7-3). On obtient 3,97 g de produit recherché.

Spectre IR : ($CHCl_3$)

| | |
|---|---|
| OH | 3596 $cm^{-1}$ |
| C=O | 1735 $cm^{-1}$ |
| C=O | 1657 $cm^{-1}$ |
| aromatique | 1612, 1593, 1511 $cm^{-1}$ |

**Préparation B de l'exemple 1 : [(5-bromopentyl) oxy] N-butyl N-méthyl acétamide.**

Stade A : bromo N-butyl N-méthyl acétamide.

A une solution refroidie à -20°C de 11,9 $cm^3$ de bromure de bromoacétyle dans 180 $cm^3$ d'éther, on ajoute 26 g de butyl-méthylamine méthylamine en solution dans 120 $cm^3$ d'éther. On ramène la température à 20°C, agite 30 minutes, dilue à l'eau et extrait à l'éther. On évapore à sec sous pression réduite. On distille le résidu (27,4 g) sous pression réduite (0,05 mbar) à 79/83°C. On obtient 19,36 g de produit recherché.

| Analyse : $C_7H_{14}BrNO$ = 208,105 | | | | |
|---|---|---|---|---|
| Calculé | C% 40,40 | H% 6,78 | N% 6,73 | Br% 38,39 |
| Trouvé | 40,3 | 7,0 | 6,7 | 38,2 |

Stade B : 5-[[(diméthyl (1,1-diméthyléthyl) silyl] oxy] pentanol.

A une solution composée de 10 g de 4-pentanol, 200 $cm^3$ de chlorure de méthylène, 19,5 $cm^3$ de triéthylamine et 566 mg de 4-diméthylaminopyridine, on ajoute en refroidissant 19,14 g de tertbutylchlorodiméthylsilane. On agite 1 heure à température ambiante, dilue à l'eau, décante la phase organique, la lave, la sèche et l'évapore à sec sous vide. On chromatographie le résidu (42 g) sur silice (éluant : essence G-acétate d'éthyle 95-5). On obtient 23,3 g de silyloxy pentène que l'on dissout dans 250 $cm^3$ de tétrahydrofuranne. On ajoute à 20°C 6 $cm^3$ de complexe borane-méthylsulfure. On agite 30 minutes à 20/25°C puis 30 minutes à 35°C. On ajoute à +10°C, 18 $cm^3$ de lessive de soude puis 18 $cm^3$ d'eau oxygénée, on agite 30 minutes. On dilue à l'eau, extrait avec de l'acétate d'éthyle, lave avec une solution de thiosulfate de sodium à 10 %, sèche et concentre à sec sous pression réduite. On chromatographie le résidu (25,85 g) sur silice (éluant : cyclohexane-acétate d'éthyle 8-2). On recueille 22,7 g de produit que l'on distille sous pression réduite (0,06 mbar) et obtient 18,7 g du composé recherché.
Eb = 73-75°C/0,06 mbar.

Stade C : N-butyl [(5-hydroxypentyl) oxy] N-méthyl acétamide.

A une solution de 8 g de l'alcool obtenu au stade B ci-dessus, dans 40 $cm^3$ de tétrahydrofuranne, on ajoute 2,16 g d'hydrure de sodium à 50 % dans l'huile, agite 30 minutes à température ambiante, ajoute goutte à goutte en 15 minutes une solution de 9,5 g du produit bromé obtenu au stade A ci-dessus dans 13 $cm^3$ de tétrahydrofuranne. On agite 16 heures à température ambiante, ajoute une solution aqueuse saturée de chlorure d'ammonium, extrait à l'acétate d'éthyle, lave, sèche et évapore à sec sous vide. On obtient 14,8 g de N-butyl [[5-[[diméthyl (1,1-diméthyléthyl) silyl] oxy] pentyl] oxy] N-méthyl acétamide intermédiaire que l'on dissout dans 83 $cm^3$ de tétrahydrofuranne et 46 $cm^3$ d'une solution 1M de fluorure de tétrabutylammonium. On agite 2 heures à température ambiante, coule dans l'eau, extrait à l'acétate d'éthyle, évapore à sec sous pression réduite. On chromatographie le résidu (13,6 g) sur silice (éluant : chlorure de méthylène-isopropanol 94-6) et obtient 7,28 g du composé recherché.

Spectre IR : ($CHCl_3$)

| | |
|---|---|
| -OH | 3628 $cm^{-1}$ |
| C=O | 1645 $cm^{-1}$ |

Stade D : [(5-bromopentyl) oxy] N-butyl N-méthyl acétamide.

A une solution de 7,2 g du produit obtenu au stade C ci-dessus dans 73 $cm^3$ de chlorure de méthylène, on ajoute à -10°C 13 g de tétrabromométhane et 10,3 g de triphénylphosphine. On agite 1 heure à 0°C et chromatographie le

milieu réactionnel sur silice (éluant : acétate d'éthyle-cyclohexane 7-3). On obtient 7,49 g du composé recherché.

Spectre IR : ($CHCl_3$)

C=O 1644 $cm^{-1}$

| Analyse : $C_{12}H_{24}BrNO_2$ = 294,24 | | | | |
|---|---|---|---|---|
| Calculé | C% 48,98 | H% 8,22 | N% 4,76 | Br% 27,15 |
| Trouvé | 48,6 | 8,2 | 4,6 | 26,3 |

**Example 1 : N-butyl-5-(4-(3,17béta-dihydroxy-estra-1,3,5(10)-trien-11béta-yl) phénoxy) pentyloxy)-N-méthyl-éthanethioamide.**

Stade A : N-butyl [5-[4-(3,17-dioxo estra-4,9dièn-11béta-yl) phénoxy] pentyloxy] N-méthyl acétamide.

A une solution de 2,5 g de produit obtenu au stade B de la préparation A de l'exemple 1 dans 26 $cm^3$ d'acétone et 6,4 $cm^3$ de soude 2N, on ajoute 3,75 g de [(5-bromopentyl) oxy] N-butyl N-méthyl acétamide obtenu ci-dessus en solution dans 6 $cm^3$ d'acétone. On agite 5 heures à 50°C, refroidit, verse dans l'eau, acidifie avec de l'acide chlorhydrique 2N, extrait avec de l'acétate d'éthyle, lave, sèche et évapore à sec sous pression réduite. On chromatographie le résidu (6,8 g) sur silice (éluant : acétate d'éthyle) et obtient 2,63 g du produit recherché.

Spectre IR : ($CHCl_3$)

| | |
|---|---|
| C=O | 1735 $cm^{-1}$ (17-céto) |
| | 1657 $cm^{-1}$ |
| C=C + aromatique | 1609, 1580, 1509 $cm^{-1}$ |

Stade B : N-butyl [5-[4-(3-hydroxy 17-oxo estra-1,3,5(10)-trièn-11béta-yl) phénoxy] pentyloxy] N-méthyl acétamide.

A une solution de 2,61 g de produit obtenu au stade A ci-dessus dans 50 $cm^3$ de méthanol, on ajoute 2,61 g d'hydroxyde de palladium à 20 % sur oxyde de magnésium. On chauffe au reflux pendant 30 minutes. On filtre le catalyseur, lave avec du méthanol, évapore le filtrat à sec sous pression réduite et reprend le résidu (2,5 g) avec 75 $cm^3$ de méthanol. A la solution obtenue, on ajoute 2,45 g de potasse en pastille et agite 45 minutes à température ambiante. On ajoute 130 g de glace et 50 $cm^3$ d'acide chlorhydrique 2N, extrait avec du chlorure de méthylène et évapore à sec sous pression réduite. On chromatographie le résidu (2,75 g) sur silice (éluant : acétate d'éthyle-essence G 9-1) et obtient 1,83 g du produit recherché.

Spectre IR : ($CHCl_3$)

| | |
|---|---|
| OH | 3598 $cm^{-1}$ |
| C=O | 1732 $cm^{-1}$ |
| | 1634 $cm^{-1}$ |
| aromatique | 1611, 1581, 1511 $cm^{-1}$ |

Stade C : N-butyl [5-[4-(3,17béta-dihydroxy estra-1,3,5(10)-trièn-11béta-yl) phénoxy] pentyloxy] N-méthyl acétamide.

A une solution de 500 mg de produit obtenu au stade B ci-dessus dans 8 $cm^3$ de méthanol, on introduit en 15 minutes 66 mg d'hydrure de bore et de sodium. On agite pendant 2 heures et verse dans 40 $cm^3$ d'eau, extrait avec du chlorure de méthylène. On évapore à sec sous pression réduite. On chromatographie le résidu (514 mg) sur silice (éluant : acétate d'éthyle) puis une seconde fois avec le même éluant. On obtient 343 mg de produit recherché.
[$alpha_D$] = -31,1° (c = 1 % chloroforme).

Spectre IR : ($CHCl_3$)

OH 3603 $cm^{-1}$
C=O 1634 $cm^{-1}$ (amide III)
aromatique 1611, 1581, 1511 $cm^{-1}$

| Analyse : $C_{36}H_{51}NO_5$ = 577,81 | | | |
|---|---|---|---|
| Calculé | C% 74,83 | H% 8,89 | N% 2,42 |
| Trouvé | 74,8 | 9,0 | 2,3 |

Stade D : N-butyl-[5-[4-(3,17béta diacétoxy-estra-1,3,5(10)-trièn-11béta-yl) phénoxy] pentyloxy]-N-méthyl-acétamide.

A une solution de 450 mg de produit obtenu au stade C ci-dessus dans 2,3 $cm^3$ de pyridine on ajoute 0,9 $cm^3$ d'anhydride acétique à 98 % et 23 mg de 4-diméthylaminopyridine.

On agite pendant 1 heure et ajoute 5 $cm^3$ d'eau et 5 $cm^3$ de méthanol. On agite pendant 10 minutes puis extrait avec de l'acétate d'éthyle. On lave avec une solution saturée de chlorure de sodium, sèche et évapore à sec sous pression réduite. On chromatographie le résidu (580 mg) sur silice (éluant : acétate d'éthyle) et obtient le produit recherché.

Spectre IR : ($CHCl_3$)

diacétate 1730, 1760 $cm^{-1}$ (épaulement)
amide 1640, 1660 $cm^{-1}$

Stade E : N-butyl-[5-[4-(3,17béta-diacétoxy-estra-1,3,5(10)-trièn-11béta-yl) phénoxy] pentoxy]-N-méthyl-éthanethioamide.

A une solution de 480 mg de produit obtenu au stade D ci-dessus dans 10 $cm^3$ de toluène on ajoute 400 mg de réactif de Lawesson. On agite pendant 1 heure à 50°C. Après refroidissement à température ambiante, on filtre puis évapore à sec sous pression réduite. On chromatographie le résidu (840 mg) sur silice (éluant : essence G-acétate d'éthyle 1-1) et obtient 450 mg de produit recherché.

Spectre IR : ($CHCl_3$)

diacétate 1730, 1760 $cm^{-1}$ (épaulement)
aromatique 1600 $cm^{-1}$

Stade F : N-butyl-[5-[4-(3,17béta-dihydroxy-estra-1,3,5(10)-trièn-11béta-yl) phénoxy] pentyloxy]-N-méthyl-éthanethioamide.

A une solution de 480 mg de produit obtenu au stade E ci-dessus dans 10 $cm^3$ de méthanol on ajoute 2,8 $cm^3$ de soude 2N. On agite pendant 4 heures à température ambiante. On neutralise avec de l'acide chlorhydrique 2N. On extrait à l'acétate d'éthyle, lave avec une solution saturée de chlorure de sodium, sèche puis évapore à sec sous pression réduite. On chromatographie le résidu (428 mg) sur silice (éluant : essence G-acétate d'éthyle 1-1). On obtient 365 mg de produit attendu.
$[alpha]_D$ -22°5 ± 1°5 (c = 1 % $CHCl_3$)

| Analyse : pour $C_{36}H_{51}NO_4S$ = 593,88 | | | | |
|---|---|---|---|---|
| | C% | H% | N% | S% |
| Calculé | 72,81 | 8,65 | 2,36 | 5,40 |
| Trouvé | 71,9 | 8,8 | 2,3 | 5,6 |

Spectre IR : ($CHCl_3$)

| | |
|---|---|
| C=O | absence |
| OH | 3603 $cm^{-1}$ + associé |
| aromatique | 1630, 1581, 1512 $cm^{-1}$ |

**Préparation de l'exemple 2 : 8-bromo-N-butyl N-méthyl octanamide.**

A une solution de 5 g d'acide 8-bromo octanoïque dans 200 $cm^3$ de chlorure de méthylène on ajoute goutte à goutte à - 10°/-15°C, 9,1 $cm^3$ de N-méthylmorpholine puis 10,4 $cm^3$ de chloroformiate d'isobutyle. On agite pendant 30 minutes à - 10°/-15°C, puis ajoute à cette température 13 $cm^3$ de N-méthylbutylamine. On laisse réchauffer à température ambiante et reposer 40 minutes, puis on ajoute 150 $cm^3$ d'une solution saturée de bicarbonate de sodium. On agite pendant 10 minutes. On décante, extrait avec du chlorure de méthylène, lave avec une solution saturée de chlorure de sodium, sèche et évapore à sec sous pression réduite. On chromatographie le résidu sur silice (éluant : chlorure de méthylène-acétone 95-5) et obtient 6,14 g du produit attendu.

Spectre IR : ($CHCl_3$)

C=O 1627 $cm^{-1}$ (amide III)

**Exemple 2 : N-butyl-8-(4-(3,17béta-dihydroxy-estra-1,3,5(10)-trien-11béta-yl) phénoxy)-N-méthyl-octanethioamide.**

Stade A : 8-[4-(3,17béta-dioxoestra-4,9-dièn-11béta-yl) phénoxy] N-butyl N-méthyl octanamide.

On opère comme au stade A de l'exemple 1 à partir de 725 mg de produit obtenu au stade B de la préparation A de l'exemple 1 en utilisant 0,2 $cm^3$ de 8-bromo N-butyl N-méthyl octanamide obtenu à la préparation ci-dessus. Après chromatosur silice (éluant : Essence G-acétate d'éthyle 4-6), on obtient 540 mg du produit attendu.

Spectre IR : ($CHCl_3$)

| | |
|---|---|
| 17-céto | 1735 $cm^{-1}$ |
| 3-céto | 1657 $cm^{-1}$ |
| amide III | 1628 $cm^{-1}$ |
| bandes aromatiques | 1580, 1509 $cm^{-1}$ du type $-O-C_6H_5$ |

Stade B : 8-[4-(3-hydroxy 17-oxo estra-1,3,5(10)-trièn-11béta-yl) phénoxy] N-butyl N-méthyl octanamide.

On opère comme au stade B de l'exemple 1 à partir de 470 mg du produit obtenu au stade A ci-dessus en utilisant 260 mg d'hydroxyde de palladium sur oxyde de magnésium. Après chromatographie sur silice (éluant : acétate d'éthyle-essence G 1-1), on obtient 360 mg du composé recherché.

Spectre IR : ($CHCl_3$)

| | |
|---|---|
| OH | 3596 $cm^{-1}$ |
| C=O | 1732 $cm^{-1}$ |
| amide III | 1623 $cm^{-1}$ |
| bandes aromatiques | 1581, 1511 $cm^{-1}$ |

Stade C : N-butyl-8-[4-(3,17béta-dihydroxy estra-1,3,5(10)-trièn-11béta-yl) phénoxy] N-méthyl octanamide.

On opère comme au stade C de l'exemple 1 à partir de 360 mg du produit obtenu au stade B ci-dessus en utilisant 72 mg d'hydrure de bore et de sodium. On obtient 460 mg de produit attendu.

Spectre IR : ($CHCl_3$)

| | |
|---|---|
| OH | 3602 $cm^{-1}$ |
| C=O | 1623 $cm^{-1}$ (amide III) |

bandes aromatiques 1581, 1511 $cm^{-1}$

Stade D : N-butyl-8-[4-(3,17béta-diacétoxy-estra-1,3,5(10)-trièn-11béta-yl) phénoxy] N-méthyl octanamide.

On opère comme au stade D de l'exemple 1 à partir de 460 mg de produit obtenu au stade C ci-dessus en utilisant 0,9 $cm^3$ d'anhydride acétique et 24 mg de 4-diméthyl amino pyridine.

On chromatographie le résidu (510 mg) sur silice (éluant : essence G-acétate d'éthyle 1-1) et obtient le produit recherché.

Spectre IR : ($CHCl_3$)

diacétate 1730 $cm^{-1}$, 1720 $cm^{-1}$ (épaulement)
amide 1630 $cm^{-1}$

Stade E : N-butyl-8-[4-3,17béta-acétoxy-estra-1,3,5(10)trièn11béta-yl) phénoxy] N-méthyl octane thioamide.

On opère comme au stade E de l'exemple 1 à partir de 510 mg de produit obtenu au stade D ci-dessus en utilisant 1,54 g de réactif de Lawesson. Le chauffage à 50°C est effectué pendant 3 h 15. On obtient 1,18 g de résidu que l'on chromatographie sur silice (éluant : essence G-acétate d'éthyle 7-3) et obtient 430 mg de produit recherché.

Spectre IR : ($CHCl_3$)

diacétate 1730 $cm^{-1}$, 1750 $cm^{-1}$ (épaulement)
C=S 1500 $cm^{-1}$

Stade F : N-butyl-8-[4-[3,17béta-dihydroxy-estra-1,3,5(10)-trièn-11béta-yl] phénoxy]-N-méthyl-octane thioamide.

On opère comme au stade F de l'exemple 1 à partir de 408 mg de produit obtenu au stade E ci-dessus. L'agitation à température ambiante est effectuée pendant 1 heure. On isole 370 mg de résidu. Après chromatographie, on isole 261 mg de produit attendu.
$[alpha]_D$ = -31°5 ± 1°5 (C = 1 % $CHCl_3$)

| Analyse : $C_{37}H_{53}NO_3S$ = 591,90 | | | | |
|---|---|---|---|---|
| | C% | H% | N% | S% |
| Calculé | 75,08 | 9,02 | 2,37 | 5,42 |
| Trouvé | 75,3 | 9,2 | 2,3 | 5,52 |

Spectre IR : ($CHCl_3$)

OH 3601 $cm^{-1}$
aromatique 1610 - 1580 - 1511 $cm^{-1}$

**Exemple 3 : monobutanedioate de 11béta-(4-((7-((butylméthylamino) carbonyl) heptyl) oxy) phényl)-3-hydroxy-estra-1,3,5(10)-trièn-17béta-yle.**

A une solution de 200 mg de produit obtenu au stade C de l'exemple 2 dans 2 $cm^3$ de pyridine, on ajoute 210 mg d'anhydride succinique. On chauffe pendant 5 heures dans un bain à 115°C. On ajoute 2 $cm^3$ d'eau, 350 mg de carbonate de potassium et 2 $cm^3$ de méthanol. On agite pendant 4 heures à température ambiante, puis acidifie avec de l'acide chlorhydrique 6N. On extrait avec de l'acétate d'éthyle, lave avec une solution saturée de chlorure de sodium, sèche puis évapore à sec sous pression réduite. On chromatographie le résidu (300 mg) sur silice (éluant : essence G-acétone 6-4, acide acétique 1 %) et obtient 176 mg de produit attendu.
$[alpha]_D$ = -75° (C = 1 % $CHCl_3$)

| Analyse : $C_{41}H_{57}NO_7$ = 675,91 | | | |
|---|---|---|---|
| | C% | H% | N% |
| Calculé | 72,86 | 8,50 | 2,07 |
| Trouvé | 72,9 | 8,7 | 2,1 |

Spectre IR : ($CHCl_3$)

| | |
|---|---|
| OH | 3600 $cm^{-1}$ |
| C=O complexe | 1730 $cm^{-1}$ (épaulement) |
| | 1717 $cm^{-1}$ |
| C=O amide III | 1622 $cm^{-1}$ |
| aromatique | 1611, 1583, 1511 $cm^{-1}$ |

**Exemple 4 : butanedioate de 11béta-(4-((7-((butylméthylamino) carbonyl) heptyl) oxy) phényl)-3-hydroxy-estra-1,3,5(10)-trièn-17béta-yle et de sodium.**

A une solution de 18 mg de bicarbonate de sodium dans 3 $cm^3$ d'eau on ajoute une solution de 160 mg du produit obtenu à l'exemple 3 dans 6 $cm^3$ d'éthanol. On agite pendant 30 minutes, distille l'éthanol sous pression réduite et filtre. Après lyophilisation, on obtient 145 mg de produit attendu.

| Analyse : $C_{41}H_{56}N NaO_7$ = 697,90 | | | |
|---|---|---|---|
| | C% | H% | N% |
| Calculé | 70,56 | 8,09 | 2,01 |
| Trouvé | 69,9 | 8,1 | 2,0 |

**Préparation de l'exemple 5 : acide 8-[4-(3,17-dioxo-estra-4,9-dièn-11béta-yl) phénoxy] octanoïque.**

A une solution de 181 mg de produit obtenu au stade B de la préparation A de l'exemple 1 dans 4 $cm^3$ d'acétone et 1,5 $cm^3$ de soude 2N on ajoute 335 mg d'acide 8-bromo octanoïque. On maintient au reflux pendant 5 heures, refroidit à température ambiante puis acidifie avec de l'acide chlorhydrique 2N. On extrait avec de l'acétate d'éthyle, lave avec une solution saturée de chlorure de sodium, sèche puis évapore à sec sous pression réduite. On obtient 600 mg de produit que l'on utilise tel que.

**Exemple 5 : 8-(4-(3,17béta-dihydroxy-estra-1,3,5(10)-trien-11béta-yl) phénoxy)-N-(2,2,3,3,4,4,4-heptafluorobutyl)-N-méthyl-octanamide.**

Stade A : 8-[4-(3,17-dioxo-estra-4,9-dièn-11béta-yl) phénoxy]-N-(2,2,3,3,4,4,4-heptafluoro butyl)-N-méthyloctanamide.

Dans un ballon sous atmosphère d'azote, on introduit successivement 1,29 g de produit brut obtenu selon la préparation ci-dessus, 13 $cm^3$ de chlorure de méthylène et 0,65 $cm^3$ de méthylmorpholine. On refroidit à -10°C et introduit goutte à goutte 0,39 $cm^3$ de chloroformiate d'isobutyle. On agite pendant 30 minutes à -10°C. Dans la solution obtenue et maintenue à -10°C on introduit 1,78 mg de chlorhydrate de N-heptafluoro butyl N-méthylamine obtenu à la préparation 1. On laisse réchauffer à température ambiante en 30 minutes, agite pendant 30 minutes, lave avec une solution saturée de chlorure de sodium et évapore à sec sous pression réduite. On chromatographie le résidu (1,88 g) sur silice (éluant : essence G-acétate d'éthyle 1-1) et obtient 990 mg de produit attendu.

Spectre IR : ($CHCl_3$)

| | |
|---|---|
| C=O | 1735 $cm^{-1}$ (17-céto) |
| | 1659 $cm^{-1}$ (diénone + amide III) |
| C=C + aromatique | 1609, 1578, 1509 $cm^{-1}$ |

Stade B : 8-[4-(3-hydroxy 17-oxo-estra-1,3,5(10)-trièn-11béta-yl) phénoxy]-N-(2,2,3,3,4,4,4-heptafluorobutyl)-N-méthyl octanamide.

Dans un ballon sous atmosphère d'azote on introduit 980 mg du produit obtenu au stade A ci-dessus et 10 $cm^3$ de chlorure de méthylène. On refroidit à 0°C et ajoute en 5 minutes 1 $cm^3$ d'anhydride acétique à 98 % et 0,5 $cm^3$ de bromure d'acétyle. On agite pendant 45 minutes à température ambiante puis alcalinise avec du bicarbonate de sodium. On agite pendant 45 minutes, recueille la phase organique, lave avec une solution saturée de chlorure de sodium, sèche puis évapore à sec sous pression réduite. Le résidu obtenu (1,02 g) est dissous sous atmosphère d'argon dans 10 $cm^3$ de méthanol, puis on ajoute 1,4 $cm^3$ de soude 2N. On agite pendant 1 heure à température ambiante, neutralise avec 1,4 $cm^3$ d'acide chlorhydrique 2N. On extrait avec du chlorure de méthylène, lave avec de l'eau, sèche, puis évapore à sec sous pression réduite. On chromatographie le résidu (1 g) sur silice (éluant : essence G-acétate d'éthyle 6-4) et obtient 630 mg de produit attendu.

Spectre IR : ($CHCl_3$)

| | |
|---|---|
| OH | 3600 $cm^{-1}$ |
| C=O | 1732 $cm^{-1}$ (17-céto) |
| | 1658 $cm^{-1}$ (amide III) |
| aromatique | 1610, 1576, 1511 $cm^{-1}$ |

Stade C : 8-[4-(3,17béta-dihydroxy-estra-1,3,5(10)-trièn-11béta-yl) phénoxy]-N-(2,2,3,3,4,4,4-heptafluorobutyl)-N-méthyl-octanamide.

A une solution de 310 mg de produit obtenu au stade B ci-dessus dans 4 $cm^3$ de méthanol que l'on refroidit à 0°C on ajoute 18 mg d'hydrure de bore et de sodium. On agite pendant 45 minutes à 0°C, extrait avec du chlorure de méthylène, sèche puis évapore à sec sous vide. On chromatographie le résidu (315 mg) sur silice (éluant : essence G-acétate d'éthyle 1-1) et obtient 280 mg de produit attendu.

| Analyse : $C_{37}H_{46}F_7NO_4$ = 701,77 | | | | |
|---|---|---|---|---|
| | C% | H% | F% | N% |
| Calculé | 63,33 | 6,60 | 18,95 | 1,99 |
| Trouvé | 63,3 | 6,6 | 19,3 | 2,0 |

Spectre IR : ($CHCl_3$)

| | |
|---|---|
| OH | 3603 $cm^{-1}$ |
| C=O | 1658 $cm^{-1}$ |
| aromatique | 1610, 1576, 1511 $cm^{-1}$ |

**Préparation de l'exemple 6 : 2-[(6-bromohexyl) oxy]-N-butyl-N-méthylacétamide.**

Stade A : 6-[[diméthyl(1,1diméthyléthyl) silyl] oxy] hexanol.

On opère comme au stade A de la préparation 3 en partant de 8,12 g d'hydrure de sodium à 50 % dans l'huile, 20 g de 1,6-hexane diol et 25,5 g de terbutyl diméthyl chloro silane. Après chromatographie, on obtient 20,7 g de produit attendu.

Spectre IR : ($CHCl_3$)

OH 3625 $cm^{-1}$
O-Si+ 1257, 837 $cm^{-1}$

Stade B : N-butyl [(6-hydroxyhexyl) oxy]-N-méthyl acétamide.

A une solution de 7 g de produit obtenu au stade A ci-dessus dans 35 $cm^3$ de tétrahydrofuranne, on ajoute 1,78 g d'hydrure de sodium à 50 % dans l'huile, agite 40 minutes à température ambiante, ajoute goutte à goutte en 10 minutes une solution de 7,8 g de bromo N-butyl N-méthylacétamide obtenu au stade A de la préparation B de l'exemple 1 dans 10 $cm^3$ de tétrahydrofuranne. On agite 16 heures à température ambiante, ajoute une solution aqueuse saturée de chlorure d'ammonium, extrait à l'acétate d'éthyle, lave, sèche et évapore à sec sous pression réduite. On dissout le résidu (13,4 g) dans 69 $cm^3$ de tétrahydrofuranne et ajoute 48 $cm^3$ d'une solution de fluorure de tétrabutylammonium 1M. On agite pendant 3 heures à température ambiante, verse dans de l'eau, extrait à l'acétate d'éthyle, lave avec une solution saturée de chlorure de sodium, sèche puis évapore sous pression réduite. On chromatographie le résidu (10,8 g) sur silice (éluant : chlorure de méthylène-isopropanol 94-6) et obtient 5,56 g de produit attendu.

Spectre IR : ($CHCl_3$)

OH 3624 $cm^{-1}$
C=O 1636 $cm^{-1}$ complexe

Stade C : 2-[(6-bromohexyl) oxy]-N-butyl-N-méthylacétamide.

A une solution de 5,52 g de produit obtenu au stade B ci-dessus dans 55 $cm^3$ de chlorure de méthylène on ajoute à -15°C 9,3 g de tétrabromo-méthane et 7,4 g de triphényl phosphine. On agite pendant 1 heure à 0°C. On chromatographie le mélange réactionnel sur silice (éluant : acétate d'éthyle-cyclohexane 7-3) et obtient 6,22 g de produit attendu.

| Analyse : $C_{13}H_{26}BrNO_2$ = 308,26 | | | | |
|---|---|---|---|---|
| | C% | H% | N% | Br% |
| Calculé | 50,65 | 8,50 | 4,54 | 25,92 |
| Trouvé | 50,4 | 8,8 | 4,6 | 25,5 |

Spectre IR : ($CHCl_3$)

C=O 1640 $cm^{-1}$

**Exemple 6 : N-butyl-2-(6-(3,17béta-dihydroxy-estra-1,3,5(10)-trien-11béta-yl) phénoxy) hexyloxy)-N-méthyl-acétamide.**

Stade A : N-butyl-2-[6-[4-(3,17-dioxo-estra-4,9-dièn-11béta-yl) phénoxy] hexyloxy]-N-méthyl acétamide.

On opère comme au stade A de l'exemple 1 à partir de 2 g de produit obtenu au stade B de la préparation A de l'exemple 1 en utilisant 3,14 g de 2-[(6-bromohexyl) oxy]-N-butyl-N-méthyl acétamide obtenu ci-dessus. On chromatographie le résidu (5,7 g) sur silice (éluant : acétate d'éthyle) et obtient 2,68 g du produit attendu.

Spectre IR : ($CHCl_3$)

| | |
|---|---|
| C=O | 1735 $cm^{-1}$ (17-céto) |
| | 1656 $cm^{-1}$ (diénone + amide III) |
| aromatique | 1609, 1580, 1509 $cm^{-1}$ |

Stade B : N-butyl-2-[6-[4-(3-hydroxy-17-oxo-estra-1,3,5(10)-trièn-11béta-yl) phénoxy] hexyloxy]-N-méthylacétamide.

On opère comme au stade B de l'exemple 1 à partir de 2,63 g de produit obtenu au stade A ci-dessus en utilisant 2,63 g d'hydroxyde de palladium sur oxyde de magnésium. On chromatographie le résidu (2,165 g) sur silice (éluant : chlorure de méthylène-isopropanol 94-6) et obtient 1,62 g de produit attendu.

Spectre IR : ($CHCl_3$)

| | |
|---|---|
| OH | 3599 $cm^{-1}$ |
| C=O | 1732 $cm^{-1}$ (17-céto) |
| | 1635 $cm^{-1}$ (amide III) |

Stade C : N-butyl-2-[6-4(3,17béta-dihydroxy-estra-1,3,5(10)-trièn-11béta-yl) phénoxy]-N-méthylacétamide.

On opère comme au stade C de l'exemple 1 à partir de 450 mg de produit obtenu au stade B ci-dessus en utilisant 58 mg d'hydrure de bore et de sodium. On chromatographie le résidu (461 mg) sur silice (éluant : chlorure de méthylène-isopropanol 94-6) puis une deuxième fois (éluant : acétate d'éthyle). On obtient 274 mg de produit attendu.
$[alpha]_D$ = -32°5 (C = 1 % éthanol)

| Analyse : $C_{37}H_{53}NO_5$ = 591,84 | | | |
|---|---|---|---|
| | C% | H% | N% |
| Calculé | 75,09 | 9,03 | 2,37 |
| Trouvé | 75,4 | 9,1 | 2,4 |

Spectre IR : ($CHCl_3$)

OH 3603 $cm^{-1}$
C=O 1635 $cm^{-1}$
aromatique 1611, 1581, 1511 $cm^{-1}$

**Préparation de l'exemple 7 : 8-bromo N-butyl-N-méthyl 2-octynamide.**

Stade A : diméthyl-(1,1-diméthyl éthyl) [(5-heptyn-1-yl) oxy] silane.

Dans un ballon sous atmosphère d'azote on introduit 1,63 g de complexe acétylure de lithium, éthylène diamine et 15 $cm^3$ de diméthyl sulfoxyde. On agite pendant 30 minutes à température ambiante, puis ajoute en 45 minutes 3,64 g du produit obtenu à la préparation 2. On agite pendant 4 heures, verse dans une solution glacée de chlorure d'ammonium saturée, lave à l'hexane, puis à l'eau, sèche et évapore à sec sous pression réduite. On obtient 3,08 g de produit brut.

Stade B : acide 8-[(diméthyl-(1,1-diméthyl éthyl)]-silyloxy]-2-octynoïque.

A une solution de 630 mg du produit brut obtenu au stade A ci-dessus dans 4 $cm^3$ de tétrahydrofuranne, on ajoute à -60°/-70°C, sous atmosphère d'azote, 2,4 $cm^3$ de solution de butyl lithium à 15 % dans l'hexane. On agite pendant 15 minutes à -60°C, puis place sous atmosphère de $CO_2$. On laisse sous $CO_2$ pendant 15 minutes à -60°C, puis laisse revenir à température ambiante. On dilue avec de l'eau et de l'acétate d'éthyle en émulsionnant. On sépare la phase aqueuse puis extrait la phase organique avec une solution aqueuse de bicarbonate de sodium. On acidifie la phase aqueuse alcaline par addition de phosphate monosodique, puis extrait avec de l'acétate d'éthyle. On évapore à sec la phase organique, sous pression réduite et obtient 540 mg du produit attendu.

Stade C : N-butyl-8-[[diméthyl-(1,1-diméthyl éthyl)] silyloxy] N-méthyl-2-octynamide.

A une solution de 4,55 g obtenu selon le procédé du stade B ci-dessus dans 20 $cm^3$ de chlorure, de méthylène on ajoute 2,8 $cm^3$ de méthyl morpholine, on refroidit à -30°C sous atmosphère d'azote puis ajoute lentement 3,3 $cm^3$ de chloroformiate d'isobutyle en solution dans 10 ml de chlorure de méthylène. On agite à -5°C pendant 35 minutes puis ajoute, à 0°C en 5 minutes, 4 $cm^3$ de méthyl butylamine. On laisse revenir à température ambiante et verse dans une solution aqueuse de bicarbonate de sodium. On extrait avec du chlorure de méthylène, sèche et évapore à sec sous pression réduite. On chromatographie le résidu (6,9 g) sur silice (éluant : cyclohexane-acétate d'éthyle 7-3) et obtient 2 g de produit attendu.

Spectre IR : ($CHCl_3$)

| | |
|---|---|
| C≡C | 2234 $cm^{-1}$ |
| C=O | 1616 $cm^{-1}$ (conjugué) |
| C-O-Si+ | 1257, 1094, 836 $cm^{-1}$ |

Stade D : N-butyl-8-hydroxy-N-méthyl 2-octynamide.

A une solution de 1,9 g de produit obtenu au stade C ci-dessus on ajoute 1 $cm^3$ d'acide chlorhydrique 2N. On maintient 1 heure à température ambiante, extrait avec de l'acétate d'éthyle, lave à l'eau, sèche et évapore à sec sous pression réduite. On chromatographie le résidu (1,79 g) sur silice (éluant : acétate d'éthyle). On obtient 870 mg de produit attendu.

Spectre IR : ($CHCl_3$)

| | |
|---|---|
| OH | 3625 $cm^{-1}$ |
| C≡C | 2285 $cm^{-1}$ |
| C=O | 1616 $cm^{-1}$ (amide) |

Stade E : 8-bromo N-butyl-N-méthyl 2-octynamide.

A une solution de 855 mg de produit obtenu au stade D ci-dessus dans 4 $cm^3$ de chlorure de méthylène on ajoute 1,205 g de triphénylphosphine. On refroidit à -10°C et ajoute 1,522 g de tétrabromométhane. On agite 1 heure à -5°C et on chromatographie le mélange réactionnel sur silice (éluant : cyclohexane-acétate d'éthyle 35-65). On obtient 0,98 g de produit attendu.

**Exemple 7 : N-butyl-8-(4-(3,17béta-dihydroxy-estra-1,3,5(10)-trien-11béta-yl) phénoxy)-N-méthyl-2-octynamide.**

Stade A : N-butyl-8-[4-(3,17-dioxo-estra-4,9-dièn-11béta-yl) phénoxy] N-méthyl-2-octynamide.

A une solution de 1,09 g du produit obtenu au stade B de la préparation A de l'exemple 1 dans 20 $cm^3$ d'acétone et 1,5 $cm^3$ de soude 2N que l'on porte au reflux sous une atmosphère d'azote on introduit en 40 minutes une solution de 970 mg de produit obtenu au stade E ci-dessus dans 6 $cm^3$ d'acétone. On maintient au reflux pendant 6 heures,

refroidit et dilue avec une solution de chlorure d'ammonium. On extrait avec du chlorure de méthylène lave, sèche et évapore à sec sous pression réduite. On chromatographie le résidu (2,13 g) sur silice (éluant : cyclohexane-acétate d'éthyle 4-6) et obtient 1,21 g de produit attendu.

Spectre IR : ($CHCl_3$)

| | |
|---|---|
| C≡C | 2235 $cm^{-1}$ |
| C=O | 1735 $cm^{-1}$ (17-céto) |
| | 1658 $cm^{-1}$ (diénone) |
| | 1614 $cm^{-1}$ (amide) |
| aromatique | 1580, 1509 $cm^{-1}$ |

Stade B : N-butyl-8-(4-(3,17béta-dihydroxy-estra-1,3,5(10)-trièn-11béta-yl) phénoxy)-N-méthyl-2-octynamide.

A la solution de 830 mg de produit obtenu au stade A ci-dessus dans 8 $cm^3$ de chlorure de méthylène refroidie à 0°/+5°C et sous atmosphère d'azote on ajoute 0,8 $cm^3$ d'anhydride acétique et 0,4 $cm^3$ de bromure d'acétyle. On maintient 10 minutes à cette température puis 1 heure à température ambiante. On ajoute une solution saturée de bicarbonate de sodium, émulsionne, ajoute 3,5 ml de méthanol puis extrait avec du chlorure de méthylène. On recueille la phase organique, lave avec la solution de bicarbonate ci-dessus, sèche et évapore à sec sons pression réduite. On obtient 950 mg de produit que l'on utilise tel que en solution dans 10 $cm^3$ de méthanol et que l'on refroidit à 0° + 5°C. On ajoute 0,2 g d'hydrure de bore et de sodium. On agite pendant 45 minutes, ajoute 2 $cm^3$ d'eau et 1 ml de soude 2N. On maintient pendant 1 heure à température ambiante, dilue avec une solution saturée de chlorure d'ammonium, extrait avec du chlorure de méthylène. On recueille la phase organique, lave à l'eau, sèche et évapore à sec sous pression réduite. On chromatographie le résidu (850 mg) sur silice (éluant : cyclohexane-acétate d'éthyle 4/6) et obtient 438 mg de produit attendu.

| Analyse : $C_{37}H_{49}NO_4$ = 571,81 | | | |
|---|---|---|---|
| | C% | H% | N% |
| Calculé | 77,72 | 8,63 | 2,44 |
| Trouvé | 77,7 | 8,9 | 2,3 |

Spectre IR : ($CHCl_3$)

| | |
|---|---|
| OH | 3603 $cm^{-1}$ |
| C≡C | 2235 $cm^{-1}$ |
| C=O | 1612 $cm^{-1}$ (amide conjugué) |
| aromatique | 1581, 1511 $cm^{-1}$ |

**Préparation de l'exemple 8 : 2-[(5-bromopentyl) thio]-N-butyl-N-méthyl acétamide.**

Stade A : 5-mercaptopentanol.

A une suspension de thioacétate de potassium dans 100 $cm^3$ d'éthanol, on ajoute 10 $cm^3$ de 5-chloropentanol. Sous atmosphère d'azote, on maintient au reflux pendant 1 heure. On refroidit, ajoute 100 $cm^3$ d'éther éthylique, filtre et évapore à sec. On ajoute 100 $cm^3$ d'éther éthylique au résidu huileux obtenu. On filtre le précipité formé et évapore à sec la solution organique. On obtient 12,5 g de résidu huileux que l'on met en solution dans 50 $cm^3$ de méthanol. On ajoute 10 $cm^3$ de soude 10N et maintient pendant 1 heure à température ambiante. On ajoute une solution saturée de chlorure d'ammonium, extrait avec de l'acétate d'éthyle puis avec du chlorure de méthylène. On sèche les phases organiques, évapore à sec sous pression réduite. On obtient 9,3 g de résidu huileux que l'on met en solution dans 50 $cm^3$ de méthanol à 10 % d'eau, sous barbotage d'azote pendant 15 minutes. On ajoute lentement en 2 minutes 5 $cm^3$ de n-

tributylphosphine et maintient pendant 2 h 30 à température ambiante. On extrait avec de l'acétate d'éthyle, sèche et évapore à sec sous pression réduite. On chromatographie le résidu (13,6 g) sur silice (éluant : cyclohexane-acétate d'éthyle 1-1) et obtient 6,55 g de produit attendu.

Spectre IR : ($CHCl_3$) sur produit purifié par distillation (Eb : 90/95°C sous 10 mm Hg)

OH 3624 $cm^{-1}$
SH 2570 $cm^{-1}$

Stade B : N-butyl-2-[(5-hydroxypentyl) thio]-N-méthyl acétamide.

A une solution de 3,36 g de produit obtenu au stade A ci-dessus dans 45 $cm^3$ de tétrahydrofuranne sous atmosphère d'azote, on ajoute 1,4 g d'hydrure de sodium à 50 % dans l'huile et 6 g de bromo N-butyl N-méthyl acétamide obtenu au stade A de la préparation B de l'exemple 1. On agite pendant 2 heures à température ambiante, verse dans une solution de chlorure d'ammonium, extrait avec de l'acétate d'éthyle, lave, évapore à sec sous pression réduite. On chromatographie le résidu (10 g) sur silice (éluant : cyclohexane-acétate d'éthyle 2-8) et obtient 5,27 g de produit attendu.

Spectre IR : ($CHCl_3$)

OH 3623 $cm^{-1}$
C=O 1632 $cm^{-1}$ (amide III)

Stade C : 2-[(5-bromopentyl) thio]-N-butyl-N-méthyl acétamide.

A une solution de 5,27 g de produit obtenu au stade B ci-dessus dans 52 $cm^3$ de chlorure de méthylène on ajoute 6,88 g de triphénylphosphine. Sous atmosphère d'azote on refroidit à 0°/+5°C, ajoute 8,68 g de tétrabromo méthane, agite pendant 1 heure à 0°C, chromatographie le mélange réactionnel sur silice (éluant : cyclohexane-acétate d'éthyle 2-1) et obtient 6,2 g de produit attendu.

Spectre IR : ($CHCl_3$)

C=O 1638 $cm^{-1}$ (amide III)

**Exemple 8 : N-butyl-2((5-(4-(3,17béta-dihydroxy-estra-1,3,5(10)-trien-11béta-yl) phénoxy) pentyl) sulfinyl)-N-méthyl-acétamide.**

Stade A : N-butyl-2-[5-[4(3,17-dioxo-estra-4,9-dien-11béta-yl) phénoxy] pentylthio]-N-méthylacétamide.

A une solution de 3,09 g de produit obtenu au stade B de la préparation A de l'exemple 1 dans 45 $cm^3$ d'acétone on ajoute 5,1 $cm^3$ de soude 2N, chauffe au reflux sous atmosphère d'azote et ajoute en 4 fractions pendant 2 heures 6,2 g de produit obtenu au stade D ci-dessus. On maintient au reflux pendant 1 h 20, refroidit, dilue avec une solution de chlorure d'ammonium saturée. On extrait avec de l'acétate d'éthyle, lave, sèche, évapore à sec sous pression réduite. On chromatographie le résidu (10 g) sur silice (éluant : essence G-acétate d'éthyle 4-6) et obtient 3,4 g de produit attendu.

Spectre IR : ($CHCl_3$)

C=O
1735 $cm^{-1}$ (17-céto)
1656 $cm^{-1}$ (diénone)
1640 $cm^{-1}$ (amide III)

| | |
|---|---|
| C=C aromatique | 1610, 1580, 1509 $cm^{-1}$ |

Stade B : N-butyl-[5-[4-(3-hydroxy-17-oxo-estra-1,3,5(10)-trièn-11béta-yl) phénoxy] pentylthio]-N-méthylacétamide.

On opère comme au stade B de l'exemple 5 en partant de 2,6 g de produit obtenu au stade A ci-dessus dans 26 $cm^3$ de chlorure de méthylène sous atmosphère d'azote et en ajoutant 2,6 $cm^3$ d'anhydride acétique et 1,3 $cm^3$ de bromure d'acétyle. Après chromatographie sur silice on obtient 1,6 g de produit attendu.

Spectre IR : ($CHCl_3$)

| | |
|---|---|
| OH | 3598 $cm^{-1}$ |
| C=O | 1732 $cm^{-1}$ (17-céto) |
| | 1627 $cm^{-1}$ (amide III) |
| aromatique | 1581, 1511 $cm^{-1}$ |

Stade C : N-butyl-[5-[4-(3,17béta-dihydroxy estra-1,3,5(10)-trièn-11béta-yl) phénoxy] pentylthio] N-méthylacétamide.

On opère comme au stade C de l'exemple 5 en partant de 600 mg de produit obtenu au stade B ci-dessus et 60 mg d'hydrure de bore et de sodium. On obtient le produit attendu.

Spectre IR : ($CHCl_3$)

| | |
|---|---|
| OH | 3603 $cm^{-1}$ (+ associé) |
| C=O | 1627 $cm^{-1}$ |
| aromatique | 1581, 1511 $cm^{-1}$ |

$[alpha]_D$ = -32°5 (C = 0,1 % $CHCl_3$)

Stade D : N-butyl-2[[5-[4-(3,17béta-dihydroxy-estra-1,3,5(10)-trièn-11béta-yl) phénoxy] pentyl] sulfinyl]-N-méthylacétamide.

A une solution de 147 mg de produit obtenu au stade C ci-dessus dans 10 $cm^3$ de méthanol on ajoute 4 $cm^3$ d'une solution de périodate de sodium 0,1 M. On chauffe au reflux pendant 40 minutes, refroidit, dilue avec de l'eau, extrait avec du chloroforme et évapore à sec sous pression réduite. On chromatographie le résidu (180 mg) sur silice (éluant : acétate d'éthyle-acétone 1-1) et obtient 150 mg de produit attendu.

| Analyse : $C_{36}H_{51}NO_5S$ = 609,88 | | | | |
|---|---|---|---|---|
| | C% | H% | N% | S% |
| Calculé | 70,90 | 8,42 | 2,29 | 5,27 |
| Trouvé | 71,0 | 8,6 | 2,3 | 5,1 |

Spectre IR : ($CHCl_3$)

| | |
|---|---|
| OH | 3604 $cm^{-1}$ (+ associé) |

| | |
|---|---|
| C=O | 1637 $cm^{-1}$ |
| aromatique | 1611, 1581, 1511 $cm^{-1}$ |

**<u>Préparation A de l'exemple 9 :</u> 3,17béta-bis[tétrahydro-2H-2-pyrannyl) oxy] (4-éthynyl phényl) estra-1,3,5(10)-trièn-11béta-yl.**

<u>Stade A :</u> (5alpha, 11béta) 3-(1,2-éthanediyl acétal cyclique) 5-hydroxy 11-[[4-(1,1-diméthyléthyl) diméthylsilyl] éthynyl-phényl] estr-9-èn-3,17-dione.

On opère comme au stade A de la préparation A de l'exemple 1 à partir de 30 g de 3-(1,2-éthanediyl acétal cyclique de 5alpha, 10alpha-époxy estr-9,11-ène-3,7-dione obtenu selon EP 0 057 115 (exemple 7) en utilisant pour la préparation du magnésien 81,254 g de dérivé bromé, 4-triméthylsilyléthynyl-bromobenzène obtenu à la préparation 4 et 7,96 g de magnésium, puis pour la condensation, 1,4 g de chlorure de cuivre. On chromatographie sur silice le produit brut obtenu auquel on joint le produit provenant d'une opération réalisée de façon identique à partir de 16,52 g d'époxyde (éluant : chlorure de méthylène-acétone 98-2). On obtient 50,8 g de produit A pur et 6 g de produit B légèrement moins pur que l'on utilise tel quel pour le stade suivant.

<u>Spectre IR</u> : ($CHCl_3$)

| | |
|---|---|
| OH | 3508 $cm^{-1}$ |
| C=C | 2156 $cm^{-1}$ |
| | 1602 $cm^{-1}$ |
| aromatiques | 1555 $cm^{-1}$ |
| | 1502 $cm^{-1}$ |

<u>Stade B :</u> 11béta-(4-éthynylphényl) estra-4,9-dièn-3,17-dione.

On agite pendant 30 minutes une suspension de 46,8 g du produit obtenu au stade A, 200 $cm^3$ d'éthanol et 8,1 $cm^3$ de lessive de soude. On ajoute ensuite 16,7 $cm^3$ d'acide chlorhydrique concentré, on agite à température ambiante puis concentre au demi, extrait avec du chlorure de méthylène, sèche et évapore sous pression réduite. On chromatographie le résidu (38,23 g) sur silice (éluant : cyclohexane-acétate d'éthyle 1-1) et obtient 31,06 g de produit recherché. F = 184°C.

<u>Spectre IR</u> : ($CHCl_3$)

| | |
|---|---|
| C≡CH | 3302 $cm^{-1}$ |
| C=O | 1736 $cm^{-1}$ (17-céto) |
| | 1659 $cm^{-1}$ |
| | 1640 $cm^{-1}$ diénone |
| aromatique | 1556, 1506 $cm^{-1}$ |

<u>Stade C :</u> 3béta-hydroxy estra 1,3,5(10)-trièn-11béta-yl (4-éthynylphényl) 17-one.

A une solution de 31 g de produit obtenu au stade B en solution dans 340 ml de chlorure de méthylène, on ajoute à 0°/-5°C, 47,1 $cm^3$ d'anhydride acétique et 23,8 $cm^3$ de bromure d'acétyle. On agite 1 h 30 à température ambiante verse sur un mélange de 2 litres de solution saturée de bicarbonate de sodium et 700 g de glace. On agite 15 minutes puis extrait avec du chlorure de méthylène, évapore à sec sous pression réduite d'azote. On chromatographie le résidu sur silice (éluant : chlorure de méthylène-acétone 9-1). On obtient 31,2 g d'acétate en 3 que l'on agite pendant 1 heure

dans 930 $cm^3$ de méthanol et 20,96 g de potasse. On refroidit à 0°C, neutralise avec de l'acide chlorhydrique 2N, extrait avec du chlorure de méthylène et évapore à sec sous pression réduite. Après chromatographie sur silice (éluant : cyclohexane-acétate d'éthyle 7-3), on obtient 27,03 g de produit brut que l'on empâte dans l'éther pour recueillir 22,85 g du produit recherché. F = 163°C.

Spectre IR : ($CHCl_3$)

| | |
|---|---|
| OH | 3597 $cm^{-1}$ |
| C≡CH | 3303 $cm^{-1}$ |
| C=O | 1733 $cm^{-1}$ |
| aromatique | 1606, 1582, 1556, 1503 $cm^{-1}$ |

Stade D : 3,17béta-bis[(tétrahydro-2H-2-pyrannyl) oxy] (4-éthynylphényl) estra-1,3,5(10)-trièn-11béta-yl.

a) Réduction de la cétone en 17.

A une solution de 14 g de produit obtenu au stade C dans 120 $cm^3$ de méthanol, on ajoute à 0°/+5°C, 10 g d'hydrure de bore et de sodium, on agite pendant 3 heures à température ambiante. On ajoute 380 g de mélange eau-glace (1-1) puis ajuste le pH entre 4 et 5 avec de l'acide chlorhydrique 2N, ajoute du chlorure de sodium jusqu'à saturation et extrait avec de l'acétate d'éthyle. On évapore à sec le résidu. On isole 17,3 g de produit brut.

b) Dihydropyranylation

On agite pendant 2 h 30 17,3 g de l'intermédiaire 17-hydroxy obtenu ci-dessus en présence de 700 $cm^3$ d'éther 34,4 $cm^3$ de dihydropyranne et 0,3 g d'acide paratoluène sulfonique. On ajoute 35 $cm^3$ de triéthylamine, verse dans un mélange (1-1) de glace et de solution saturée de bicarbonate de sodium. On extrait à l'éther, filtre et évapore à sec sous pression réduite d'azote. On chromatographie le résidu sur silice (éluant : cyclohexane-acétate d'éthyle 1-1). On obtient 13,6 g de produit brut que l'on reprend dans l'éther isopropylique pour recueillir 10,23 g de produit recherché. F = 213-215°C.

Spectre IR : ($CHCl_3$)

| | |
|---|---|
| C≡CH | 3302 $cm^{-1}$ |
| aromatique | 1607, 1570, 1556, 1498 $cm^{-1}$ |

**Préparation B de l'exemple 9 : 5-[4-[3,17béta-bis [(tétrahydro-2H-2-pyrannyl) oxy]-estra-1,3,5(10)-trièn-11béta-yl] phényl]-4-pentyl-1-ol.**

Stade A : 3,17béta-bis [(tétrahydro-2H-2-pyrannyl) oxy]-11béta-[4-5[[diméthyl(11-diméthyl éthyl) silyl] oxy] 1-pentynyl] phényl]-estra-1,3,5(10)-triène.

A une solution de 714 mg de produit obtenu au stade D de la préparation A ci-dessus dans 5,9 $cm^3$ de tétrahydrofuranne, on ajoute 5,9 $cm^3$ d'hexaméthylphosphorotriamide. On ajoute en 5 minutes à -35°C 1,5 $cm^3$ de solution de butyl lithium 1,32 M dans l'hexane, puis 669 mg de produit bromé obtenu au stade B de la préparation 3. On agite pendant 1 heure à température ambiante, puis verse dans une solution aqueuse saturée de chlorure de sodium. On extrait à l'acétate d'éthyle, lave avec une solution saturée de chlorure de sodium, sèche puis évapore à sec sous pression réduite. On dissout le résidu obtenu dans de l'eau puis extrait avec de l'éther éthylique, lave à l'eau, sèche et évapore à sec sous pression réduite. On chromatographie le résidu obtenu (1,198 g) sur silice (éluant : acétate d'éthyle-cyclohexane 1-9 à 0,1 % de triéthylamine) et obtient 941 mg de produit intermédiaire attendu.

Stade B : 5-[4-[3,17béta-bis[(tétrahydro-2H-2-pyrannyl) oxy]-estra-1,3,5(10)-trien-11béta-yl] phényl]-4-pentyn-1-ol.

A une solution de 931 mg de produit obtenu au stade A ci-dessus en solution dans 10 $cm^3$ de tétrahydrofuranne, on ajoute 1,4 $cm^3$ de solution de fluorure de tétrabutylammonium 1M. On agite pendant 1 h 45 à température ambiante puis verse dans de l'eau, extrait avec de l'acétate d'éthyle, lave avec une solution saturée de chlorure de sodium, sèche et évapore à sec sous pression réduite. On chromatographie le résidu (755 mg) sur silice (éluant : acétate d'éthyle-cyclohexane 4-6 à 0,1 % de triéthylamine). On obtient 408 mg de produit attendu.

Spectre IR : ($CHCl_3$)

OH 3640 $cm^{-1}$
aromatique 1500, 1610 $cm^{-1}$

**Préparation C de l'exemple 9 : N-méthyl-N-butyl mercaptoacétamide.**

Stade A : 2-(acétylthio)-N-butyl-N-méthylacétamide.

A une suspension de 2,28 g de thioacétate de potassium dans 10 $cm^3$ de méthanol, on ajoute 4,16 g de bromo-N-butyl-N-méthylacétamide obtenu à la préparation B de l'exemple 1 stade A dans 1,5 $cm^3$ de méthanol, chauffe pendant 1 heure à 40°C sous atmosphère d'argon. On évapore à sec sous pression réduite, dissout le résidu avec de l'éther éthylique, filtre insoluble et évapore à sec la solution. On obtient 4,06 g de produit huileux.

Spectre IR : ($CHCl_3$)

C=O 1644 $cm^{-1}$ (amide)
acétate 1700 $cm^{-1}$

Stade B : N-méthyl-N-butyl mercaptoacétamide.

A une solution de 3,99 g de produit obtenu au stade A ci-dessus dans 10 $cm^3$ de tétrahydrofuranne, on ajoute 1 $cm^3$ d'hydrate d'hydrazine, agite pendant 16 heures à température ambiante, ajoute 0,5 $cm^3$ d'hydrate d'hydrazine, agite 16 heures à température ambiante et verse dans un mélange de 40 $cm^3$ d'acide chlorhydrique 2N et 40 g de glace. On extrait avec de l'acétate d'éthyle, lave avec une solution saturée de chlorure de sodium, sèche, évapore à sec sous pression réduite. On chromatographie le résidu (3,13 g) sur silice (éluant : acétate d'éthyle-éther éthylique 7-3) et obtient 79,5 mg de produit attendu.

**Exemple 9 : N-butyl-2-((5-(4-(3,17béta-dihydroxy-estra-1,3,5(10)-trien-11béta-yl) phényl) pentyl) thio)-N-méthyl-acétamide.**

Stade A : 2-[[5-[4-[3,17béta-bis[(tétrahydro-2H-2-pyrannyl) oxy]-estra-1,3,5(10)-trièn-11béta-yl] phényl]-4-pentynyl] thio]-N-butyl-N-méthyl-acétamide.

A une solution de 408 mg de produit obtenu au stade B de la préparation B ci-dessus on ajoute 1,5 $cm^3$ de pyridine et 259 mg de chlorure de tosyle. On agite pendant 4 heures à température ambiante puis verse dans une solution aqueuse saturée de bicarbonate de potassium, extrait avec de l'acétate d'éthyle, lave avec une solution saturée de chlorure de sodium, sèche et évapore à sec sous pression réduite. On dissout le produit brut tosylé obtenu (512 mg) dans 4,5 ml de méthanol et ajoute 165 mg de N-méthyl-N-butyl mercapto-acétamide obtenu à la préparation C ci-dessus et 0,94 $cm^3$ d'une solution de méthylate de sodium à 58,4 mg/$cm^3$ dans le méthanol. On maintient au reflux jusqu'à réaction totale, évapore le méthanol sous pression réduite. On ajoute de l'eau, extrait avec de l'acétate d'éthyle, lave avec une solution aqueuse saturée de chlorure de sodium. On sèche puis évapore à sec sous pression réduite. On chromatographie le résidu (677 mg) sur silice (éluant : acétate d'éthyle-cyclohexane 4-6 à 0,1 % de triéthylamine) et obtient 315 mg de produit attendu.

Spectre IR : ($CHCl_3$)

C=O 1640 $cm^{-1}$ (amide III)
aromatique 1500, 1580 $cm^{-1}$

Stade B : N-butyl-2-[[5-[4-[3,17bèta-dihydroxy-estra-1,3,5(10)-trien-11béta-yl] phényl]-4-pentynyl] thio]-N-méthyl-acétamide.

A une solution de 315 g de produit obtenu au stade A ci-dessus, on ajoute 16 $cm^3$ de méthanol, puis 3,2 $cm^3$ d'acide chlorhydrique 2N. On agite pendant 1 heure à température ambiante, puis verse dans une solution aqueuse saturée de chlorure de sodium. On extrait avec du chlorure de méthylène, lave, sèche puis évapore à sec sous pression réduite. On obtient 225 mg de produit attendu.

Spectre IR : ($CHCl_3$)

| | |
|---|---|
| OH | 3610 $cm^{-1}$ |
| C=O | 1640 $cm^{-1}$ (amide III) |
| aromatique | 1500, 1590 $cm^{-1}$ |

Stade C : N-butyl-2-((5-(4-(3,17béta-dihydroxy-estra-1,3,5(10)-trièn-11béta-yl) phényl) pentyl) thio)-N-méthylacétamide.

A une solution de 222 mg de produit obtenu au stade B ci-dessus dans 2,2 $cm^3$ de méthanol et 2,2 $cm^3$ de toluène, on ajoute 90 mg de chloro(tris triphénylphosphine) rhodium. On hydrogène sous 1885 mbars pendant 18 heures. On ajoute à nouveau 22,5 mg de chloro(tris triphénylphosphine) rhodium, 0,7 $cm^3$ d'éthanol et 0,7 $cm^3$ de toluène. On hydrogène jusqu'à obtention du palier. On évapore à sec sous pression réduite. On chromatographie le résidu (333 mg) sur silice (éluant : acétate d'éthyle-cyclohexane 7-3) et obtient 103 mg de produit attendu.

| Analyse : $C_{36}H_{51}NO_3S$ = 577,88 | | | | |
|---|---|---|---|---|
| | C% | H% | S% | N% |
| Calculé | 74,82 | 8,89 | 5,55 | 2,42 |
| Trouvé | 74,4 | 8,8 | 6,0 | 2,7 |

Spectre IR : ($CHCl_3$)

| | |
|---|---|
| OH | 3608 $cm^{-1}$ (+ associé) |
| C=O | 1625 $cm^{-1}$ (amide III) |
| aromatique | 1583, 1499 $cm^{-1}$ |

**Préparation de l'exemple 10 : 7-bromo-N-butyl N-méthylheptanamide.**

On opère comme à la préparation de l'exemple 2 en partant de 707 mg d'acide 7-bromoheptanoïque, obtenu par agitation pendant 3 heures de 3 g de 7-bromoheptanoate d'éthyle dans 28 $cm^3$ de soude 2N et 10 $cm^3$ d'éthanol puis extraction à pH=1 avec de l'éther éthylique et évaporation à sec sous vide, de 1,4 $cm^3$ de N-méthylmorpholine, 1,69 $cm^3$ de chloroformiate d'isobutyle, puis 2,1 $cm^3$ de N-méthylbutylamine. On isole 3,124 g de résidu huileux que l'on distille sous pression réduite et obtient 676 mg de produit attendu sous forme d'huile.

Spectre IR : ($CHCl_3$)

C=O 1630 $cm^{-1}$ (amide III)

**Exemple 10 : N-butyl-4-(3,17béta-dihydroxy-estra-1,3,5(10)-trien-11béta-yl)-N-méthyl-benzenenonamide.**

Stade A : 9-[4-[3,17béta-bis[(tétrahydro-2H-2-pyrannyl) oxy]-estra-1,3,5(10)-trien-11béta-yl] phényl]-N-butyl-N-méthyl-8-nonynamide.

A une solution de 0,6 g de 3,17béta-bis[(tétrahydro-2H-2-pyrannyl) oxy] (4-éthynylphényl) estra-1,3,5(10)-trièn-11béta-yl obtenu au stade D de la préparation A de l'exemple 9 dans 5 $cm^3$ de tétrahydrofuranne on ajoute à -30°C 5 $cm^3$ d'hexaméthylphosphorotriamide puis lentement 1,22 $cm^3$ d'une solution de butyl lithium 1,1 M dans l'hexane. On agite à -30°C pendant 30 minutes puis ajoute 0,401 g de produit bromé obtenu à la préparation ci-dessus. On agite pendant 1 h 30 à température ambiante puis verse dans 20 $cm^3$ d'une solution aqueuse de phosphate mono sodique saturée. On extrait avec du chlorure de méthylène, sèche, évapore à sec sous pression réduite. On chromatographie le résidu (5,4 g) sur silice (éluant : cyclohexane-acétate d'éthyle 1-1) et obtient 0,67 g de produit attendu.

Spectre IR : ($CHCl_3$)

| | |
|---|---|
| C=O | 1626 $cm^{-1}$ (amide III) |
| aromatique | 1580, 1550, 1497 $cm^{-1}$ |

Stade B : N-butyl-4-(3,17béta-dihydroxy-estra-1,3,5(10)-trièn-11béta-yl) N-méthyl-benzènenonamide.

A une solution de 0,640 g de produit obtenu au stade A ci-dessus dans 20 $cm^3$ d'éthanol on ajoute 10 $cm^3$ d'acide chlorhydrique 2N puis 20 $cm^3$ d'eau. On extrait avec du chlorure de méthylène. On sèche et évapore à sec sous pression réduite et isole 0,494 mg de résidu.

A une solution de 0,26 g de produit brut ci-dessus dans 20 $cm^3$ d'éthanol on ajoute 30 mg de palladium sur charbon et hydrogène sous 1120 mbars pendant 20 minutes. On filtre, lave à l'éthanol et évapore à sec la solution sous pression réduite. On chromatographie le résidu (0,272 g) sur silice (éluant : cyclohexane-acétate d'éthyle 1-1) et obtient 0,198 mg de produit attendu.

| Analyse : $C_{38}H_{55}NO_3$ = 573,87 | | | |
|---|---|---|---|
| | C% | H% | N% |
| Calculé | 79,53 | 9,66 | 2,44 |
| Trouvé | 79,8 | 9,8 | 2,4 |

Spectre IR : ($CHCl_3$)

OH 3603 $cm^{-1}$ + associé
C=O 1622 $cm^{-1}$
aromatique 1582, 1429 $cm^{-1}$

**Préparation de l'exemple 11 : [(3-bromopropyl) oxy]-N-butyl-N-méthylacétamide.**

Stade A : N-butyl [(3-hydroxypropyl) oxy]-N-méthylacétamide.

On opère comme au stade B de la préparation de l'exemple 6 en partant de 2,5 g de 3-[[(diméthyl (1,1-diméthyléthyl) silyl] oxy] propanol obtenu au stade A de la préparation 3, 775 mg d'hydrure de sodium à 50 % dans l'huile et 3,42 g de bromo-N-butyl-N-méthylacétamide. On obtient 4,92 g de résidu que l'on traite avec 16,4 $cm^3$ d'une solution de fluorure de tétrabutyl ammonium. Après chromatographie on obtient 1,61 g de produit attendu.

| Analyse : $C_{10}H_{21}O_3N$ = 202,283 | | | |
|---|---|---|---|
| | C% | H% | N% |
| Calculé | 59,08 | 10,41 | 6,89 |
| Trouvé | 58,5 | 10,6 | 6,4 |

Spectre IR : ($CHCl_3$)

OH 3620 $cm^{-1}$ (+ associé)
C=O 1645 $cm^{-1}$ (amide III)

Stade B : [(3-bromopropyl) oxy]-N-butyl-N-méthylacétamide.

On opère comme au stade C de la préparation de l'exemple 6 en partant de 1,56 g de produit obtenu au stade A ci-dessus, 3,18 g tétrabromométhane et 2,51 g de triphénylphosphine. Après chromatographie on obtient 1,356 g de produit attendu.

| Analyse : $C_{10}H_{20}BrNO_2$ = 266,18 | | | | |
|---|---|---|---|---|
| | C% | H% | N% | Br% |
| Calculé | 45,12 | 7,57 | 5,26 | 30,2 |
| Trouvé | 45,2 | 7,8 | 5,1 | 29,4 |

Spectre IR : ($CHCl_3$)

C=O 1646 $cm^{-1}$ (amide III)

**Exemple 11 : N-butyl-2-((5-(4-(3,17béta-dihydroxy-estra-1,3,5(10)-trien-11béta-yl) phényl) pentyl) oxy)-N-méthylacétamide.**

Stade A : N-butyl-N-méthyl-2-[[5-[4-[3,17béta-bis [(tétrahydro 2H-2-pyrannyl) oxy]-estra-1,3,5(10)-trièn-11béta-yl] phényl] 4-pentynyl] oxy]-acétamide.

On opère comme au stade A de l'exemple 9 en partant de 800 mg de produit obtenu au stade D de la préparation A de l'exemple 9, 6,5 $cm^3$ d'hexaméthyl phosphorotriamide et 1,9 $cm^3$ de solution de butyl lithium 1M puis 517 mg de produit obtenu au stade B de la préparation ci-dessus. On extrait au chlorure de méthylène et obtient 5,858 g de résidu intermédiaire. On chromatographie le résidu (1,142 g) sur silice (éluant : acétate d'éthyle-cyclohexane 1-1 à 0,1 % de triéthylamine) et obtient 478 mg de produit attendu.

Spectre IR : ($CHCl_3$)

C=O 1645 $cm^{-1}$ (amide III)
aromatique 1583, 1500 $cm^{-1}$

Stade B : N-butyl-2-[[5-[4-(3,17béta-dihydroxy-estra-1,3,5(10)-trièn-11béta-yl) phényl pentyl] oxy]-N-méthylacétamide.

On opère comme au stade B de l'exemple 10 en partant de 478 mg de produit obtenu au stade A ci-dessus en solution dans le méthanol puis hydrogène le résidu intermédiaire (356 mg) en présence de 178 mg de palladium sur charbon. Après chromatographie du résidu (317 mg) (éluant : acétate d'éthylecyclohexane 8-2), on obtient 199 mg de produit attendu.

| Analyse : $C_{36}H_{51}NO_4$ = 561,81 | | | |
|---|---|---|---|
| | C% | H% | N% |
| Calculé | 76,96 | 9,15 | 2,49 |
| Trouvé | 77,0 | 9,3 | 2,6 |

Spectre IR : ($CHCl_3$)

OH 3604 $cm^{-1}$ (+ associé)
C=O 1633 $cm^{-1}$ (amide III)
aromatique 1583, 1500 $cm^{-1}$

**Préparation de l'exemple 12 : 6-bromo hexanoate de sodium.**

A une solution de 14,60 g d'acide 6-bromohexanoïque dans 20 $cm^3$ de chlorure de méthylène on ajoute 75 $cm^3$ de soude N puis distille à 60°C. On lave le résidu avec du toluène et utilise le produit obtenu tel quel.

**Exemple 12** : **8-(4-(3,17béta-dihydroxy-estra-1,3,5(10)-trien-11béta-yl) phényl)-N-(2,2,3,3,4,4,4-heptafluorobutyl)-N-méthyl-7-octynamide.**

Stade A : acide 8-[4-[3,17béta-bis[(tétrahydro-2H-2-pyrannyl) oxy]-estra-1,3,5(10)-trièn-11béta-yl] phényl]-7-octynoïque.

A une solution de 17 g de 3,17béta-bis[(tétrahydro-2H-2-pyrannyl) oxy] (4-éthynyl phényl) estra-1,3,5(10)-trièn-11béta-yl obtenu au stade D de la préparation A de l'exemple 9 dans 60 $cm^3$ de tétrahydrofuranne on ajoute à -30°C 60 $cm^3$ d'hexaméthylphosphorotriamide puis 31,5 $cm^3$ de solution de butyl lithium 1M dans l'hexane. On agite pendant 30 minutes à -30°C puis ajoute 15,7 g de 6-bromohexanoate de sodium obtenu selon la préparation ci-dessus. On agite 5 heures à température ambiante, verse dans une solution de phosphate mono sodique à 0°C, extrait à l'éther éthylique. On évapore à sec et chromatographie le résidu (41 g) sur silice (éluant : cyclohexane-acétate d'éthyle 1-1) et obtient 10,163 g de produit attendu.

Spectre IR : ($CHCl_3$)

| | |
|---|---|
| C=O | 1709 $cm^{-1}$ |
| aromatique | 1607, 1572, 1497 $cm^{-1}$ |

Stade B : 8-[4-(3,17béta-dihydroxy estra-1,3,5(10)-trien-11béta-yl) phényl]-N-(2,2,3,3,4,4,4-heptafluorobutyl)-N-méthyl octynamide.

A une solution de 0,604 g de produit obtenu au stade A ci-dessus dans 10 $cm^3$ de chlorure de méthylène, on ajoute 0,31 $cm^3$ de N-méthylmorpholine puis 0,39 $cm^3$ de chloroformiate d'isobutyle. On agite pendant 10 minutes puis ajoute 0,826 g de chlorhydrate de N-heptafluorobutyl-N-méthylamine obtenu à la préparation 1. On agite pendant 30 minutes à température ambiante, ajoute 20 $cm^3$ de solution saturée de bicarbonate de sodium, extrait avec du chlorure de méthylène, sèche et évapore à sec sous pression réduite. On chromatographie le résidu (3,369 g) sur silice (éluant : chlorure de méthylène-acétone 9-1). On obtient 0,782 mg de produit intermédiaire que l'on met en solution dans 10 $cm^3$ d'acide chlorhydrique 2N en présence de 10 $cm^3$ d'éthanol et 2 $cm^3$ de chlorure de méthylène. On concentre 2 fois par distillation sous pression réduite, ajoute de l'eau, extrait avec du chlorure de méthylène, sèche et évapore à sec sous pression réduite. On chromatographie le résidu (1,403 g) sur silice (éluant : cyclohexane-acétate d'éthyle 1-1) et obtient 0,440 g de produit attendu sous forme d'huile.
$[alpha]_D$ = 28°5 ± 2°5 (C = 0,45 % éthanol)

| Analyse : $C_{37}H_{42}F_7NO_3$ = 681,74 | | | | |
|---|---|---|---|---|
| | C% | H% | F% | N% |
| Calculé | 65,19 | 6,21 | 19,51 | 2,05 |
| Trouvé | 65,0 | 6,3 | 19,1 | 2,0 |

Spectre IR : ($CHCl_3$) absence de C C

| | |
|---|---|
| OH | 3602 $cm^{-1}$ (+ associé) |
| C=O | 1656 $cm^{-1}$ |
| aromatique | 1584, 1505, 1483 $cm^{-1}$ |

**Exemple 13 : 1-(8-(4-(3,17béta-dihydroxy-estra-1,3,5(10)-trien-11béta-yl) phényl)-1-oxo-7-octynyl)-4-(phénylméthyl)-pipéridine.**

On opère comme au stade B de l'exemple 12 en partant de 1 g d'acide 8-[4-[3,17béta-bis [(tétrahydro-2H-2-pyrannyl) oxy]-estra-1,3,5(10)-trien-11béta-yl] phényl]-7-octynoïque, 0,7 $cm^3$ de chloroformiate d'isobutyle et 0,8 $cm^3$ de 4-benzylpipéridine. On chromatographie le résidu (2,4 g) sur silice (éluant : chlorure de méthylène-acétone 95-5) et obtient 1,01 g de produit intermédiaire que l'on dépyrannyle. Après chromatographie sur silice (éluant : chlorure de méthylèneacétone 9-1), on obtient 0,462 g de produit attendu. $[alpha]_D$ = -38° ± 2°5 (C = 0,5 % éthanol)

| Analyse : $C_{44}H_{53}NO_3$ = 643,92 | | | |
|---|---|---|---|
| | C% | H% | N% |
| Calculé | 82,07 | 8,30 | 2,18 |
| Trouvé | 81,9 | 8,5 | 1,9 |

Spectre IR : ($CHCl_3$)

| | |
|---|---|
| OH | 3602 $cm^{-1}$ (+ associé) |
| C=O | 1625 $cm^{-1}$ (amide III) |
| aromatique | 1585, 1555, 1504 $cm^{-1}$ |

**Préparation A de l'exemple 14 : bromo-N-méthyl N-(1-méthyl éthyl) acétamide.**

A une solution de 10 $cm^3$ de bromure de bromo acétyle dans 150 $cm^3$ d'éther refroidie à -20°C, on ajoute une solution de 26 $cm^3$ de méthylisopropylamine dans 100 $cm^3$ d'éther. On laisse revenir à 20°C et agite 30 minutes à 20°C. On dilue à l'eau, décante, extrait à l'éther, sèche et sépare par distillation. On obtient 13 g de produit attendu. Eb : 71/72°C sous 1 mmHg.

**Préparation B de l'exemple 14 : 11béta-(8-hydroxy octyl) estra-4,9-dièn-3,17dione.**

Stade A : 3-(1,2-éthanediyl) acétal cyclique de 11béta-[8-diméthyl (1,1-diméthyléthyl) silyloxy] octyl 5alpha-hydroxy estr-9-èn-3,17-dione.

On opère comme au stade A de la préparation A de l'exemple 9 à partir de 3,96 g de 3-(1,2-éthanediyl acétal cyclique) de 5alpha, 10alpha-époxy estr-9,11-èn-3,17-dione obtenu selon EP 0057115 (ex. 7) en utilisant pour la préparation du magnésien 5,4 g de [(8-bromo octyl) oxy] diméthyl (1,1-diméthyléthyl) silane obtenu à la préparation 5 et 1 g de magnésium en tournures, puis pour la condensation 0,4 g de chlorure de cuivre. Après chromatographie sur Lichrosorb Rp18 (éluant : méthanol-eau 9-1), on obtient 3,85 g du composé attendu utilisé tel quel pour le stade suivant.

Stade B : 11béta-(8-hydroxy octyl) estra-4,9-dièn-3,17-dione.

On agite pendant 1 h 15 à température ambiante 1,77 g de produit obtenu au stade A ci-dessus, 35 $cm^3$ de méthanol et 8,85 $cm^3$ d'acide chlorhydrique 2N. On alcalinise à pH environ 9 avec de l'ammoniaque concentré puis évapore à sec sous pression réduite. On reprend le résidu avec de l'acétate d'éthyle, lave avec une solution saturée de chlorure de sodium, sèche et évapore à sec sous pression réduite. Après chromatographie sur silice (éluant : chlorure de méthylène-acétate d'éthyle 1-1), on obtient 1,08 g du composé recherché.

Spectre IR : ($CHCl_3$)

| | |
|---|---|
| OH | 3624 $cm^{-1}$ |
| C=O | 1735 $cm^{-1}$ (17-céto) |
| | 1656, 1602 $cm^{-1}$ (diénone) |

**Exemple 14 : 2-((8-(3,17béta-dihydroxy19-nor-17alpha-pregna-1,3,5(10)-trien-20-yn-11béta-yl) octyl) oxy)-N-méthyl-N-(1-méthyléthyl)-acétamide.**

Stade A : [[8-(3,17-dioxo estra-4,9-dièn-11béta-yl) octyl] oxy] N-méthyl N-(1-méthyléthyl) acétamide.

A une solution de 570 mg du composé obtenu à la préparation ci-dessus dans 10 $cm^3$ de tétrahydrofuranne, on ajoute 1,4 $cm^3$ de bromo-N-méthyl N-(1-méthyléthyl) acétamide (obtenu à la préparation A ci-dessus et 285 mg

d'iodure de sodium puis 140 mg d'hydrure de sodium à 50 % dans l'huile. On agite 1 heure, verse dans une solution d'acide chlorhydrique N à 0°C et extrait avec du chlorure de méthylène. On évapore à sec et chromatographie le résidu (2 g) sur silice (éluant : acétate d'éthyle-éther 8-2). On obtient 350 mg du produit recherché.

Spectre IR : ($CHCl_3$)

17-céto 1736 $cm^{-1}$
3-céto 1655 $cm^{-1}$
amide III 1628 $cm^{-1}$
C=C 1603 $cm^{-1}$

Stade B : 2-[[8-(3-hydroxy-17-oxo-estra-1,3,5(10)-trièn-11béta-yl) octyl] oxy]-N-méthyl-N-(1-méthyléthyl) acétamide.

A une solution de 9,4 g de produit obtenu au stade A ci-dessus dans 195 $cm^3$ de chlorure de méthylène placé sous atmosphère d'azote, on ajoute à 0°C 10 $cm^3$ d'anhydride acétique et 5 $cm^3$ de bromure d'acétyle. On agite pendant 5 minutes à 0°C puis pendant 2 h 30 à température ambiante. On refroidit à 0°/+5°C, ajoute lentement de l'eau puis du méthanol, agite 5 minutes et ajoute 50 $cm^3$ de soude 2N. On recueille la phase organique, extrait à nouveau avec du chlorure de méthylène, lave, sèche et évapore à sec sous pression réduite. On obtient 10 g de résidu acétylé que l'on dissout dans 100 $cm^3$ de méthanol sous atmosphère d'azote. On ajoute 20 $cm^3$ de soude 2N et laisse réagir pendant 1 heure à température ambiante. On acidifie avec de l'acide chlorhydrique 2N, extrait avec du chlorure de méthylène, lave, sèche et évapore à sec sous pression réduite. On chromatographie le résidu (9 g) sur silice (éluant : cyclohexane-acétate d'éthyle 3-7) et obtient 7,9 g de produit attendu.

Stade D : 2-[[8-(3,17béta-dihydroxy 19-nor-17alpha-pregna-1,3,5(10)-trièn-20-yn-11béta-yl) octyl] oxy]-N-méthyl-N-(1-méthyléthyl)-acétamide. méthyléthyl)-acétamide.

Dans un ballon placé sous atmosphère d'azote, on obtient une suspension d'acétylure de potassium en partant de 3 $cm^3$ de solution 0,88 M de terbutylate de potassium dans le tétrahydrofuranne dans laquelle on ajoute 5 $cm^3$ de tétrahydrofuranne puis fait barboter de l'acétylène pendant 15 minutes. On ajoute une solution de 520 mg de produit obtenu au stade C ci-dessus dans 5 $cm^3$ de tétrahydrofuranne. Après réaction pendant 30 minutes à température ambiante, on ajoute une solution aqueuse de chlorure d'ammonium saturée, extrait avec de l'acétate d'éthyle, lave, sèche et évapore à sec sous pression réduite. On chromatographie le résidu (550 mg) sur silice (éluant : méthanol-eau 85-15) et obtient 470 mg de produit attendu.

| Analyse : $C_{34}H_{51}NO_4$ = 537,79 | | | |
|---|---|---|---|
| | C% | H% | N% |
| Calculé | 75,93 | 9,55 | 2,60 |
| Trouvé | 75,8 | 9,7 | 2,30 |

Spectre IR : ($CHCl_3$)

OH 3600 $cm^{-1}$
C≡CH 3305 $cm^{-1}$
C=O 1625 $cm^{-1}$ (amide III)
aromatique 1583, 1499 $cm^{-1}$

**Préparation A de l'exemple 15 : 2-[(4-bromophényl) thio] tétrahydro-2H-pyranne.**

A une solution de 1 g de 4-bromo thiophénol dans 1 $cm^3$ de 3,4-dihydro-[2H]-pyranne et 1 $cm^3$ de tétrahydrofuranne, on ajoute 2,3 mg d'acide paratoluène sulfonique, puis agite pendant 20 heures à température ambiante. On ajoute 0,1 $cm^3$ de triéthylamine, verse dans une solution saturée de bicarbonate de sodium, extrait avec de l'acétate d'éthyle, lave avec une solution aqueuse saturée de chlorure de sodium, sèche et évapore à sec sous pression réduite. On chromatographie le résidu (1,603 g) sur silice (éluant : cyclohexane-acétate d'éthyle 97,5-2,5) et obtient 1,246 g de produit attendu.

| Analyse : $C_{11}H_{13}BrOS$ = 273,19 | | | | |
|---|---|---|---|---|
| | C% | H% | Br% | S% |
| Calculé | 48,36 | 4,79 | 29,24 | 11,73 |
| Trouvé | 47,9 | 4,6 | 28,8 | 11,6 |

Spectre IR : ($CHCl_3$)

aromatique 1477 $cm^{-1}$ type 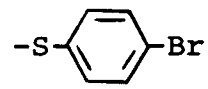

présence de 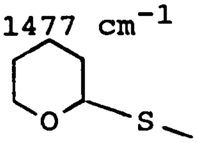

**Préparation B de l'exemple 15 : 11béta-[4-[(tétrahydro-2H-2-pyrannyl) thio] phényl]-estra-4,9-dièn-3,17-dione.**

a) préparation du magnésien

A une suspension de 139 mg de magnésium en poudre dans 0,65 $cm^3$ de tétrahydrofuranne placée sous atmosphère d'argon, on ajoute en 15 minutes à 60°/65°C 1,045 g de 2-[(4-bromo phényl) thio]-tétrahydro-2H-pyranne obtenu à la préparation A ci-dessus en solution dans 2,5 $cm^3$ de tétrahydrofuranne et agite pendant 30 minutes.

b) condensation

On refroidit la suspension obtenue ci-dessus à 0°/+5°C, ajoute 29 mg de chlorure de cuivre, 1 $cm^3$ de tétrahydrofuranne et agite pendant 15 minutes à 0°C. On refroidit à -20°C et ajoute 650 mg de 3-(1,2-éthanediyl) acétal cyclique de 5alpha, 10alpha-époxy estra-9,11-èn-3,17-dione obtenu selon EP 0057115 (ex 7) en solution dans 4 $cm^3$ de tétrahydrofuranne. On agite pendant 30 minutes à -15°C puis pendant 1 h 30 à 0°C. On verse dans une solution aqueuse saturée de chlorure d'ammonium, extrait avec de l'acétate d'éthyle, sèche et évapore à sec sous pression réduite. On obtient 1,409 g de produit brut.

c) décétalisation

A une solution de 1,409 g de produit obtenu ci-dessus dans 30 $cm^3$ de méthanol placée sous atmosphère d'argon on ajoute 6 $cm^3$ d'acide chlorhydrique 2N. On agite pendant 1 heure à température ambiante et verse dans une solution aqueuse saturée de bicarbonate de sodium, extrait à l'acétate d'éthyle, lave avec une solution saturée de chlorure de sodium, sèche et évapore à sec sous pression réduite. On chromatographie le résidu (1,27 g) sur silice (éluant : acétate d'éthyle-cyclohexane 1-1) et obtient 647 mg de produit attendu que l'on cristallise dans un mélange chlorure de méthylène-éther isopropylique. On obtient 596 mg de cristaux.

| Analyse : $C_{29}H_{34}O_3S$ = 462,65 | | | |
|---|---|---|---|
| | C% | H% | S% |
| Calculé | 75,28 | 7,40 | 6,93 |
| Trouvé | 75,0 | 7,5 | 6,8 |

Spectre IR : ($CHCl_3$)

| | |
|---|---|
| C=O | 1736 $cm^{-1}$ (17-céto) |
| | 1656 $cm^{-1}$ (diénone) |
| | 1603 $cm^{-1}$ |
| aromatique | 1493 $cm^{-1}$ |
| présence de | 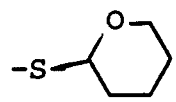 |

**Exemple 15 : N-butyl-8-(4-(3,17béta-dihydroxy-estra-1,3,5(10)-trien-11béta-yl) phénylthio)-N-méthyl-octanamide.**

Stade A : N-butyl-[8-[4-(3,17-dioxo-estra-4,9-dièn-11béta-yl) phénylthio]-N-méthyloctamide.

a) formation du thiolate d'argent

A une solution de 368 mg de produit obtenu à la préparation B ci-dessus dans 3,7 $cm^3$ de chloroforme à 40°C on ajoute 4,5 $cm^3$ de méthanol, puis 1 $cm^3$ d'une solution aqueuse 1M de nitrate d'argent. On agite pendant 10 minutes à 40°C, refroidit à 0/+5°C et conserve le précipité par décantation du surnageant.

b) S-alkylation

Au précipité obtenu ci-dessus en solution dans 3,7 $cm^3$ de chloroforme on ajoute une solution de 353 mg de 8-bromo N-butyl N-méthyl octanamide obtenu à la préparation de l'exemple 2, dans 7,4 $cm^3$ d'acétone, puis 103 mg de bicarbonate de potassium et 2,95 $cm^3$ d'hexaméthyl phosphorotriamide. On chauffe à 60°C pendant 70 heures, refroidit à température ambiante et verse dans une solution d'acide chlorhydrique 1M. On extrait à l'acétate d'éthyle, filtre, lave la phase organique avec une solution saturée de chlorure de sodium, sèche puis évapore à sec sous pression réduite. Au résidu huileux obtenu (1,362 g) mis en solution avec 3,7 $cm^3$ d'acétone on ajoute 0,74 $cm^3$ de solution de soude 2N. On chauffe à 60°C pendant 1 heure puis verse dans de l'eau, acidifie avec de l'acide chlorhydrique 2N, extrait avec de l'acétate d'éthyle, sèche et évapore à sec sous pression réduite. On chromatographie le résidu (914 mg) sur silice (éluant : acétate éthylechlorure de méthylène 1-1) et obtient, sous forme d'huile, 332 mg de produit attendu.

Spectre IR : ($CHCl_3$)

| | |
|---|---|
| C=O | 1736 $cm^{-1}$ (17-céto) |
| | 1655 $cm^{-1}$ (diénone) |
| | 1628 $cm^{-1}$ (amide III) |
| aromatique | 1580, 1492 $cm^{-1}$ |

Stade B : N-butyl-8-(4-(3,17béta-dihydroxy-estra-1,3,5(10)-trièn-11béta-yl) phényl) thio)-N-méthyl-octanamide.

On opère comme au stade B de l'exemple 7 en partant de 333 mg de produit obtenu au stade A ci-dessus, 0,34 $cm^3$ d'anhydride acétique et 0,17 $cm^3$ de bromure d'acétyle, puis on extrait avec de l'acétate d'éthyle. On traite le résidu (335 mg) avec 27,5 mg d'hydrure de bore et de sodium, puis 0,7 ml de soude 2N. Après extraction a l'acétate d'éthyle, on chromatographie le résidu (303 mg) sur Lichroprep Si 60 (éluant : acétone-chlorure de méthylène 1-9) puis sur Lichrosorb RP 18 (éluant : méthanol-eau 9-1) et obtient 134 mg de produit attendu.
$[alpha]_D$ = -49°5 (C = 1,1 % $CHCl_3$)

| analyse : $C_{37}H_{53}NO_3S$ = 591,90 | | | | |
|---|---|---|---|---|
| | C% | H% | S% | N% |
| Calculé | 75,08 | 9,02 | 5,41 | 2,36 |
| Trouvé | 75,4 | 9,3 | 5,5 | 2,4 |

Spectre IR : ($CHCl_3$)

OH 3605 $cm^{-1}$ (+ assosié)
C=O 1621 $cm^{-1}$ (amide III)
aromatique 1582, 1558, 1498, 1492 $cm^{-1}$

**Préparation de l'exemple 16 : 3,17abéta-bis[(tétrahydro-2H-2-pyrannyl) oxy]-11béta-(4-éthynylphényl)-D-homo-estra-1,3,5(10)-triène.**

Stade A : 1,2-éthanediyl acétalcyclique de 5alpha, 10alpha-époxy 17alpha-[(tétrahydro-2H-2-pyrannyl) oxy]-D-homo estr-9(11)èn-3one.

A une solution de 6 g de 1,2-éthanediyl acétal cyclique de 17abéta-[(tétrahydro-2H-2-pyrannyl) oxy]-D-homo estra-5(10), 9(11)-dièn-3one que l'on peut obtenir selon le brevet FR 2594830 (exemple 6 - stade A) dans 60 $cm^3$ de chlorure de méthylène, on ajoute 0,6 $cm^3$ de pyridine puis à 0°C 1,7 $cm^3$ d'hexafluoroacétone et en 5 minutes 3,4 $cm^3$ d'eau oxygénée à 50 %. On agite pendant 6 h 30 à 0°C, verse dans une solution saturée de thiosulfate de sodium, lave jusqu'à fin de péroxyde. On extrait avec du chlorure de méthylène, lave avec une solution saturée de chlorure de sodium, sèche, évapore à sec sous pression réduite. On chromatographie le résidu (12 g) correspondant à 2 préparations identiques sur silice (éluant : cyclohexane-acétate d'éthyle 9-1 à 0,5 % de triéthylamine) et obtient 7,1 g d'alpha-époxyde attendu.

Stade B : 11béta-(4-éthynylphényl)-17abéta-hydroxy-D-homo estra-4,9-dien-3one.

a) condensation

On opère comme au stade A de la préparation A de l'exemple 1 en partant de 5,7 g d'époxyde ci-dessus en solution dans 46 $cm^3$ de tétrahydrofuranne, 232 mg de chlorure de cuivre et 60 $cm^3$ de magnésien préparé selon le stade A de la préparation A de l'exemple 9 à partir de 4-triméthyl éthynyl-bromobenzène décrit à la préparation 4, que l'on introduit lentement, sous atmosphère d'azote à -5°C/0°C. On chromatographie le résidu sur silice (éluant : cyclohexane-acétate d'éthyle 7-3 à 0,5 % de triéthylamine) et obtient 6,3 g de produit que l'on désilyle en solution dans 100 $cm^3$ de méthanol et 20 $cm^3$ de chlorure de méthylène par addition de 2,6 $cm^3$ de soude 10N. On libère ensuite les fonctions protégées en 17 et 3 par addition à 0°C/+5°C de 7,2 $cm^3$ d'acide chlorhydrique concentré, puis après dilution avec de la glace, par ajustage du pH à 8-9 avec de l'ammoniaque concentrée. On extrait avec du chlorure de méthylène, lave avec une solution saturée de chlorure de sodium, sèche et évapore à sec sous pression réduite. On chromatographie le résidu (4,3 g) sur silice (éluant : chlorure de méthylène-acétone 96-4) et obtient 3,36 g de produit attendu.

Spectre IR : ($CHCl_3$)

OH 3612 $cm^{-1}$
≡CH 3302 $cm^{-1}$
C=O 1656, 1604 $cm^{-1}$ (diénone)
aromatique 1555, 1504 $cm^{-1}$

Stade C : 3,17abéta-diacétate de 11béta-[4-éthynyl phényl]-D-homo-estra-1,3,5(10) trien-3,17abéta-diol.

A une solution de 200 mg de produit obtenu au stade B ci-dessus dans 2 $cm^3$ de chlorure de méthylène, on ajoute à 0/+ 5°C 0,09 $cm^3$ de bromure d'acétyle puis 0,18 $cm^3$ d'anhydride acétique. On agite pendant 3 heures à température ambiante, verse dans 20 g de mélange (1-1) de glace et de solution saturée de bicarbonate de potassium, extrait avec du chlorure de méthylène, lave avec une solution saturée de chlorure de sodium, sèche et évapore à sec sous pression

réduite. On chromatographie le résidu (230 mg) sur silice (éluant : cyclohexane-acétate d'éthyle 9-1) et obtient 147 mg de produit attendu.

Spectre IR : ($CHCl_3$)

| | |
|---|---|
| ≡CH | 3303 $cm^{-1}$ |
| acétate phénolique | 1749 $cm^{-1}$ |
| acétate en 17 | 1727 $cm^{-1}$ |
| aromatique | 1608, 1588, 1556, 1505, 1494 $cm^{-1}$ |

Stade D : 3,17abéta-bis[(tétrahydro-2H-2-pyrannyl) oxy]11béta-(4-éthynylphényl)-D-homo-estra-1,3,5(10)-triène.

On agite 2,395 g de produit obtenu selon le procédé décrit au stade C ci-dessus dans une solution de potasse 1M dans le méthanol sous atmosphère d'argon. On chauffe à 35°C pendant 30 minutes, verse dans de l'eau glacée, ajuste le pH à 7 avec de l'acide chlorhydrique 2N, extrait avec du chlorure de méthylène, lave avec une solution saturée de chlorure de sodium, sèche, évapore à sec sous pression réduite. On chromatographie le résidu (2,14 g) sur silice (éluant : cyclohexane-acétate d'éthyle 6-4) et obtient 1,89 g de produit désacétylé intermédiaire. En partant de 1,8 g de produit ci-dessus en solution dans 60 $cm^3$ d'éther éthylique on ajoute 3,2 $cm^3$ de dihydropyranne et 50 mg d'acide paratoluène sulfonique. On agite pendant 3 heures à température ambiante. On ajoute 30 $cm^3$ de solution saturée de bicarbonate de sodium, extrait avec de l'acétate d'éthyle, lave, sèche et évapore à sec sous pression réduite. On chromatographie le résidu (2,7 g) sur silice (éluant : cyclohexane-acétate d'éthyle 9-1 à 0,5 % de triéthylamine) et obtient 2,429 g de produit attendu.

Spectre IR : ($CHCl_3$)

| | |
|---|---|
| ≡CH | 3302 $cm^{-1}$ |
| aromatique | 1607, 1575, 1555, 1499 $cm^{-1}$ |

**Exemple 16 : N-butyl-8-(4-(3,17abéta-dihydroxy-D-homoestra-1,3,5(10)-trien-11béta-yl) phényl)-N-méthyl-7-octynamide.**

On opère comme au stade A de l'exemple 10 en partant de 0,8 g de produit obtenu au stade D de la préparation ci-dessus, 6,5 $cm^3$ d'hexaméthylphosphorotriamide et 1,7 $cm^3$ d'une solution de butyl lithium 1,1 M, puis ajoute 530 mg de 6-bromo N-butyl N-méthyl hexanamide préparé comme à la préparation de l'exemple 2 en partant d'acide 6-bromohexanoïque. On verse dans une solution saturée de chlorure de sodium, extrait avec de l'acétate d'éthyle. Après chromatographie du résidu (3 g), on obtient 844 mg de produit dont on élimine le groupement pyrannyle en 3 et 17 en solution dans 40 ml de méthanol auquel on ajoute 8 ml d'acide chlorhydrique 2N. On verse sur un mélange eau-glace (1-1) extrait au chlorure de méthylène, lave, sèche et évapore à sec sous pression réduite. On chromatographie le résidu (610 mg) sur silice (éluant : chlorure de méthylène-acétone 8-2) et obtient 536 mg de produit attendu.

| Analyse : $C_{38}H_{51}NO_3$ = 569,84 | | | |
|---|---|---|---|
| | C% | H% | N% |
| Calculé | 80,10 | 9,02 | 2,46 |
| Trouvé | 80,1 | 9,2 | 2,4 |

Spectre IR : ($CHCl_3$)

| | |
|---|---|
| OH | 3603 $cm^{-1}$ |
| C=O | 1625 $cm^{-1}$ (amide III) |
| aromatique | 1580, 1556, 1507 $cm^{-1}$ |

**Exemple 17 : N-butyl-4-(3,17béta-dihydroxy-D-homoestra-1,3,5(10)-trien-11béta-yl)-N-méthyl-benzeneoctanamide.**

On opère comme pour la réduction au Stade B de l'exemple 10 en partant de 360 mg de produit obtenu à l'exemple

16 et 180 mg de palladium sur charbon. On chromatographie le résidu (375 mg ) sur silice (éluant : chlorure de méthylène-acétone 9-1) et obtient 336 mg de produit attendu. $[alpha]_D$ = -26°5 ± 1° (C = 1 % éthanol)

| Analyse : $C_{38}H_{55}NO_3$ = 573,87 | | | |
|---|---|---|---|
| | C% | H% | N% |
| Calculé | 79,54 | 9,66 | 2,44 |
| Trouvé | 79,2 | 9,8 | 2,4 |

Spectre IR : ($CHCl_3$)

| | |
|---|---|
| OH | 3606 $cm^{-1}$ |
| C=O | 1622 $cm^{-1}$ (amide III) |
| aromatique | 1585, 1500 $cm^{-1}$ |

**Exemple 18 : N-butyl-2-(5-(4-(3,17béta-dihydroxy-estra-1,3,5(10)-trien-11béta-yl) phénoxy) pentyl) thio)-N-méthyléthanethioamide.**

On opère comme aux stades D, E et F de l'exemple 1 en partant de 570 g de N-butyl-[5-[4-(3,17-béta-dihydroxy estra-1,3,5(10)-trien-11béta-yl) phénoxy] pentylthio] N-méthylacétamide obtenu au stade C de l'exemple 8 :

- pour le stade D, on utilise 0,57 $cm^3$ d'anhydride acétique et 28 mg de 4-diméthyl-aminopyridine et obtient 682 mg de résidu brut du produit 3,17-diacétoxy attendu.

Spectre IR : ($CHCl_3$)

| | |
|---|---|
| absence | d'OH |
| C=O | 1720, 1760 $cm^{-1}$ (ester) |
| | 1640 $cm^{-1}$ (amide) |
| aromatique | 1605, 1580, 1500 $cm^{-1}$ |

- pour le stade E, on utilise 680 mg du produit brut ci-dessus, 250 mg de réactif de Lawesson, agite pendant 4 heures à 50°C et obtient 560 mg de résidu brut du produit thioamide attendu.

Spectre IR : ($CHCl_3$)

| | |
|---|---|
| acétate | 1720 à 1760 $cm^{-1}$ |
| C=O (amide) | absence |
| C=S | 1605 $cm^{-1}$ |
| aromatique | 1500, 1580 $cm^{-1}$ |

- pour le stade F on utilise 560 mg du produit brut précédent, 4 $cm^3$ de soude 2N, obtient 516 mg de résidu brut et après chromatographie (éluant : essence G-acétate d'éthyle 6-4) isole 395 mg de produit attendu.

$[alpha]_D$ = -30° ± 1° (C = 0,45 % $CHCl_3$)

| Analyse : $C_{36}H_{51}NO_3S_2$ = 609,94 | | | | |
|---|---|---|---|---|
| | C% | H% | N% | S% |
| Calculé | 70,89 | 8,43 | 2,30 | 10,51 |
| Trouvé | 70,9 | 8,4 | 2,3 | 10,4 |

Spectre IR : ($CHCl_3$)

OH 3602 $cm^{-1}$
aromatique 1610, 1578, 1512 $cm^{-1}$

**EXEMPLE 19 : Monobutanedioate de 11béta-(4-(5-((2-(butylméthylamino) 2-oxoéthyl) thio) pentyloxy) phényl) 3-hydroxy estra 1,3,5(10)-trièn-17béta-yle.**

On chauffe au reflux pendant 3 heures et demie 760 mg de N-butyl (5-(4-(3,17béta-dihydroxy estra-1,3,5(10)-trièn-11béta-yl) phénoxy) pentylthio) N-méthyl acétamide préparé comme à l'exemple 77 de la demande de brevet européen n° 90400493.4 et 760 mg d'anhydride succinique avec 80 mg de diméthylaminopyridine et 8 $cm^3$ de pyridine. On refroidit à température ambiante la solution comprenant un mélange de monoester et de diester, ajoute 8 $cm^3$ de méthanol, 8 $cm^3$ d'eau et 1,3 g de carbonate de potassium. On agite 6 heures à température ambiante, refroidit à 4°C, acidifie le milieu réactionnel à l'aide d'acide chlorhydrique 2N, extrait à l'acétate d'éthyle, lave à l'acide chlorhydrique 2N, puis à l'eau salée, sèche et élimine les solvants sous pression réduite. On obtient 1,2 g du monoester brut attendu que l'on chromatographie sur silice (éluant : éther de pétrole 40-70°-acétone-acétate d'éthyle 60-40-1) et obtient 650 mg d'ester pur. Rf = 0,22.

Spectre IR ($CHCl_3$)

OH : 3600 $cm^{-1}$
C=O : 1717 $cm^{-1}$ (complexe) et 1626 $cm^{-1}$
aromatique : 1611, 1581 et 1511 $cm^{-1}$

**EXEMPLE 20 : Butanedioate de 11béta-(4-(5-((2-(butylméthylamino) 2-oxoéthyl) thio) pentyloxy) phényl) 3-hydroxy estra 1,3,5(10)-trièn-17béta-yle et de sodium.**

On dissout 72 mg de bicarbonate de sodium dans 7 $cm^3$ d'eau puis ajoute 630 mg de produit obtenu ci-dessus dans 8 $cm^3$ d'éthanol. On agite la solution obtenue pendant 20 minutes, élimine l'éthanol sous pression réduite, dilue à l'eau, filtre et lyophilise. On obtient 600 mg de produit attendu.

| Analyse : $C_{40}H_{54}NNaO_7S$ | | | | |
|---|---|---|---|---|
| Calculé | C% 67,13 | H% 7,60 | N% 1,96 | S% 4,48 |
| Trouvé | 67,0 | 7,7 | 1,9 | 4,8 |

Compositions pharmaceutiques:

On a préparé des comprimés répondant a la formule suivante :

| | |
|---|---|
| - Produit de l'exemple 2 | 50 mg |
| - Excipient (talc, amidon, stéarate de magnésium) q.s. pour un comprimé termine à | 120 mg |

Etude pharmacologique des produits de l'invention 1 - Etude de l'activité des produits de l'invention sur le récepteur estrogène de l'utérus de souris :

Des souris femelles impubères âgées de 18 a 21 jours sont sacrifiées, les utérus sont prélevés puis homogénéisés a 0°C à l'aide d'un Potter teflon-verre dans une solution tamponnée TS (Tris 10 mM, saccharose 0,25 M, HCl pH 7,4 (1 g de tissu pour 25 ml de TS). L'homogénat est ensuite ultracentrifugé (105 000 g x 90 mn) à 0°C. Des aliquotes du surnageant ainsi obtenu, sont incubées a 0°C ou a 25°C pendant un temps (t) avec une concentration constante (T) d'estradiol tritié en présence de concentrations croissantes (0 - $1000.10^{-9}$ M) soit d'estradiol froid, soit du produit froid a tester. La concentration d'estradiol tritié lié (B) est ensuite mesurée dans chaque incubat par la technique d'adsorption au charbon-dextran.

Calcul de l'affinité relative de liaison :

Le calcul de l'affinité relative de liaison (ARL) est identique pour tous les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en fonction du logarithme de la concentration du produit froid testé.

On détermine la droite d'équation

$$I_{50} = (\frac{B}{T} \max + \frac{B}{T} \min)/2.$$

$\frac{B}{T}$ max = Pourcentage de l'hormone tritiée liée pour une incubation de cette hormone tritiée à la concentration (T). $\frac{B}{T}$ min = Pourcentage de l'hormone tritiée liée pour une incubation de cette hormone tritiée à la concentration (T) en présence d'un grand excès d'hormone froide ($2500.10^{-9}$M).

Les intersections de la droit $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation

$$ARL = 100 \frac{(CH)}{(CX)}.$$

Les résultats obtenus sont les suivants :

| Produits des Exemples | Récepteurs Oestrogène Temps d'incubation à 0°C | |
|---|---|---|
| | 2H | 5H |
| 1 | | 20 |
| 2 | | 16 |
| 5 | 0 | 23 |
| 6 | 3,3 | 29 |
| 9 | | 11,7 |
| 14 | 2,8 | 47 |
| 18 | | 16 |

Conclusion :

Les produits étudiés, en particulier le produit de l' exemple 14 ont une affinité marquée pour les récepteurs oestrogènes au deuxième temps.

En outre la majorité des produits sont dépourvus d'activité utérotrophique.

2 - Activité anti-proliférative des produits de l'invention sur la croissance de cellules tumorales mammaires MCF-7. Description du test :

a) Culture cellulaire :

Les lignées MCF-7 sont maintenues en culture en milieu SVF(1) à 37°C en atmosphère humide contenant 5 % $CO_2$. Les cellules à subconfluence sont récoltées par trypsination (trypsine 0,05 %, EDTA 0,02 %) puis rincées par centrifugation douce. Un échantillon des cellules en suspension est compté sur cellule de Malassez.

b) Etude de la croissance :

Les cellules resuspendues dans le milieu SVF sont ensemencées à raison de 30.000 cellules par puits dans des

plaques multipuits (24 puits de 2,5 $cm^2$). Vingt quatre heures après l'ensemencement (JO), le produit à tester est ajouté au milieu en solution éthanolique (concentration finale en éthanol : 0,1 %), à la concentration de $10^{-12}$ à $10^{-6}$M, les puits contrôles recevant la même concentration en éthanol. Les milieux sont renouvelés toutes les 48 heures. En fin d'expérience (J6), le milieu est aspiré et les cellules sont immédiatement fixées par 150 microlitres de méthanol afin de doser l'ADN.

L'activité anti-proliférative des produits est évaluée par leur capacité à inhiber l'augmentation d'ADN.

c) Dosage de l'ADN :

L'ADN est dosé par une méthode fluorimétrique utilisant le DABA (Acide 3,5 diaminobenzoïque) (2) : 150 microlitres de DABA sont ajoutés dans chaque puits ; les plaques sont alors incubées 45 mn à 56°C, puis 2 ml d'HCl 1N sont ajoutés. La fluorescence est mesurée à l'aide d'un fluorimètre (longueur d'onde excitatrice : 408 nm, longueur d'onde d'émission : 510 nm).

La quantité d'ADN par puits est évaluée par rapport à une gamme étalon obtenue en traitant dans les mêmes conditions un standard d'ADN de thymus de veau.

Résultats

La concentration en nm qui inhibe de 50 % la croissance des cellules $MCF_7$ ($CI_{50}$) a été déterminée de la manière indiquée ci-dessus :

Résultats :

Produit de l'exemple 1 : $CI_{50}$ = 0,03 nM
Produit de l'exemple 2 : $CI_{50}$ = 0,035 nM
Produit de l'exemple 5 : $CI_{50}$ = 0,02 nM
Produit de l'exemple 6 : $CI_{50}$ = 0,007 nM
Produit de l'exemple 9 : $CI_{50}$ = 0,009 nM
Produit de l'exemple 14 : $CI_{50}$ = 0,015 nM

En outre, l'effet inhibiteur maximal des produits atteint environ 90 %.

(1) Le milieu de culture sérum de veau foetal (SVF) est préparé comme suit :
Milieu MEM (minimal Essential Medium) auquel sont ajoutés :

- acides aminés non essentiels (GIBCO),
- peni-strepto (penicilline 100 U/ml, streptomycine 0,1 mg/ml),
- fungizone 0,1 %,
- insuline (50 ng/ml),
- sérum de veau foetal destéroïdé (10 % concentration finale). (2) Puzas et Goodman, Analytical Biochemistry, Vol. 86, pp. 50, 1978.

**Revendications**

**Revendications pour les Etats contractants suivants : AT, BE, CH, DE, DK, FR, GB, IT, LI, LU, NL, SE**

1. Les composés de formule (I) :

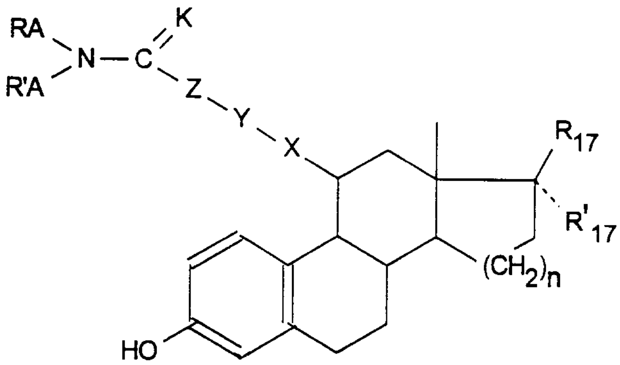

(I)

dans laquelle :

soit

n = 1, K représente un atome d'oxygène,

$R_{17}$ représente un radical hydroxyle ou un radical de formule

$$-O-\underset{\underset{O}{\|}}{C}-(CH_2)_2-COOH$$

éventuellement salifié,

$R'_{17}$ représente un atome d'hydrogène ou un radical éthynyle,

$R_A$ représente un radical méthyle,

$R'_A$ représente un radical isopropyle ou butyle linéaire

X représente un radical méthylène, phénylène ou phényloxy lié au stéroïde par un atome de carbone,

Y représente un des radicaux choisis dans le groupe constitué par $-(CH_2)_7$ ; $-(CH_2)_8-$ ; $-(CH_2)_5-C{\equiv}C-$ ; $-(CH_2)q-O-CH_2-$ avec q = 5 à 7 et $-(CH_2)_5-S-CH_2-$ dans lequel l'atome de soufre est éventuellement oxydé sous forme de sulfoxyde ou de sulfone,

dont les noms suivent :

- le monobutanedioate de 11béta-(4-((7-(butylméthylamino) carbonyl) heptyl) oxy) phényl)-3-hydroxy-estra-1,3,5(10)-trien-17béta-yle,
- le butanedioate de 11béta-(4-((7-((butylméthylamino) carbonyl) heptyl) oxy) phényl)-hydroxy-estra-1,3,5(10)-trien-17béta-yle et de sodium,
- le N-butyl-2-(6-(4-(3,17béta-dihydroxy-estra-1,3,5(10)-trien-11béta-yl)phénoxy)hexyloxy-N-méthyl-acétamide,
- le N-butyl-8-(4-(3,17béta-dihydroxy-estra-1,3,5(10)-trien-11béta-yl)phénoxy)-N-méthyl-2-octynamide,
- le N-butyl-2-((5-(4-(3,17béta-dihydroxy-estra-1,3,5(10)-trien-11béta-yl) phényl) pentyl) thio) N-méthyl acétamide,
- le N-butyl-4-(3,17béta-dihydroxy-estra-1,3,5(10)-trien-11béta-yl)-N-méthyl-benzenenonamide,
- le N-butyl-2-((5-(4(3,17béta-dihydroxy-estra-1,3,5(10)-trien-11béta-yl) phenyl) pentyl) oxy)-N-méthyl-acétamide,
- le 2-((8-(3,17béta-dihydroxy 19-nor-17alpha-pregna-1,3,5(10)-trien-20-yn-11béta-yl) octyl) oxy)-N-méthyl-N-(1-méthyléthyl)-acétamide.

Z représente une simple liaison,

soit

n = 1 ou 2,

K représente un atome d'oxygène ou un atome de soufre, $R_{17}$ et $R'_{17}$ sont tels que :

- soit $R_{17}$ et $R'_{17}$ forment ensemble une fonction cétone,

- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O, arylènoxy ou arylènethio lié au stéroïde par un atome de carbone,
- Y représente une simple liaison ou une chaine aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison étant entendu que lorsque Y et Z sont une simple liaison, X ne peut-être un radical méthylène ou $CH_2$-O,

$R_A$ ou $R_{A'}$ identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié ou $R_A$ et $R_{A'}$ forment avec l'atome d'azote auquel ils sont liés un hétérocycle à 5 ou 6 chainons, saturé ou non, renfermant éventuellement un ou plusieurs hétéroatomes choisis parmi le groupe constitué par les atomes d'oxygène, d'azote et de soufre et éventuellement substitué par un radical alkyle ayant de 1 à 4 atomes de carbone, un radical aryle ou aralkyle ayant de 6 à 10 atomes de carbone, étant entendu que l'un au moins des radicaux $R_A$ ou $R_{A'}$ n'est pas un atome d'hydrogène et qu'au moins l'une quelconque des conditions suivantes est remplie :

- soit n = 2,
- soit K est un atome de soufre,
- soit X est un radical arylénethio lié au stéroïde par un atome de carbone,
- soit $R_A$ et $R_{A'}$ forment avec l'atome de carbone auquel ils sont liés un hétérocycle à 5 ou 6 chainons substitué par un radical aryle ou aralkyle.

2. Les produits répondant à la formule ($I_A$) correspondant à la formule (I) telle que définie à la revendication 1 dans laquelle :

n = 1 ou 2,
K représente un atome d'oxygène ou un atome de soufre, $R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O, arylènoxy ou arylènethio lié au stéroïde par un atome de carbone,
- Y représente une simple liaison ou une chaine aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison étant entendu que lorsque Y et Z sont une simple liaison, X ne peut-être un radical méthylène ou $CH_2$-O,

$R_A$ ou $R_{A'}$ identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié ou $R_A$ et $R_{A'}$ forment avec l'atome d'azote auquel ils sont liés un hétérocycle à 5 ou 6 chainons, saturé ou non, renfermant éventuellement un ou plusieurs hétéroatomes choisis parmi le groupe constitué par les atomes d'oxygène, d'azote et de soufre et éventuellement substitué par un radical alkyle ayant de 1 à 4 atomes de carbone, un radical aryle ou aralkyle ayant de 6 à 10 atomes de carbone, étant entendu que l'un au moins des radicaux $R_A$ ou $R_{A'}$ n'est pas un atome d'hydrogène et qu'au moins l'une quelconque des conditions suivantes est remplie :

- soit n = 2,
- soit K est un atome de soufre,
- soit X est un radical arylénethio lié au stéroïde par un atome de carbone,
- soit $R_A$ et $R_{A'}$ forment avec l'atome de carbone auquel ils sont liés un hétérocycle à 5 ou 6 chainons substitué par un radical aryle ou aralkyle.

3. Les produits répondant à la formule ($I_A$) telle que définie à la revendication 2 pour lesquels n est égal à 1.

4. Les produits selon la revendication 3 pour lesquels K est un atome de soufre, X est un radical arylèneoxy et Y représente une chaine linéaire saturée renfermant de 5 à 10 atomes de carbone, éventuellement interrompue par un atome d'oxygène.

5. Les produits selon la revendication 3 pour lesquels X est un radical arylènethio et Y représente une chaine linéaire saturée renfermant de 5 à 10 atomes de carbone.

6. Les produits selon la revendication 3 pour lesquels X est un radical arylène, Y représente une chaine linéaire insaturée renfermant de 5 à 10 atomes de carbone, $R_A$ et $R_{A'}$ forment avec l'atome auquel ils sont liés une pipéridine N-substituée par un radical aralkyle.

7. Les produits de formule (I) selon la revendication 1 dont les noms suivent :

   - le N-butyl-5-(4-(3,17béta-dihydroxy-estra-1,3,5(10)-trien-11béta-yl) phénoxy) pentyloxy)-N-méthyl-éthanethioamide,
   - le N-butyl-8-(4-(3,17béta-dihydroxy-estra-1,3,5(10)-trien-11béta-yl) phénoxy)-N-méthyl-octanethioamide.

8. Procédé de préparation des produits de formule (I) telle que définie à la revendication 1, caractérisé en ce que l'on soumet un composé de formule (II) :

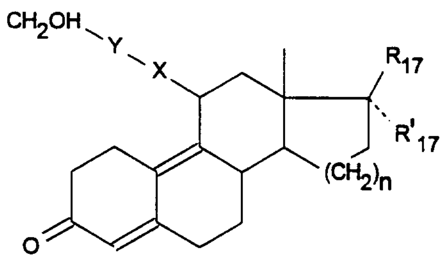

(II)

dans laquelle X, Y, n, $R_{17}$ et $R'_{17}$ ont la même signification qu'à la revendication 1, étant entendu que lorsque $R_{17}$ représente un radical hydroxyle, ce radical est protégé, à l'action d'un agent d'oxydation pour obtenir le produit de formule (III) :

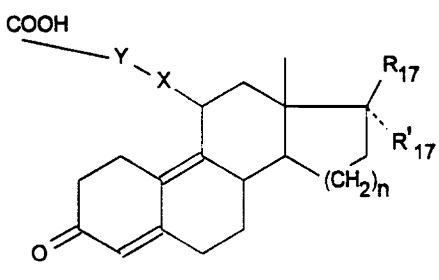

(III)

que l'on soumet à l'action d'un agent permettant d'activer la fonction carboxylique, puis à l'action d'un composé de formule (IV) :

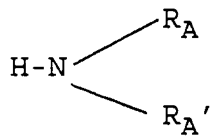

(IV)

dans laquelle $R_A$ et $R_A$' ont la même signification qu'à la revendication 1, pour obtenir le produit de formule (V) :

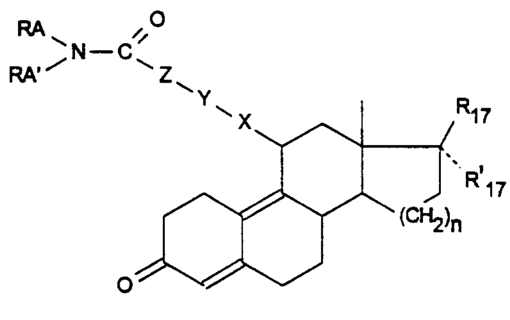

(V)

dans laquelle Z est une simple liaison, produits (V) que, si nécessaire, l'on soumet à une réaction de déprotection de la fonction hydroxy et, si désiré,

- à un agent de réduction lorsque $R_{17}$ et $R'_{17}$ forment ensemble une fonction cétone, puis le cas échéant soumet à un agent d'acylation le dérivé hydroxylé en 17 ainsi obtenu,
- ou à un agent de saponification lorsque $R_{17}$ représente une fonction acyloxy,

composé de formule (V) que l'on soumet à un agent d'aromatisation du cycle A, puis à un agent de saponification ménagée pour obtenir les produits de formule (I) que, si désiré, l'on soumet à une ou à plusieurs des réactions suivantes :

soit, lorsque $R_{17}$ et $R'_{17}$ forment ensemble une fonction cétone, l'on soumet à un agent de réduction ou à un complexe métallique de formule (VI):

$$M\text{-}R'_{17} \qquad \text{(VI)}$$

dans laquelle M représente un atome métallique et $R'_{17}$ a la même signification qu'à la revendication 1, à l'exception d'hydrogène,
soit, lorsque $R_{17}$ est un radical hydroxyle, l'on soumet à un agent d'acylation sélective en position 17,
soit, lorsque $R_A$ ou $R_A$' est un atome d'hydrogène, on soumet à un agent d'alkylation approprié,
soit, lorsque Y représente une chaine aliphatique insaturée, l'on soumet à un agent d'hydrogénation,
pour obtenir les produits de formule (I) dans laquelle K est un atome d'oxygène que, si désiré, l'on soumet à un agent de sulfuration pour obtenir les produits de formule (I) dans laquelle K est un atome de soufre.

**9.** Procédé de préparation des produits de formule (I') correspondant aux produits de formule (I) telle que définie à la revendication 1 dans laquelle X représente un radical arylène et Y représente une chaine aliphatique éventuellement liée au groupe arylène par une double ou une triple liaison et comportant au moins 3 atomes de carbone ou liée au groupe arylène par un atome d'oxygène ou un atome de soufre, caractérisé en ce que l'on soumet un composé de formule (VII) :

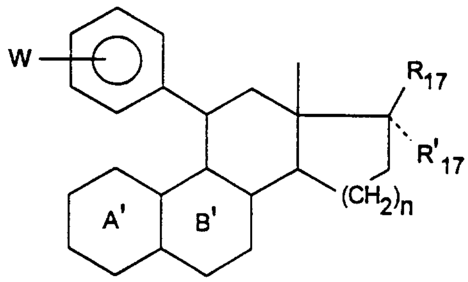

(VII)

dans laquelle W représente soit un radical -OH, soit un radical mercapto éventuellement activé, soit un radical -C≡CH, n, $R_{17}$ et R' ayant la même signification que celle indiquée à la revendication 1, étant entendu que lorsque $R_{17}$ représente un radical -OH, il est éventuellement protégé, les cycles A' et B' sont :

- soit

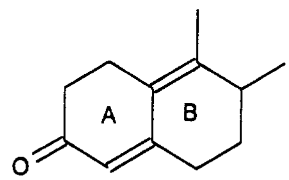

- soit

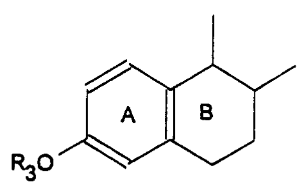

où $R_3$ représente un atome d'hydrogène ou un groupement protecteur de la fonction hydroxyle,

**<u>ou bien</u>**, dans le cas où W représente un radical C≡CH à l'action d'un dérivé halogéné de formule (VIII) :

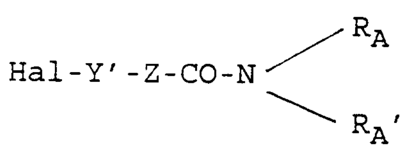

(VIII)

dans laquelle Hal est un atome d'halogène, Z, $R_A$ et $R_A$' ont la même signification que celle indiquée à la revendication 1 et Y' représente la chaine aliphatique Y ci-dessus comportant 2 atomes de carbone en moins, en présence d'une base forte et soumet le cas échéant à l'action d'un agent de déprotection en 3 et/ou en 17, pour obtenir le produit de formule ($IX_A$) :

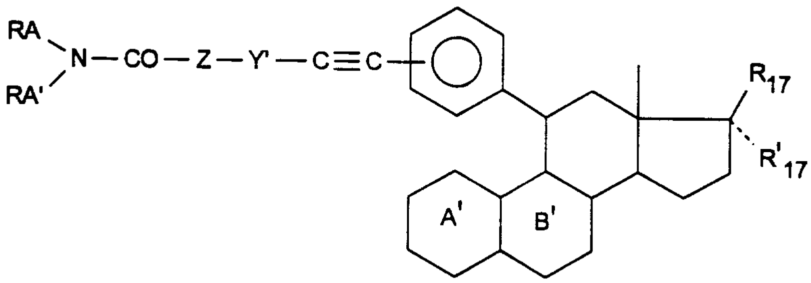

$(IX_A)$

produit que, ai désiré, l'on soumet à un agent de réduction partielle ou totale de la triple liaison pour obtenir le produit de formule $(IX_B)$ :

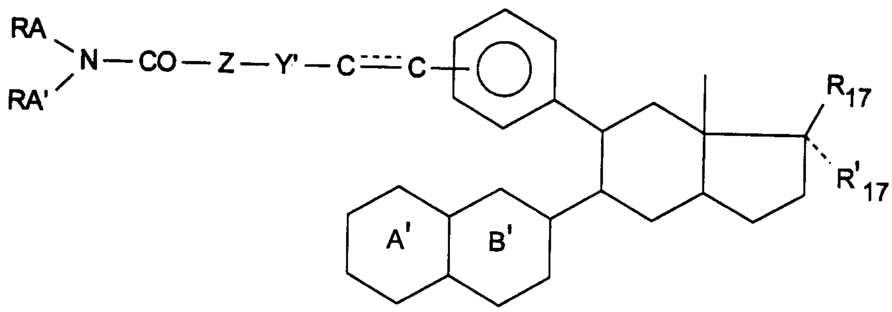

$(IX_B)$

**<u>ou bien</u>**, dans le cas où W représente un radical -OH, un radical mercapto ou un radical mercapto activé, à l'action d'un dérivé halogéné de formule (X) :

$$Hal-Y-Z-CO-N\begin{matrix} R_A \\ \\ R_A' \end{matrix} \qquad (X)$$

dans laquelle Hal, Y, Z, $R_A$ et $R_A'$ ont la même signification que celle indiquée à la revendication 1, en présence d'un agent alcalin, puis le cas échéant, à l'action d'un agent de déprotection pour obtenir un produit de formule $(IX_C)$ :

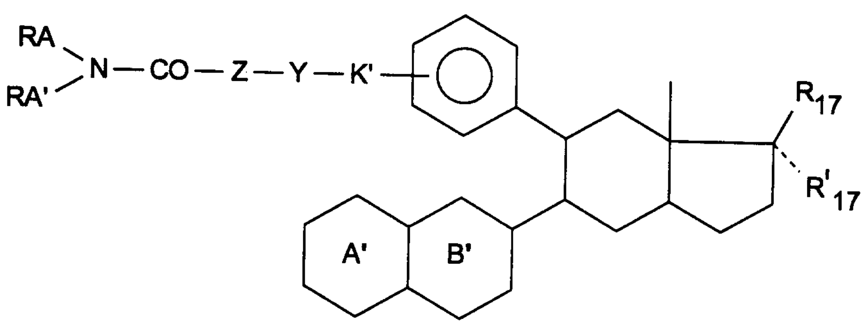

$(IX_C)$

dans laquelle K' représente soit un atome d'oxygène, soit un atome de soufre, produits de formule $(IX_A)$, $(IX_B)$ ou $(IX_C)$ que le cas échéant l'on soumet à un agent d'aromatisation du cycle A, puis à un agent de saponification ménagée pour obtenir des produits de formule (I') que le cas échéant l'on traite comme indiqué à la revendication 8 pour les produits de formule (I).

10. A titre de médicament, les produits de formule (I) et tels que définis à l'une quelconque des revendications 1 à 7.

11. Les compositions pharmaceutiques renfermant comme principe actif au moins l'un des médicaments tel que défini à la revendication 10.

12. A titre de produits intermédiaires nouveaux, les produits de formule (II) et (III) telles que définies à la revendication 8 dans lesquelles :

n = 2.

13. A titre de produits intermédiaires nouveaux les produits de formule (VII) telle que définie à la revendication 9 dans laquelle W représente un radical mercapto éventuellement activé.

**Revendications pour l'Etat contractant suivant : ES**

1. Procédé pour préparer des composés de formule (I) :

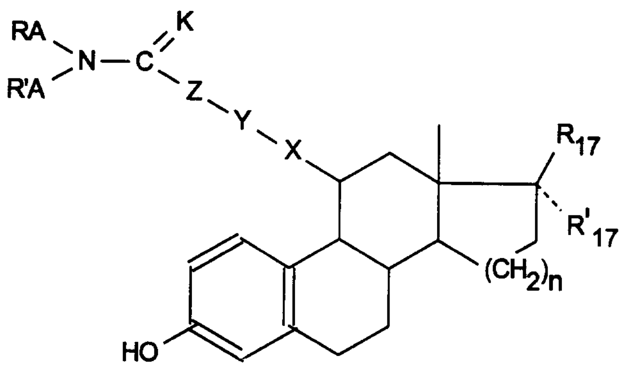

(I)

dans laquelle :
<u>soit</u>

n = 1, K représente un atome d'oxygène, $R_{17}$ représente un radical hydroxyle ou un radical de formule

$$-O-\underset{\underset{O}{\|}}{C}-(CH_2)_2-COOH$$

éventuellement salifié,
$R'_{17}$ représente un atome d'hydrogène ou un radical éthynyle,
$R_A$ représente un radical méthyle,
$R'_A$ représente un radical isopropyle ou butyle linéaire,
X représente un radical méthylène, phénylène ou phényloxy lié au stéroïde par un atome de carbone,
Y représente un des radicaux choisis dans le groupe constitué par $-(CH_2)_7$ ; $-(CH_2)_8-$ ; $-(CH_2)_5-C\equiv C-$ ; $-(CH_2)q-O-CH_2-$ avec q = 5 à 7 et $-(CH_2)_5-S-CH_2-$ dans lequel l'atome de soufre est éventuellement oxydé sous forme de sulfoxyde ou de sulfone,
dont les noms suivent :

- le monobutanedioate de 11béta-(4-((7-(butylméthylamino) carbonyl) heptyl) oxy) phényl)-3-hydroxy-estra-1,3,5(10)-trien-17béta-yle,
- le butanedioate de 11béta-(4-((7-((butylméthylamino) carbonyl) heptyl) oxy) phényl)-hydroxy-estra-1,3,5(10)-trien-17béta-yle et de sodium,
- le N-butyl-2-(6-(4-(3,17béta-dihydroxy-estra-1,3,5(10)-trien-11béta-yl)phénoxy)hexyloxy-N-méthyl-acétamide,
- le N-butyl-8-(4-(3,17béta-dihydroxy-estra-1,3,5(10)-trien-11béta-yl)phénoxy)-N-méthyl-2-octynamide,
- le N-butyl-2-((5-(4-(3,17béta-dihydroxy-estra-1,3,5(10)-trien-11béta-yl) phényl) pentyl) thio) N-méthyl acétamide,

- le N-butyl-4-(3,17béta-dihydroxy-estra-1,3,5(10)-trien-11béta-yl)-N-méthyl-benzenenonamide,
- le N-butyl-2-((5-(4(3,17béta-dihydroxy-estra-1,3,5(10)-trien-11béta-yl) phenyl) pentyl) oxy)-N-méthyl-acétamide,
- le 2-((8-(3,17béta-dihydroxy 19-nor-17alpha-pregna-1,3,5(10)-trien-20-yn-11béta-yl) octyl) oxy)-N-méthyl-N-(1-méthyléthyl)-acétamide.

Z représente une simple liaison,

soit

n = 1 ou 2,
K représente un atome d'oxygène ou un atome de soufre, $R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O, arylènoxy ou arylènethio lié au stéroïde par un atome de carbone,
- Y représente une simple liaison ou une chaine aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison étant entendu que lorsque Y et Z sont une simple liaison, X ne peut-être un radical méthylène ou $CH_2$-O,

$R_A$ ou $R_{A'}$ identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié ou $R_A$ et $R_{A'}$ forment avec l'atome d'azote auquel ils sont liés un hétérocycle à 5 ou 6 chainons, saturé ou non, renfermant éventuellement un ou plusieurs hétéroatomes choisis parmi le groupe constitué par les atomes d'oxygène, d'azote et de soufre et éventuellement substitué par un radical alkyle ayant de 1 à 4 atomes de carbone, un radical aryle ou aralkyle ayant de 6 à 10 atomes de carbone, étant entendu que l'un au moins des radicaux $R_A$ ou $R_{A'}$ n'est pas un atome d'hydrogène et qu'au moins l'une quelconque des conditions suivantes est remplie :

- soit n = 2,
- soit K est un atome de soufre,
- soit X est un radical arylénethio lié au stéroïde par un atome de carbone,
- soit $R_A$ et $R_{A'}$ forment avec l'atome de carbone auquel ils sont liés un hétérocycle à 5 ou 6 chainons substitué par un radical aryle ou aralkyle, caractérisé en ce que :

a) pour préparer des produits de formule (I) telle que définie ci-dessus, l'on soumet un composé de formule (II) :

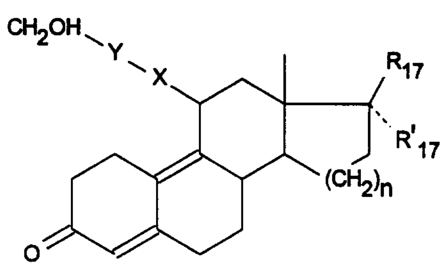

(II)

dans laquelle X, Y, n, $R_{17}$ et $R'_{17}$ ont la même signification que ci-dessus, étant entendu que lorsque $R_{17}$ repré-

sente un radical hydroxyle, ce radical est protégé, à l'action d'un agent d'oxydation pour obtenir le produit de formule (III) :

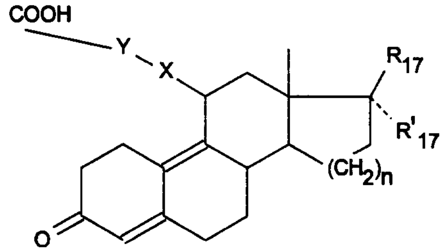

(III)

que l'on soumet à l'action d'un agent permettant d'activer la fonction carboxylique, puis à l'action d'un composé de formule (IV) :

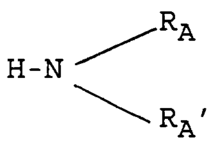

(IV)

dans laquelle $R_A$ et $R_{A'}$ ont la même signification que ci-dessus, pour obtenir le produit de formule (V) :

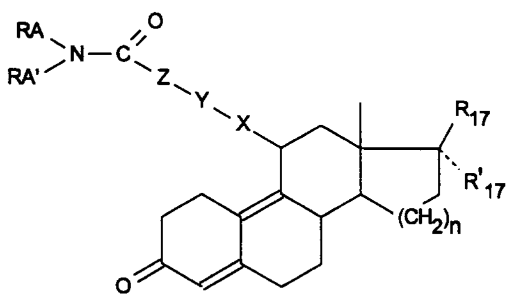

(V)

dans laquelle Z est une simple liaison, produits (V) que, si nécessaire, l'on soumet à une réaction de déprotection de la fonction hydroxy et, si désiré,

- à un agent de réduction lorsque $R_{17}$ et $R'_{17}$ forment ensemble une fonction cétone, puis le cas échéant soumet à un agent d'acylation le dérivé hydroxylé en 17 ainsi obtenu,
- ou à un agent de saponification lorsque $R_{17}$ représente une fonction acyloxy,

composé de formule (V) que l'on soumet à un agent d'aromatisation du cycle A, puis à un agent de saponification ménagée pour obtenir les produits de formule (I) que, si désiré, l'on soumet à une ou à plusieurs des réactions suivantes :

soit, lorsque $R_{17}$ et $R'_{17}$ forment ensemble une fonction cétone, l'on soumet à un agent de réduction ou à un complexe métallique de formule (VI) :

$$M\text{-}R'_{17} \qquad (VI)$$

dans laquelle M représente un atome métallique et $R'_{17}$ a la même signification que ci-dessus, à l'exception d'hydrogène,
soit, lorsque $R_{17}$ est un radical hydroxyle, l'on soumet à un agent d'acylation sélective en position 17,
soit, lorsque $R_A$ ou $R_A'$ est un atome d'hydrogène, on soumet à un agent d'alkylation approprié,
soit, lorsque Y représente une chaine aliphatique insaturée, l'on soumet à un agent d'hydrogénation,
pour obtenir les produits de formule (I) dans laquelle K est un atome d'oxygène que, si désiré, l'on soumet

à un agent de sulfuration pour obtenir les produits de formule (I) dans laquelle K est un atome de soufre,

b) pour préparer des produits de formule (I) dans laquelle X représente un radical arylène et Y représente une chaine aliphatique éventuellement liée au groupe arylène par une double ou une triple liaison et comportant au moins 3 atomes de carbone ou liée au groupe arylène par un atome d'oxygène ou un atome de soufre, l'on soumet un composé de formule (VII) :

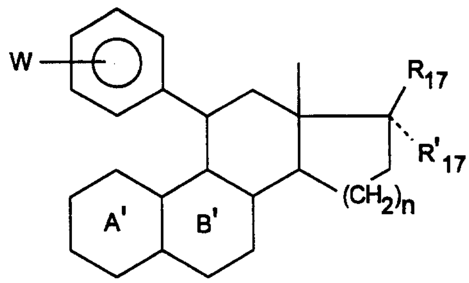

(VII)

dans laquelle W représente soit un radical -OH, soit un radical mercapto éventuellement activé, soit un radical -C≡CH, n, $R_{17}$ et R' ayant la même signification que celle indiquée ci-dessus, étant entendu que lorsque $R_{17}$ représente un radical -OH, il est éventuellement protégé, les cycles A' et B' sont :

- soit

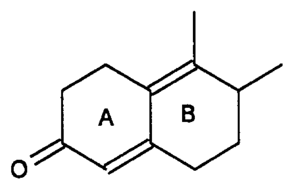

- soit

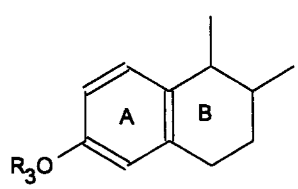

où $R_3$ représente un atome d'hydrogène ou un groupement protecteur de la fonction hydroxyle,

<u>**ou bien**</u>, dans le cas où W représente un radical C≡CH à l'action d'un dérivé halogéné de formule (VIII) :

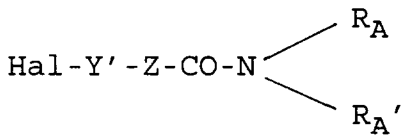

(VIII)

dans laquelle Hal est un atome d'halogène, Z, $R_A$ et $R_A$' ont la même signification que celle indiquée ci-dessus et Y' représente la chaine aliphatique Y ci-dessus comportant 2 atomes de carbone en moins, en présence

d'une base forte et soumet le cas échéant à l'action d'un agent de déprotection en 3 et/ou en 17, pour obtenir le produit de formule ($IX_A$) :

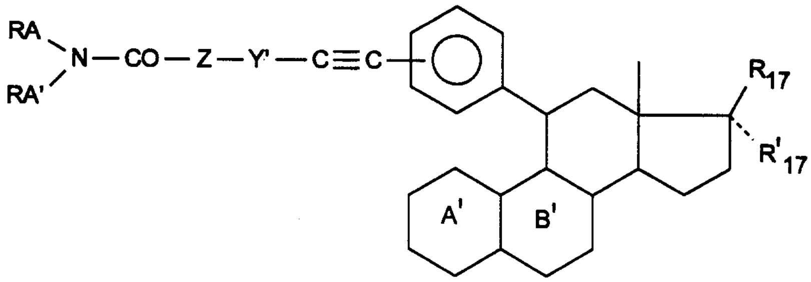

($IX_A$)

produit que, si désiré, l'on soumet à un agent de réduction partielle ou totale de la triple liaison pour obtenir le produit de formule ($IX_B$) :

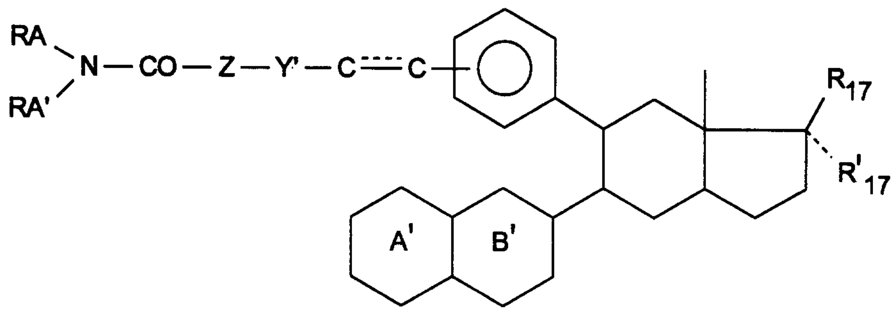

($IX_B$)

**ou bien**, dans le cas où W représente un radical -OH, un radical mercapto ou un radical mercapto activé, à l'action d'un dérivé halogéné de formule (X) :

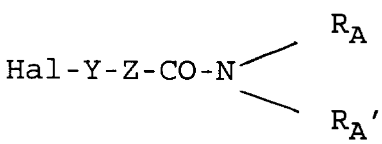

(X)

dans laquelle Hal, Y, Z, $R_A$ et $R_A$' ont la même signification que celle indiquée ci-dessus, en présence d'un agent alcalin, puis le cas échéant, à l'action d'un agent de déprotection pour obtenir un produit de formule ($IX_C$) :

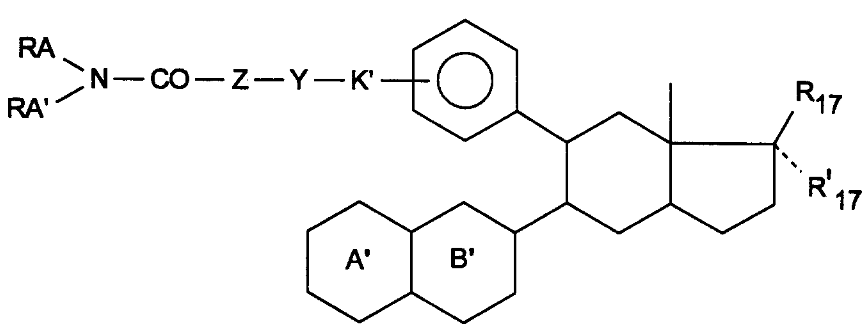

($IX_C$)

dans laquelle K' représente soit un atome d'oxygène, soit un atome de soufre, produits de formule ($IX_A$), ($IX_B$) ou ($IX_C$) que le cas échéant l'on soumet à un agent d'aromatisation du cycle A, puis a un agent de saponification ménagée, pour obtenir des produits de formule (I') que le cas échéant, l'on traite comme indiqué ci-dessus pour les produits de formule (I).

**2.** Procédé selon la revendication 1 pour préparer des produits de formule (I) telle que définie à la revendication 1, l'on soumet un composé de formule (II) :

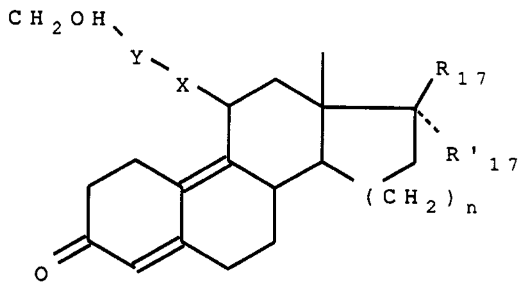

(II)

dans laquelle X, Y, n, $R_{17}$ et $R'_{17}$ ont la même signification qu'à la revendication 1, étant entendu que lorsque $R_{17}$ représente un radical hydroxyle, ce radical est protégé, à l'action d'un agent d'oxydation pour obtenir le produit de formule (III) :

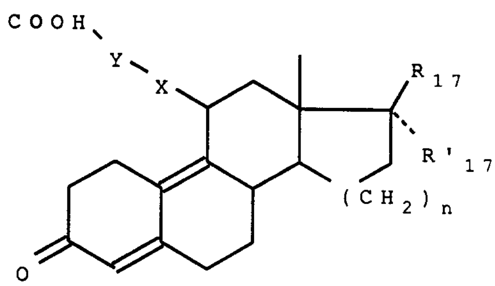

(III)

que l'on soumet à l'action d'un agent permettant d'activer la fonction carboxylique, puis à l'action d'un composé de formule (IV) :

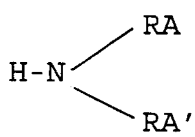

(IV)

dans laquelle $R_A$ et $R_A'$ ont la même signification qu'à la revendication 1, pour obtenir le produit de formule (V) :

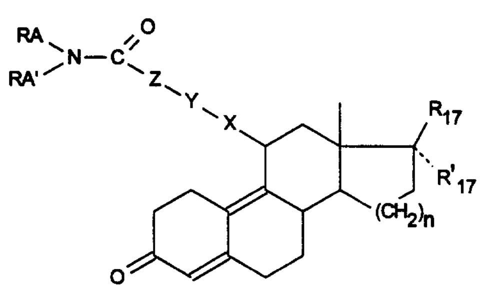

(V)

dans laquelle Z est une simple liaison, produits (V) que, si nécessaire, l'on soumet à une réaction de déprotection de la fonction hydroxy et, si désiré,

- à un agent de réduction lorsque $R_{17}$ et $R'_{17}$ forment ensemble une fonction cétone, puis le cas échéant soumet

à un agent d'acylation le dérivé hydroxylé en 17 ainsi obtenu,

- ou à un agent de saponification lorsque $R_{17}$ représente une fonction acyloxy,

composé de formule (V) que l'on soumet à un agent d'aromatisation du cycle A, puis à un agent de saponification ménagée pour obtenir les produits de formule (I) que, si désiré, l'on soumet à une ou à plusieurs des réactions suivantes :

soit, lorsque $R_{17}$ et $R'_{17}$ forment ensemble une fonction cétone, l'on soumet à un agent de réduction ou à un complexe métallique de formule (VI) :

$$M\text{-}R'_{17} \qquad (VI)$$

dans laquelle M représente un atome métallique et $R'_{17}$ a la même signification qu'à la revendication 1, à l'exception d'hydrogène,
soit, lorsque $R_{17}$ est un radical hydroxyle, l'on soumet à un agent d'acylation sélective en position 17,
soit, lorsque $R_A$ ou $R_A$' est un atome d'hydrogène, on soumet à un agent d'alkylation approprié,
soit, lorsque Y représente une chaine aliphatique insaturée, l'on soumet à un agent d'hydrogénation,
pour obtenir les produits de formule (I) dans laquelle K est un atome d'oxygène que, si désiré, l'on soumet à un agent de sulfuration pour obtenir les produits de formule (I) dans laquelle K est un atome de soufre.

3. Procédé selon la revendication 1 pour préparer des produits de formule (I) dans laquelle X représente un radical arylène et Y représente une chaine aliphatique éventuellement liée au groupe arylène par une double ou une triple liaison et comportant au moins 3 atomes de carbone ou liée au groupe arylène par un atome d'oxygène ou un atome de soufre, l'on soumet un composé de formule (VII) :

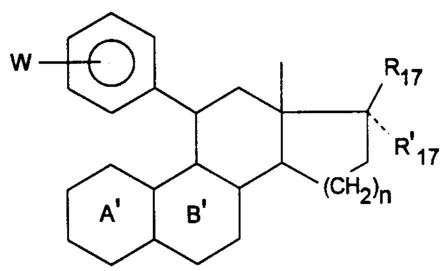

(VII)

dans laquelle W représente soit un radical -OH, soit un radical mercapto éventuellement activé, soit un radical -C≡CH, n, $R_{17}$ et R' ayant la même signification que celle indiquée à la revendication 1, étant entendu que lorsque $R_{17}$ représente un radical -OH, il est éventuellement protégé, les cycles A' et B' sont :

- soit

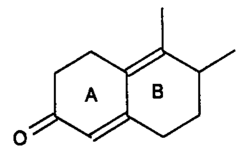

- soit

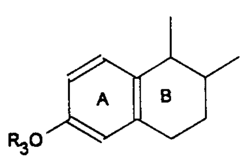

où $R_3$ représente un atome d'hydrogène ou un groupement protecteur de la fonction hydroxyle,

**ou bien**, dans le cas où W représente un radical C≡CH à l'action d'un dérivé halogéné de formule (VIII):

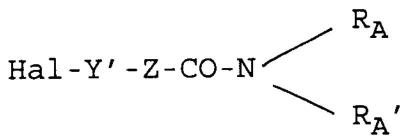

(VIII)

dans laquelle Hal est un atome d'halogène, Z, $R_A$ et $R_A'$ ont la même signification que celle indiquée à la revendication 1 et Y' représente la chaine aliphatique Y ci-dessus comportant 2 atomes de carbone en moins, en présence d'une base forte et soumet le cas échéant à l'action d'un agent de déprotection en 3 et/ou en 17, pour obtenir le produit de formule ($IX_A$) :

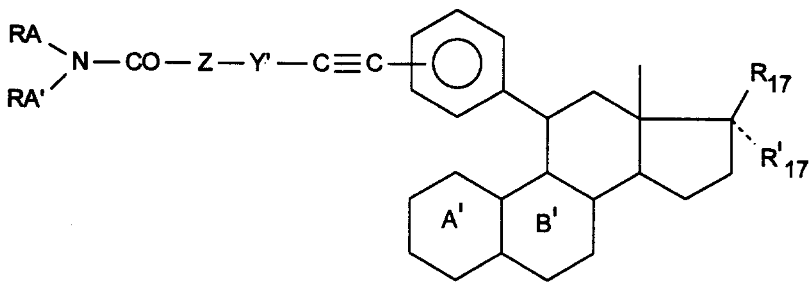

($IX_A$)

produit que, si désiré, l'on soumet à un agent de réduction partielle ou totale de la triple liaison pour obtenir le produit de formule ($IX_B$) :

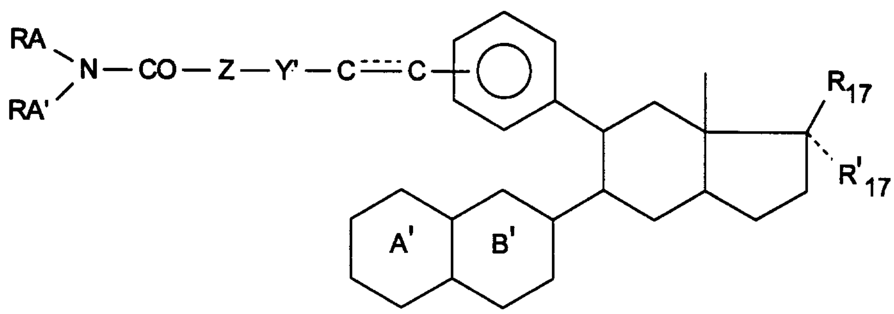

($IX_B$)

**ou bien**, dans le cas où W représente un radical -OH, un radical mercapto ou un radical mercapto activé, à l'action d'un dérivé halogéné de formule (X) :

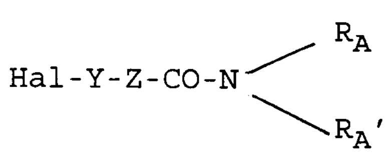

(X)

dans laquelle Hal, Y, Z, $R_A$ et $R_A'$ ont la même signification que celle indiquée à la revendication 1, en présence d'un agent alcalin, puis le cas échéant, à l'action d'un agent de déprotection pour obtenir un produit de formule ($IX_C$) :

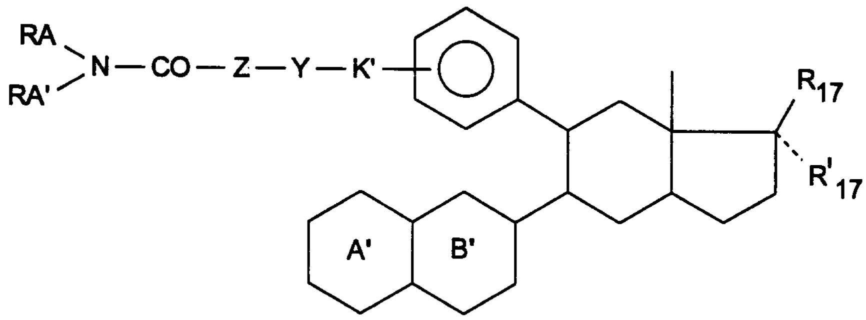

($IX_C$)

dans laquelle K' représente soit un atome d'oxygène, soit un atome de soufre, produits de formule ($IX_A$), ($IX_B$) ou ($IX_C$) que le cas échéant l'on soumet à un agent d'aromatisation du cycle A, puis a un agent de saponification ménagée, pour obtenir des produits de formule (I') que le cas échéant, l'on traite comme indiqué à la revendication 1 pour les produits de formule (I).

4. Procédé selon l'une quelconque des revendications 1 à 3, caractérisé en ce que l'on utilise au départ un produit de formule (II) ou (VII) dans laquelle n = 1.

5. Procédé selon la revendication 4 caractérisé en ce que l'on utilise au départ un produit de formule (II) dans laquelle X est un radical arylèneoxy et Y représente une chaine linéaire saturée renfermant de 5 à 10 atomes de carbone, éventuellement interrompue par un atome d'oxygène ou un produit de formule (VII) dans laquelle W représente un radical OH et un produit de formule (X) dans laquelle Y a la signification indiquée ci-dessus.

6. Procédé selon la revendication 4 caractérisé en ce que l'on utilise au départ un produit de formule (II) dans laquelle X est un radical arylènethio et Y représente une chaine linéaire saturée renfermant de 5 à 10 atomes de carbone ou un produit de formule (VII) dans laquelle W représente un radical mercapto éventuellement activé et un produit de formule (X) dans laquelle Y a la signification indiquée ci-dessus.

7. Procédé selon la revendication 4 caractérisé en ce que l'on utilise au départ un produit de formule (II) dans laquelle X est un radical arylène, Y représente une chaine linéaire insaturée renfermant de 5 à 10 atomes de carbone, et un produit de formule (VII) dans laquelle $R_A$ et $R_{A'}$ forment avec l'atome auquel ils sont liés une pipéridine N-substituée par un radical aralkyle ou un produit de formule (VII) dans laquelle W représente un radical -C≡CH et un produit de formule (VII) dans laquelle $R_A$ et $R_A$' ont la signification indiquée ci-dessus.

**Revendications pour l'Etat contractant suivant : GR**

1. Procédé pour préparer des composés de formule (I) :

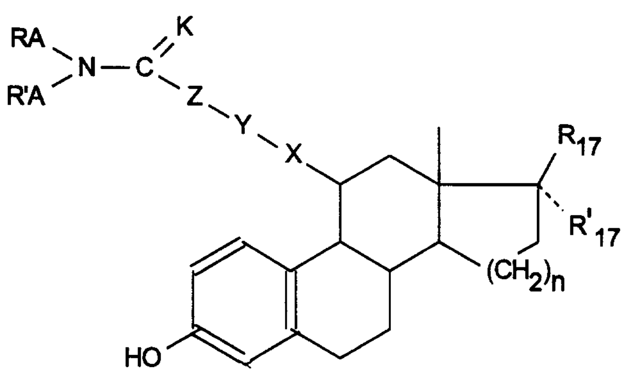

(I)

dans laquelle :
soit

n = 1, K représente un atome d'oxygène,
$R_{17}$ représente un radical hydroxyle ou un radical de formule

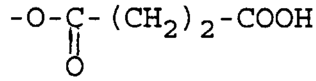

éventuellement salifié,
$R'_{17}$ représente un atome d'hydrogène ou un radical éthynyle,
$R_A$ représente un radical méthyle,
$R'_A$ représente un radical isopropyle ou butyle linéaire,
X représente un radical méthylène, phénylène ou phényloxy lié au stéroïde par un atome de carbone,
Y représente un des radicaux choisis dans le groupe constitué par $-(CH_2)_7$ ; $-(CH_2)_8-$ ; $-(CH_2)_5-C\equiv C-$ ; $-(CH_2)q-O-CH_2-$ avec q = 5 à 7 et $-(CH_2)_5-S-CH_2-$ dans lequel l'atome de soufre est éventuellement oxydé sous forme de sulfoxyde ou de sulfone,
dont les noms suivent :

- le monobutanedioate de 11béta-(4-((7-(butylméthylamino) carbonyl) heptyl) oxy) phényl)-3-hydroxy-estra-1,3,5(10)-trien-17béta-yle,
- le butanedioate de 11béta-(4-((7-((butylméthylamino) carbonyl) heptyl) oxy) phényl)-hydroxy-estra-1,3,5(10)-trien-17béta-yle et de sodium,
- le N-butyl-2-(6-(4-(3,17béta-dihydroxy-estra-1,3,5(10)-trien-11béta-yl)phénoxy)hexyloxy-N-méthyl-acétamide,
- le N-butyl-8-(4-(3,17béta-dihydroxy-estra-1,3,5(10)-trien-11béta-yl)phénoxy)-N-méthyl-2-octynamide,
- le N-butyl-2-((5-(4-(3,17béta-dihydroxy-estra-1,3,5(10)-trien-11béta-yl) phényl) pentyl) thio) N-méthyl acétamide,
- le N-butyl-4-(3,17béta-dihydroxy-estra-1,3,5(10)-trien-11béta-yl)-N-méthyl-benzenenonamide,
- le N-butyl-2-((5-(4(3,17béta-dihydroxy-estra-1,3,5(10)-trien-11béta-yl) phenyl) pentyl) oxy)-N-méthyl-acétamide,
- le 2-((8-(3,17béta-dihydroxy 19-nor-17alpha-pregna-1,3,5(10)-trien-20-yn-11béta-yl) octyl) oxy)-N-méthyl-N-(1-méthyléthyl)-acétamide.

Z représente une simple liaison,

<u>soit</u>

n = 1 ou 2,
K représente un atome d'oxygène ou un atome de soufre, $R_{17}$ et
$R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O, arylènoxy ou arylènethio lié au stéroïde par un atome de carbone,
- Y représente une simple liaison ou une chaine aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison étant entendu que lorsque Y et Z sont une simple liaison, X ne peut-être un radical méthylène ou $CH_2$-O,

$R_A$ ou $R_{A'}$ identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié ou $R_A$ et $R_{A'}$ forment avec l'atome d'azote auquel ils sont liés un hétérocycle à 5 ou 6 chainons, saturé ou non, renfermant éventuellement un ou plusieurs hétéroatomes choisis parmi le groupe constitué par les atomes d'oxygène, d'azote et de soufre et éventuellement substitué par un radical alkyle ayant de 1 à 4 atomes de carbone, un radical aryle ou aralkyle ayant de 6 à 10 atomes de carbone, étant entendu que l'un au moins des radicaux $R_A$ ou $R_{A'}$ n'est pas un atome d'hydrogène et qu'au moins l'une quelconque des conditions suivantes est remplie :

- soit n = 2,
- soit K est un atome de soufre,
- soit X est un radical arylénethio lié au stéroïde par un atome de carbone,
- soit $R_A$ et $R_{A'}$ forment avec l'atome de carbone auquel ils sont liés un hétérocycle à 5 ou 6 chainons substitué par un radical aryle ou aralkyle, caractérisé en ce que :

a) pour préparer des produits de formule (I) telle que définie ci-dessus, l'on soumet un composé de formule (II) :

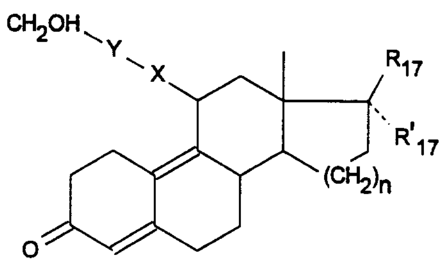

(II)

dans laquelle X, Y, n, $R_{17}$ et $R'_{17}$ ont la même signification que ci-dessus, étant entendu que lorsque $R_{17}$ représente un radical hydroxyle, ce radical est protégé, à l'action d'un agent d'oxydation pour obtenir le produit de formule (III) :

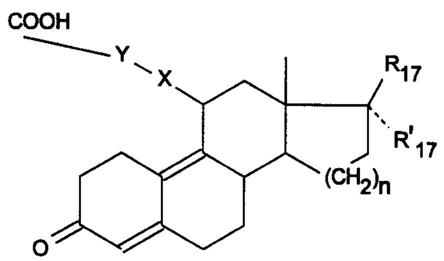

(III)

que l'on soumet à l'action d'un agent permettant d'activer la fonction carboxylique, puis à l'action d'un composé de formule (IV) :

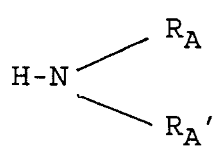

(IV)

dans laquelle $R_A$ et $R_A'$ ont la même signification que ci-dessus, pour obtenir le produit de formule (V) :

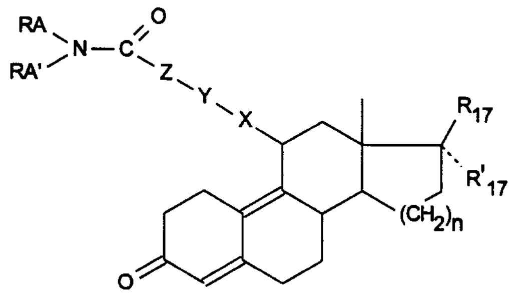

(V)

dans laquelle Z est une simple liaison, produits (V) que, si nécessaire, l'on soumet à une réaction de déprotection de la fonction hydroxy et, si désiré,

- à un agent de réduction lorsque $R_{17}$ et $R'_{17}$ forment ensemble une fonction cétone, puis le cas échéant soumet à un agent d'acylation le dérivé hydroxylé en 17 ainsi obtenu,
- ou à un agent de saponification lorsque $R_{17}$ représente une fonction acyloxy,

composé de formule (V) que l'on soumet à un agent d'aromatisation du cycle A, puis à un agent de saponification ménagée pour obtenir les produits de formule (I) que, si désiré, l'on soumet à une ou à plusieurs des réactions suivantes :

soit, lorsque $R_{17}$ et $R'_{17}$ forment ensemble une fonction cétone, l'on soumet à un agent de réduction ou à un complexe métallique de formule (VI) :

$$M\text{-}R'_{17} \qquad (VI)$$

dans laquelle M représente un atome métallique et $R'_{17}$ a la même signification que ci-dessus, à l'exception d'hydrogène,
soit, lorsque $R_{17}$ est un radical hydroxyle, l'on soumet à un agent d'acylation sélective en position 17,
soit, lorsque $R_A$ ou $R_A'$ est un atome d'hydrogène, on soumet à un agent d'alkylation approprié,
soit, lorsque Y représente une chaine aliphatique insaturée, l'on soumet à un agent d'hydrogénation,
pour obtenir les produits de formule (I) dans laquelle K est un atome d'oxygène que, si désiré, l'on soumet à un agent de sulfuration pour obtenir les produits de formule (I) dans laquelle K est un atome de soufre,

b) pour préparer des produits de formule (I) dans laquelle X représente un radical arylène et Y représente une chaine aliphatique éventuellement liée au groupe arylène par une double ou une triple liaison et comportant au moins 3 atomes de carbone ou liée au groupe arylène par un atome d'oxygène ou un atome de soufre, l'on soumet un composé de formule (VII) :

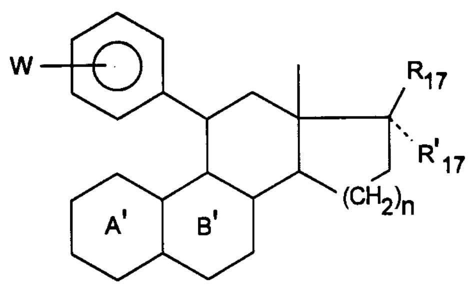

(VII)

dans laquelle W représente soit un radical -OH, soit un radical mercapto éventuellement activé, soit un radical -C≡CH, n, $R_{17}$ et R' ayant la même signification que celle indiquée ci-dessus, étant entendu que lorsque $R_{17}$ représente un radical -OH, il est éventuellement protégé, les cycles A' et B' sont :

- soit

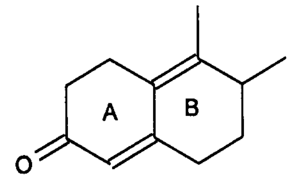

- soit

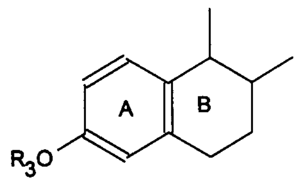

où $R_3$ représente un atome d'hydrogène ou un groupement protecteur de la fonction hydroxyle,

**ou bien**, dans le cas où W représente un radical C≡CH à l'action d'un dérivé halogéné de formule (VIII) :

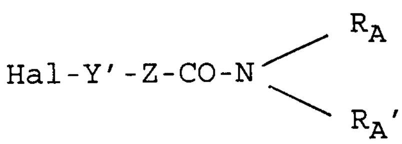

(VIII)

dans laquelle Hal est un atome d'halogène, Z, $R_A$ et $R_A$' ont la même signification que celle indiquée ci-dessus et Y' représente la chaine aliphatique Y ci-dessus comportant 2 atomes de carbone en moins, en présence d'une base forte et soumet le cas échéant à l'action d'un agent de déprotection en 3 et/ou en 17, pour obtenir le produit de formule ($IX_A$) :

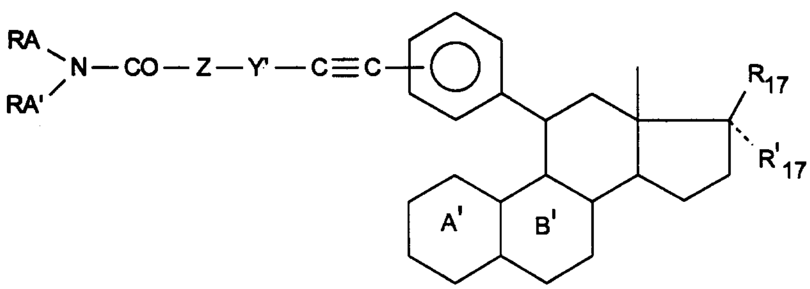

($IX_A$)

produit que, Si désiré, l'on soumet à un agent de réduction partielle ou totale de la triple liaison pour obtenir le produit de formule ($IX_B$) :

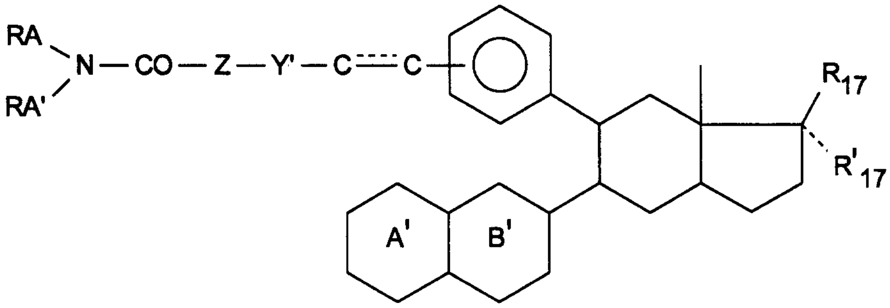

$(IX_B)$

**ou bien**, dans le cas où W représente un radical -OH, un radical mercapto ou un radical mercapto activé, à l'action d'un dérivé halogéné de formule (X) :

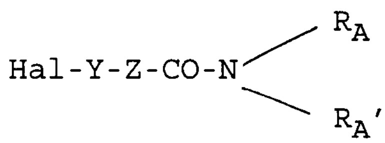

(X)

dans laquelle Hal, Y, Z, $R_A$ et $R_A'$ ont la même signification que celle indiquée ci-dessus, en présence d'un agent alcalin, puis le cas échéant, à l'action d'un agent de déprotection pour obtenir un produit de formule $(IX_C)$ :

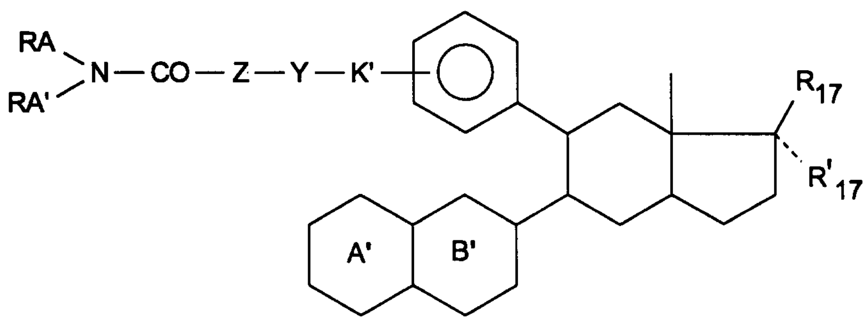

$(IX_C)$

dans laquelle K' représente soit un atome d'oxygène, soit un atome de soufre, produits de formule $(IX_A)$, $(IX_B)$ ou $(IX_C)$ que le cas échéant l'on soumet à un agent d'aromatisation du cycle A, puis a un agent de saponification ménagée, pour obtenir des produits de formule (I') que le cas échéant, l'on traite comme indiqué ci-dessus pour les produits de formule (I).

2. Procédé selon la revendication 1 pour préparer des produits de formule (I) telle que définie à la revendication 1, l'on soumet un composé de formule (II) :

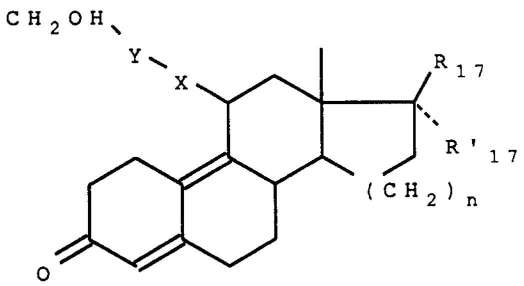

(II)

dans laquelle X, Y, n, $R_{17}$ et $R'_{17}$ ont la même signification qu'à la revendication 1, étant entendu que lorsque $R_{17}$ représente un radical hydroxyle, ce radical est protégé, à l'action d'un agent d'oxydation pour obtenir le produit de formule (III) :

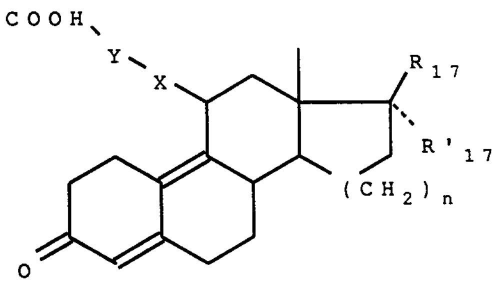

(III)

que l'on soumet à l'action d'un agent permettant d'activer la fonction carboxylique, puis à l'action d'un composé de formule (IV) :

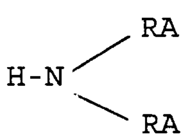

(IV)

dans laquelle $R_A$ et $R_A$' ont la même signification qu'à la revendication 1, pour obtenir le produit de formule (V) :

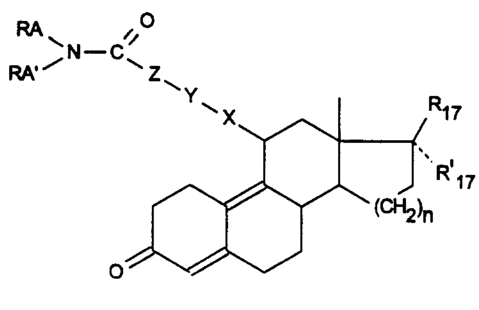

(V)

dans laquelle Z est une simple liaison, produits (V) que, si nécessaire, l'on soumet à une réaction de déprotection de la fonction hydroxy et, si désiré,

- à un agent de réduction lorsque $R_{17}$ et $R'_{17}$ forment ensemble une fonction cétone, puis le cas échéant soumet à un agent d'acylation le dérivé hydroxylé en 17 ainsi obtenu,
- ou à un agent de saponification lorsque $R_{17}$ représente une fonction acyloxy,

composé de formule (V) que l'on soumet à un agent d'aromatisation du cycle A, puis à un agent de saponification ménagée pour obtenir les produits de formule (I) que, si désiré, l'on soumet à une ou à plusieurs des réactions sui-

vantes :

soit, lorsque $R_{17}$ et $R'_{17}$ forment ensemble une fonction cétone, l'on soumet à un agent de réduction ou à un complexe métallique de formule (VI) :

$$M\text{-}R'_{17} \quad (VI)$$

dans laquelle M représente un atome métallique et $R'_{17}$ a la même signification qu'à la revendication 1, à l'exception d'hydrogène,
soit, lorsque $R_{17}$ est un radical hydroxyle, l'on soumet à un agent d'acylation sélective en position 17,
soit, lorsque $R_A$ ou $R_A$' est un atome d'hydrogène, on soumet à un agent d'alkylation approprié,
soit, lorsque Y représente une chaine aliphatique insaturée, l'on soumet à un agent d'hydrogénation,
pour obtenir les produits de formule (I) dans laquelle K est un atome d'oxygène que, si désiré, l'on soumet à un agent de sulfuration pour obtenir les produits de formule (I) dans laquelle K est un atome de soufre.

**3.** Procédé selon la revendication 1 pour préparer des produits de formule (I) dans laquelle X représente un radical arylène et Y représente une chaine aliphatique éventuellement liée au groupe arylène par une double ou une triple liaison et comportant au moins 3 atomes de carbone ou liée au groupe arylène par un atome d'oxygène ou un atome de soufre, l'on soumet un composé de formule (VII) :

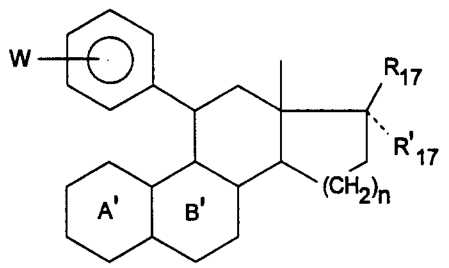

(VII)

dans laquelle W représente soit un radical -OH, soit un radical mercapto éventuellement activé, soit un radical -C≡CH, n, $R_{17}$ et R' ayant la même signification que celle indiquée à la revendication 1, étant entendu que lorsque $R_{17}$ représente un radical -OH, il est éventuellement protégé, les cycles A' et B' sont :

- soit

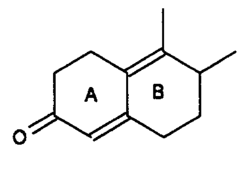

- soit

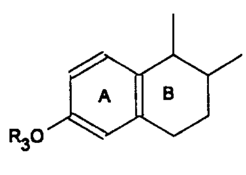

où $R_3$ représente un atome d'hydrogène ou un groupement protecteur de la fonction hydroxyle,

**ou bien**, dans le cas où W représente un radical C≡CH à l'action d'un dérivé halogéné de formule (VIII) :

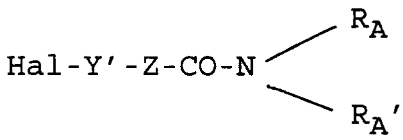

(VIII)

dans laquelle Hal est un atome d'halogène, Z, $R_A$ et $R_A$' ont la même signification que celle indiquée à la revendication 1 et Y' représente la chaine aliphatique Y ci-dessus comportant 2 atomes de carbone en moins, en présence d'une base forte et soumet le cas échéant à l'action d'un agent de déprotection en 3 et/ou en 17, pour obtenir le produit de formule ($IX_A$) :

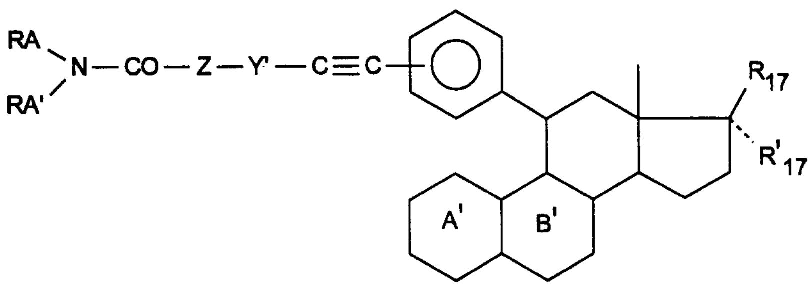

($IX_A$)

produit que, si désiré, l'on soumet à un agent de réduction partielle ou totale de la triple liaison pour obtenir le produit de formule ($IX_B$) :

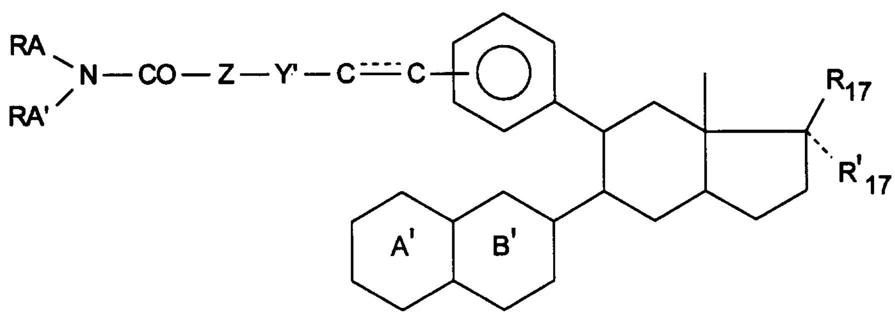

($IX_B$)

**ou bien**, dans le cas où W représente un radical -OH, un radical mercapto ou un radical mercapto activé, à l'action d'un dérivé halogéné de formule (X) :

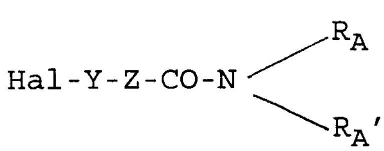

(X)

dans laquelle Hal, Y, Z, $R_A$ et $R_A$' ont la même signification que celle indiquée à la revendication 1, en présence d'un agent alcalin, puis le cas échéant, à l'action d'un agent de déprotection pour obtenir un produit de formule ($IX_C$) :

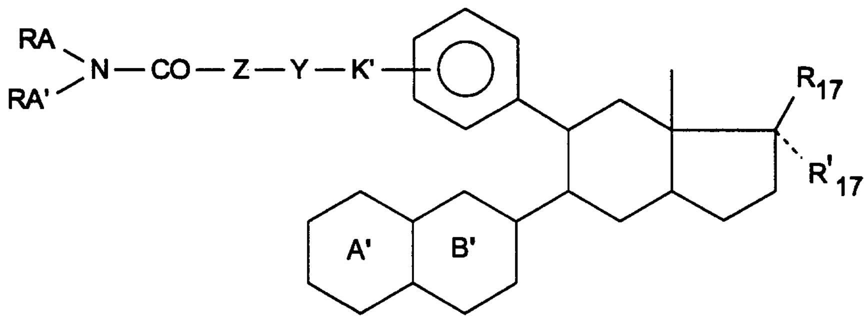

$(IX_C)$

dans laquelle K' représente soit un atome d'oxygène, soit un atome de soufre, produits de formule ($IX_A$), ($IX_B$) ou ($IX_C$) que le cas échéant l'on soumet à un agent d'aromatisation du cycle A, puis a un agent de saponification ménagée, pour obtenir des produits de formule (I') que le cas échéant, l'on traite comme indiqué à la revendication 1 pour les produits de formule (I).

**4.** Procédé selon l'une quelconque des revendications 1 à 3, caractérisé en ce que l'on utilise au départ un produit de formule (II) ou (VII) dans laquelle n = 1.

**5.** Procédé selon la revendication 4 caractérisé en ce que l'on utilise au départ un produit de formule (II) dans laquelle X est un radical arylèneoxy et Y représente une chaine linéaire saturée renfermant de 5 à 10 atomes de carbone, éventuellement interrompue par un atome d'oxygène ou un produit de formule (VII) dans laquelle W représente un radical OH et un produit de formule (X) dans laquelle Y a la signification indiquée ci-dessus.

**6.** Procédé selon la revendication 4 caractérisé en ce que l'on utilise au départ un produit de formule (II) dans laquelle X est un radical arylènethio et Y représente une chaine linéaire saturée renfermant de 5 à 10 atomes de carbone ou un produit de formule (VII) dans laquelle W représente un radical mercapto éventuellement activé et un produit de formule (X) dans laquelle Y a la signification indiquée ci-dessus.

**7.** Procédé selon la revendication 4 caractérisé en ce que l'on utilise au départ un produit de formule (II) dans laquelle X est un radical arylène, Y représente une chaine linéaire insaturée renfermant de 5 à 10 atomes de carbone, et un produit de formule (VII) dans laquelle $R_A$ et $R_{A'}$ forment avec l'atome auquel ils sont liés une pipéridine N-substituée par un radical aralkyle ou un produit de formule (VII) dans laquelle W représente un radical -C≡CH et un produit de formule (VII) dans laquelle $R_A$ et $R_A$' ont la signification indiquée ci-dessus.

**8.** Procédé selon la revendication 1, caractérisé en ce que l'on choisit les produits de départ de manière telle que l'on prépare l'un quelconque des produits de formule (I) dont les noms suivent :

- le N-butyl-5-(4-(3,17béta-dihydroxy-estra-1,3,5(10)-trien-11béta-yl) phénoxy) pentyloxy)-N-méthyl-éthanethioamide,
- le N-butyl-8-(4-(3,17béta-dihydroxy-estra-1,3,5(10)-trien-11béta-yl) phénoxy)-N-méthyl-octanethioamide.

**9.** Procédé de préparation de compositions pharmaceutiques, caractérisé en ce que l'on met à titre de principe actif l'un au moins des produits de formule (I) tels que définis à la revendication 1 sous une forme destinée à cet usage.

**10.** Procédé de préparation de compositions pharmaceutiques, caractérisé en ce que l'on met à titre de principe actif l'un au moins des produits de formule (I) tels qu'obtenus à l'une quelconque des revendications 4 à 8 sous une forme destinée à cet usage.

**11.** A titre de produits intermédiaires nouveaux, les produits de formule (II) et (III) telles que définies à la revendication 1 ou 2 dans lesquelles :

n = 2.

**12.** A titre de produits intermédiaires nouveaux les produits de formule (VII) telle que définie à la revendication 1 ou 3 dans laquelle W représente un radical mercapto éventuellement activé.

## Claims

### Claims for the following Contracting States : AT, BE, CH, DE, DK, FR, GB, IT, LI, LU, NL, SE

**1.** The compounds of formula (I):

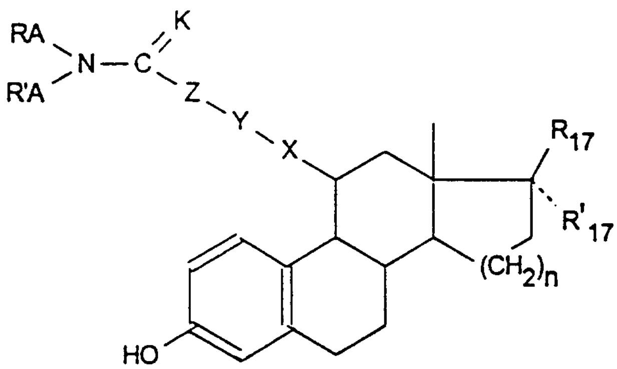

(I)

in which:
<u>either</u>

n = 1, K represents an oxygen atom,
$R_{17}$ represents a hydroxyl radical or a radical of formula

$$-O-\underset{\underset{O}{\|}}{C}-(CH_2)_2-COOH$$

optionally salified,
$R'_{17}$ represents a hydrogen atom or an ethynyl radical,
$R_A$ represents a methyl radical,
$R'_A$ represents an isopropyl or linear butyl radical
X represents a methylene, phenylene or phenyloxy radical linked to the steroid by a carbon atom,
Y represents one of the radicals chosen from the group constituted by $-(CH_2)_7$; $-(CH_2)_8-$; $-(CH_2)_5-C{\equiv}C-$; $-(CH_2)q-O-CH_2-$ with q = 5 to 7 and $-(CH_2)_5-S-CH_2-$ in which the sulphur atom is optionally oxidized in the form of the sulphoxide or sulphone,
the names of which follow:

- 11beta-(4-((7-(butylmethylamino) carbonyl) heptyl) oxy) phenyl)-3-hydroxy-estra-1,3,5(10)-trien-17beta-yl monobutanedioate,
- 11beta-(4-((7-((butylmethylamino) carbonyl) heptyl) oxy) phenyl)-hydroxy-estra-1,3,5(10)-trien-17beta-yl and sodium butanedioate,
- N-butyl-2-(6-(4-(3,17beta-dihydroxy-estra-1,3,5(10)-trien-11beta-yl) phenoxy) hexyloxy-N-methyl acetamide,
- N-butyl-8-(4-(3,17beta-dihydroxy-estra-1,3,5(10)-trien-11beta-yl) phenoxy)-N-methyl-2-octynamide,
- N-butyl-2-((5-(4-(3,17beta-dihydroxy-estra-1,3,5(10)-trien-11beta-yl) phenyl) pentyl) thio)-N-methyl acetamide,
- N-butyl-4-(3,17beta-dihydroxy-estra-1,3,5(10)-trien-11beta-yl)-N-methyl benzenenonamide,
- N-butyl-2-((5-(4-(3,17beta-dihydroxy-estra-1,3,5(10)-trien-11beta-yl) phenyl) pentyl) oxy)-N-methyl acetamide,
- 2-((8-(3,17beta-dihydroxy-19-nor-17alpha-pregna-1,3,5(10)-(trien-20-yn-11beta-yl) octyl) oxy)-N-methyl-N-(1-methylethyl) acetamide,

Z represents a single bond,

<u>or</u>

n = 1 or 2
K represents an oxygen or sulphur atom, $R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms,

X, Y and Z are such that:

- X represents a methylene radical, an arylene group, a $CH_2$-O radical, an arylenoxy or arylenethio radical linked to the steroid by a carbon atom,
- Y represents a single bond or a saturated or unsaturated, linear or branched aliphatic chain, containing 1 to 18 carbon atoms, optionally interrupted by one or more radicals chosen from arylene, oxygen or sulphur radicals optionally oxidized in the form of the sulphoxide or sulphone and optionally terminated by an arylene radical,
- Z represents a single bond, it being understood that when Y and Z are a single bond, X cannot be a methylene or $CH_2$-O radical,

$R_A$ or $R_{A'}$, identical or different, represent a hydrogen atom, a linear or branched alkyl radical containing 1 to 8 carbon atoms, optionally substituted by an aryl, alkyl or dialkylamino, hydroxy, halogen or an esterified carboxyl radical or $R_A$ and $R_{A'}$ form together with the nitrogen atom to which they are linked a heterocycle with 5 or 6 members, saturated or not, optionally containing one or more heteroatoms chosen from the group constituted by oxygen, nitrogen and sulphur atoms and optionally substituted by an alkyl radical having 1 to 4 carbon atoms, an aryl or aralkyl radical having 6 to 10 carbon atoms, it being understood that at least one of the $R_A$ or $R_{A'}$ radicals is not a hydrogen atom and that at least any one of the following conditions is fulfilled:

- either n = 2,
- or K is a sulphur atom,
- or X is an arylenethio radical linked to the steroid by a carbon atom,
- or $R_A$ and $R_{A'}$ form together with the carbon atom to which they are linked a heterocycle with 5 or 6 members substituted by an aryl or aralkyl radical.

**2.** The products corresponding to formula ($I_A$) which corresponds to formula (I) as defined in claim 1 in which:

n = 1 or 2,
K represents an oxygen or sulphur atom, $R_{17}$ and $R'_{17}$ are such that:

- either $R_{17}$ and $R'_{17}$ together form a ketone function,
- or $R_{17}$ is a hydroxyl or acyloxy radical and $R'_{17}$ represents an hydrogen atom, an optionally substituted alkyl, alkenyl or alkynyl radical having at most 8 carbon atoms, X, Y and Z are such that:
- X represents a methylene radical, an arylene group, a $CH_2$-O, arylenoxy or arylenethio radical linked to the steroid by a carbon atom,
- Y represents a single bond or a saturated or unsaturated, linear or branched aliphatic chain containing 1 to 18 carbon atoms, optionally interrupted by one or more radicals chosen from arylene or oxygen radicals or sulphur radicals optionally oxidized in the form of the sulphoxide or sulphone and optionally terminated by an arylene radical,
- Z represents a single bond, it being understood that when Y and Z are a single bond, X cannot be a methylene or $CH_2$-O radical,

$R_A$ or $R_{A'}$, identical or different, represent a hydrogen atom, a linear or branched alkyl radical containing 1 to 8 carbon atoms, optionally substituted by an aryl, alkyl or dialkylamino, hydroxy, halogen radical or an esterified carboxyl radical or $R_A$ and $R_{A'}$ form with the nitrogen atom to which they are linked a heterocycle with 5 or 6 members, saturated or not, optionally containing one or more heteroatoms chosen from the group constituted by oxygen, nitrogen and sulphur atoms and optionally substituted by an alkyl radical having 1 to 4 carbon atoms, an aryl or aralkyl radical having 6 to 10 carbon atoms, it being understood that at least one of the $R_A$ or $R_{A'}$ radicals is not a hydrogen atom and that at least any one of the following conditions is fulfilled:

- either n = 2,

- or K is a sulphur atom,
- or X is an arylenethio radical linked to the steroid by a carbon atom,
- or $R_A$ and $R_{A'}$ form with the carbon atom to which they are linked a heterocycle with 5 or 6 members substituted by an aryl or aralkyl radical.

**3.** The products corresponding to formula ($I_A$) as defined in claim 2 in which n is equal to 1.

**4.** The products according to claim 3 in which K is a sulphur atom, X is an aryleneoxy radical and Y represents a saturated linear chain containing 5 to 10 carbon atoms, optionally interrupted by an oxygen atom.

**5.** The products according to claim 3 in which X is an arylenethio radical and Y represents a saturated linear chain containing 5 to 10 carbon atoms.

**6.** The products according to claim 3 in which X is an arylene radical, Y represents an unsaturated linear chain containing 5 to 10 carbon atoms, $R_A$ and $R_{A'}$ form with the atom to which they linked a piperidine N-substituted by an aralkyl radical.

**7.** The products of formula (I) according to claim 1 the names of which follow:

- N-butyl-5-(4-(3,17beta-dihydroxy-estra-1,3,5(10)-trien-11beta-yl) phenoxy) pentyloxy)-N-methyl-ethanethioamide,
- N-butyl-8-(4-(3,17beta-dihydroxy-estra-1,3,5(10)-trien-11beta-yl) phenoxy)-N-methyl-octanethioamide,

**8.** Preparation process for the products of formula (I) as defined in claim 1, characterized in that a compound of formula (II):

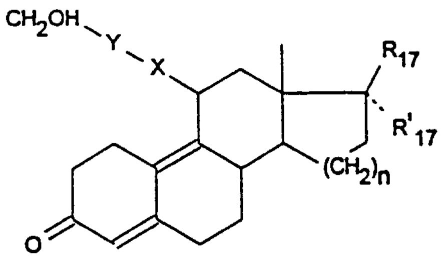

(II)

in which X, Y, n, $R_{17}$ and $R'_{17}$ have the same meaning as in claim 1, it being understood that when $R_{17}$ represents a hydroxyl radical, this radical is protected, is subjected to the action of an oxidation agent in order to obtain the product of formula (III):

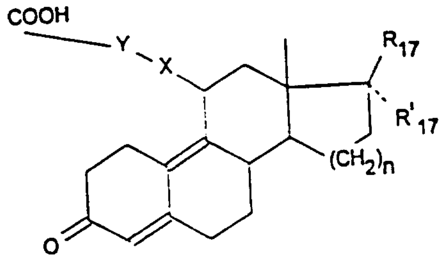

(III)

which is subjected to the action of an agent which allows the carboxylic function to be activated, then to the action of a compound of formula (IV):

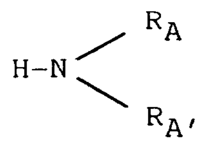

(IV)

in which $R_A$ and $R_{A'}$ have the same meaning as in claim 1, in order to obtain the product of formula (V):

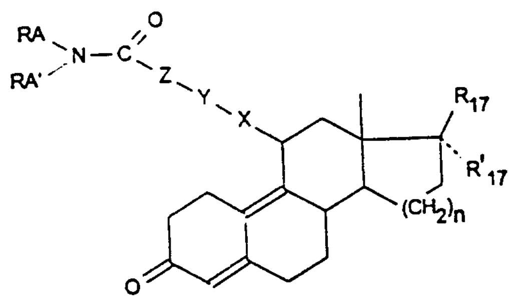

(V)

in which Z is a single bond, which products (V), if necessary, are subjected to a deprotection reaction of the hydroxy function and, if desired,

- to a reducing agent when $R_{17}$ and $R'_{17}$ together form a ketone function, then if appropriate, the hydroxylated derivative in position 17 thus obtained is subjected to an acylation agent,
- or to a saponification agent when $R_{17}$ represents an acyloxy function,

which compound of formula (V) is subjected to an aromatization agent of the A ring, then to a controlled saponification agent so as to obtain the products of formula (I) which, if desired, are subjected to one or more of the following reactions:

either, when $R_{17}$ and $R'_{17}$ together form a ketone function, they are subjected to a reducing agent or to a metal complex of formula (VI):

$$M\text{-}R'_{17} \qquad \text{(VI)}$$

in which M represents a metal atom and $R'_{17}$ has the same meaning as in claim 1, with the exception of hydrogen,
or , when $R_{17}$ is a hydroxyl radical, they are subjected to a selective acylation agent in position 17,
or, when $R_A$ or $R_{A'}$ is a hydrogen atom, they are subjected to an appropriate alkylation agent,
or, when Y represents an unsaturated aliphatic chain, they are subjected to a hydrogenation agent,
in order to obtain the products of formula (I) in which K is an oxygen atom which, if desired, are subjected to a sulphuration agent in order to obtain the products of formula (I) in which K is a sulphur atom.

**9.** Preparation process for products of formula (I') corresponding to products of formula (I) as defined in claim 1 in which X represents an arylene radical and Y represents an aliphatic chain optionally linked to the arylene group by a double or a triple bond and containing at least 3 carbon atoms or linked to the arylene group by an oxygen or sulphur atom, characterized in that a compound of formula (VII):

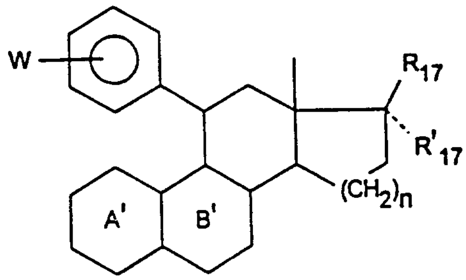

(VII)

in which W represents either an -OH radical, or an optionally activated mercapto radical, or a -C≡CH radical, n, $R_{17}$ and R' having the same meaning as indicated in claim 1, it being understood that when $R_{17}$ represents an -OH radical, it is optionally protected, the A' and B' rings are:

- either

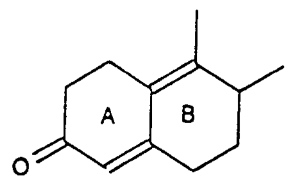

- or

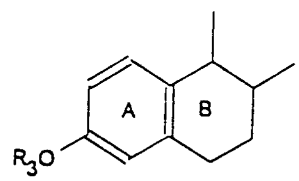

where $R_3$ represents a hydrogen atom or a protective group of the hydroxyl function, is subjected

**either**, in the case where W represents a C≡CH radical, to the action of a halogenated derivative of formula (VIII):

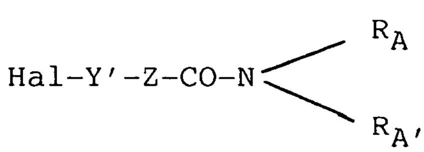

(VIII)

in which Hal is a halogen atom, Z, $R_A$ and $R_{A'}$ have the same meaning as indicated in claim 1 and Y' represents the above aliphatic chain Y comprising at least 2 carbon atoms, in the presence of a strong base and if appropriate, is subjected to the action of a deprotection agent in position 3 and/or position 17, in order to obtain the product of formula ($IX_A$):

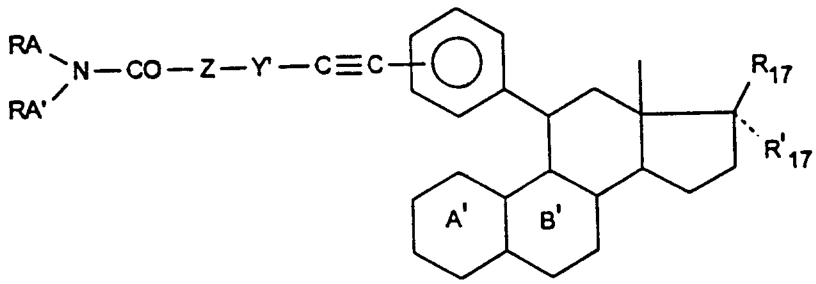

$(IX_A)$

which product, if desired, is subjected to an agent giving partial or complete reduction of the triple bond in order to obtain the product of formula ($IX_B$):

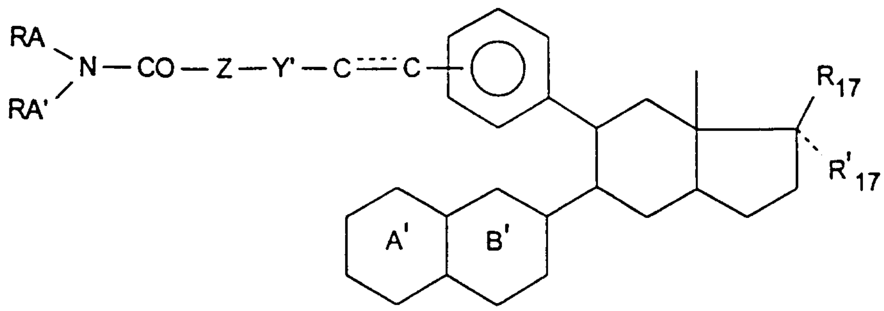

$(IX_B)$

**or**, in the case where W represents an -OH radical, a mercapto radical or an activated mercapto radical, to the action of a halogenated derivative of formula (X):

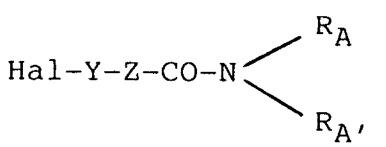

(X)

in which Hal, Y, Z, $R_A$ and $R_{A'}$ have the same meaning as indicated in claim 1, in the presence of an alkaline agent, then if appropriate, to the action of a deprotection agent in order to obtain a product of formula ($IX_C$):

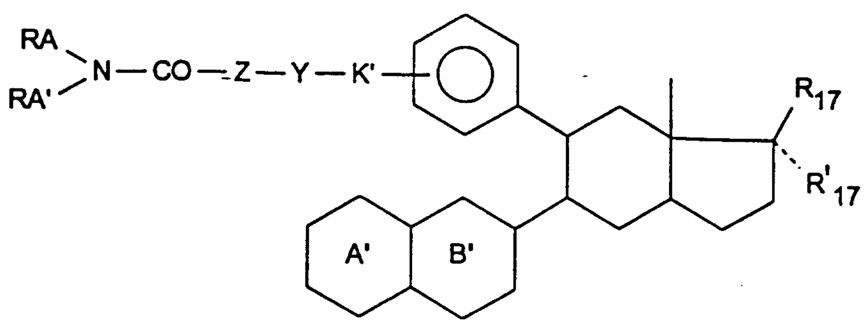

$(IX_C)$

in which K' represents either an oxygen atom, or a sulphur atom, which products of formula ($IX_A$), ($IX_B$) or ($IX_C$) are, if appropriate, subjected to an aromatization agent of ring A, then to a controlled saponification in order to obtain the products of formula (I') which, if appropriate, are treated as indicated in claim 8 for the products of formula (I).

**10.** As medicaments, the products of formula (I) and as defined in any one of claims 1 to 7.

**11.** The pharmaceutical compositions containing as active ingredient at least one of the medicaments as defined in claim 10.

**12.** As new intermediate products, the products of formula (II) and (III) as defined in claim 8 in which:

n = 2.

**13.** As new intermediate products, the products of formula (VII) as defined in claim 9 in which W represents an optionally activated mercapto radical.

**Claims for the following Contracting State : ES**

**1.** Preparation process for the compounds of formula (I):

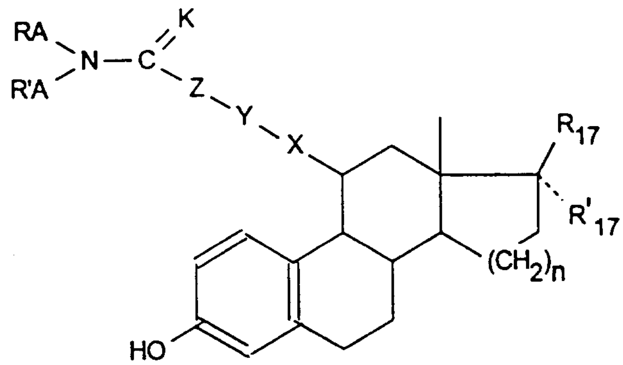

(I)

in which:
<u>either</u>

n = 1, K represents an oxygen atom,
$R_{17}$ represents a hydroxyl radical or a radical of formula

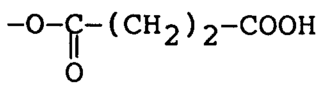

optionally salified,
$R'_{17}$ represents a hydrogen atom or an ethynyl radical,
$R_A$ represents a methyl radical,
$R'_A$ represents an isopropyl or linear butyl radical X represents a methylene, phenylene or phenyloxy radical linked to the steroid by a carbon atom,
Y represents one of the radicals chosen from the group constituted by $-(CH_2)_7$; $-(CH_2)_8$-; $-(CH_2)_5-C{\equiv}C$-; $-(CH_2)q-O-CH_2$- with q = 5 to 7 and $-(CH_2)_5-S-CH_2$- in which the sulphur atom is optionally oxidized in the form of the sulphoxide or sulphone,
the names of which follow:

- 11beta-(4-((7-(butylmethylamino) carbonyl) heptyl) oxy) phenyl)-3-hydroxy-estra-1,3,5(10)-trien-17beta-yl monobutanedioate,
- 11beta-(4-((7-((butylmethylamino) carbonyl) heptyl) oxy) phenyl)-hydroxy-estra-1,3,5(10)-trien-17beta-yl and sodium butanedloate,
- N-butyl-2-(6-(4-(3,17beta-dihydroxy-estra-1,3,5(10)-trien-11beta-yl) phenoxy) hexyloxy-N-methyl acetamide,
- N-butyl-8-(4-(3,17beta-dihydroxy-estra-1,3,5(10)-trien-11beta-yl) phenoxy)-N-methyl-2-octynamide,
- N-butyl-2-((5-(4-(3,17beta-dihydroxy-estra-1,3,5(10)-trien-11beta-yl) phenyl) pentyl) thio) N-methyl acetamide,

- N-butyl-4-(3,17beta-dihydroxy-estra-1,3,5(10)-trien-11beta-yl)-N-methyl benzenenonamide,
- N-butyl-2-((5-(4-(3,17beta-dihydroxy-estra-1,3,5(10)-trien-11beta-yl) phenyl) pentyl) oxy)-N-methyl acetamide,
- 2-((8-(3,17beta-dihydroxy 19-nor-17alpha-pregna-1,3,5(10)-(trien-20-yn-11beta-yl) octyl) oxy)-N-methyl-N-(1-methylethyl) acetamide,

Z represents a single bond,

or

n = 1 or 2
K represents an oxygen or sulphur atom, $R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms,

X, Y and Z are such that:

- X represents a methylene radical, an arylene group, a $CH_2$-O radical, an arylenoxy or arylenethio radical linked to the steroid by a carbon atom,
- Y represents a single bond or a saturated or unsaturated, linear or branched aliphatic chain, containing 1 to 18 carbon atoms, optionally interrupted by one or more radicals chosen from arylene, oxygen or sulphur radicals optionally oxidized in the form of the sulphoxide or sulphone and optionally terminated by an arylene radical,
- Z represents a single bond, it being understood that when Y and Z are a single bond, X cannot be a methylene or $CH_2$-O radical,

$R_A$ or $R_{A'}$, identical or different, represent a hydrogen atom, a linear or branched alkyl radical containing 1 to 8 carbon atoms, optionally substituted by an aryl, alkyl or dialkylamino, hydroxy, halogen or an esterified carboxyl radical or $R_A$ and $R_{A'}$ form together with the nitrogen atom to which they are linked a heterocycle with 5 or 6 members, saturated or not, optionally containing one or more heteroatoms chosen from the group constituted by oxygen, nitrogen and sulphur atoms and optionally substituted by an alkyl radical having 1 to 4 carbon atoms, an aryl or aralkyl radical having 6 to 10 carbon atoms, it being understood that at least one of the $R_A$ or $R_{A'}$ radicals is not a hydrogen atom and that at least any one of the following conditions is fulfilled:

- either n = 2,
- or K is a sulphur atom,
- or X is an arylenethio radical linked to the steroid by a carbon atom,
- or $R_A$ and $R_{A'}$ form together with the carbon atom to which they are linked a heterocycle with 5 or 6 members substituted by an aryl or aralkyl radical, characterized in that:

a) to prepare the products of formula (I) as defined above, a compound of formula (II):

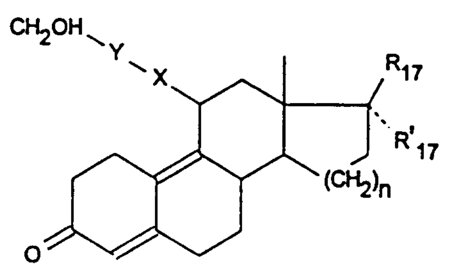

(II)

in which X, Y, n, $R_{17}$ and $R'_{17}$ have the same meaning as above, it being understood that when $R_{17}$ represents a hydroxyl radical, this radical is protected, is subjected to the action of an oxidation agent in order to obtain the product of formula (III):

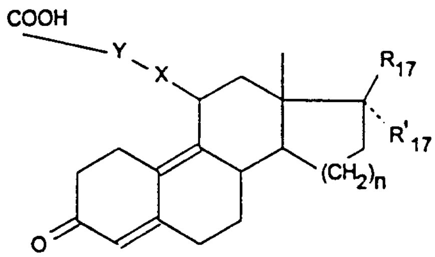

(III)

which is subjected to the action of an agent which allows the carboxylic function to be activated, then to the action of a compound of formula (IV):

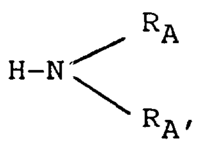

(IV)

in which $R_A$ and $R_{A'}$ have the same meaning as above, in order to obtain the product of formula (V):

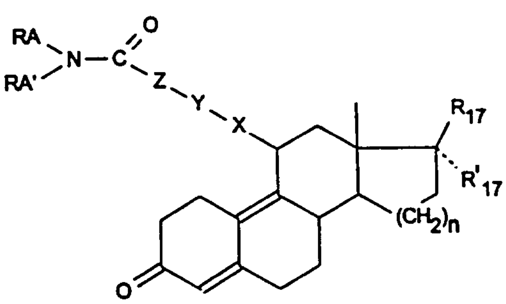

(V)

in which Z is a single bond, which products (V), if necessary, are subjected to a deprotection reaction of the hydroxy function and, if desired,

- to a reducing agent when $R_{17}$ and $R'_{17}$ together form a ketone function, then if appropriate, the hydroxylated derivative in position 17 thus obtained is subjected to an acylation agent,
- or to a saponification agent when $R_{17}$ represents an acyloxy function,

which compound of formula (V) is subjected to an aromatization agent of the A ring, then to a controlled saponification agent so as to obtain the products of formula (I) which, if desired, are subjected to one or more of the following reactions:

either, when $R_{17}$ and $R'_{17}$ together form a ketone function, they are subjected to a reducing agent or to a metal complex of formula (VI):

$$M\text{-}R'_{17} \qquad (VI)$$

in which M represents a metal atom and $R'_{17}$ has the same meaning as above, with the exception of hydrogen,
or , when $R_{17}$ is a hydroxyl radical, they are subjected to a selective acylation agent in position 17,
or, when $R_A$ or $R_{A'}$ is a hydrogen atom, they are subjected to an appropriate alkylation agent,
or, when Y represents an unsaturated aliphatic chain, they are subjected to a hydrogenation agent,
in order to obtain the products of formula (I) in which K is an oxygen atom which, if desired, are subjected to a sulphuration agent in order to obtain the products of formula (I) in which K is a sulphur atom,

b) to prepare the products of formula (I) in which X represents an arylene radical and Y represents an aliphatic chain optionally linked to the arylene group by a double or a triple bond and containing at least 3 carbon atoms or linked to the arylene group by an oxygen or sulphur atom, a compound of formula (VII):

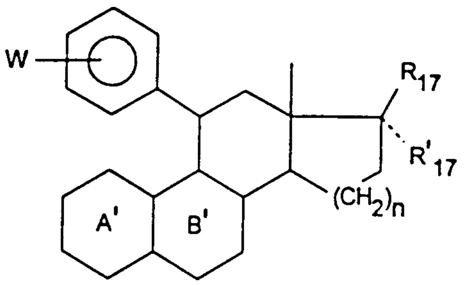

(VII)

in which W represents either an -OH radical, or an optionally activated mercapto radical, or a -C≡CH radical, n, $R_{17}$ and R' having the same meaning as indicated above, it being understood that when $R_{17}$ represents an -OH radical, it is optionally protected, the A' and B' rings are:

- either

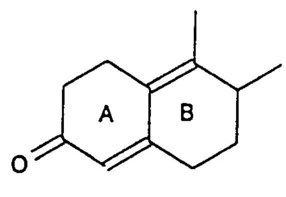

- or

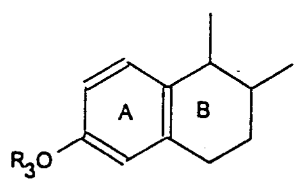

where $R_3$ represents a hydrogen atom or a protective group of the hydroxyl function,

is subjected

**either**, in the case where W represents a C≡CH radical, to the action of a halogenated derivative of formula (VIII):

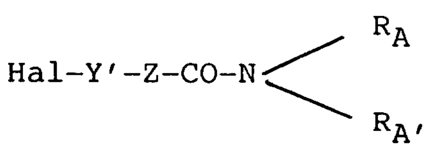

(VIII)

in which Hal is a halogen atom, Z, $R_A$ and $R_{A'}$ have the same meaning as indicated above and Y' represents the above aliphatic chain Y comprising at least 2 carbon atoms, in the presence of a strong base and if appropriate, is subjected to the action of a deprotection agent in position 3 and/or position 17, in order to obtain the product of formula ($IX_A$):

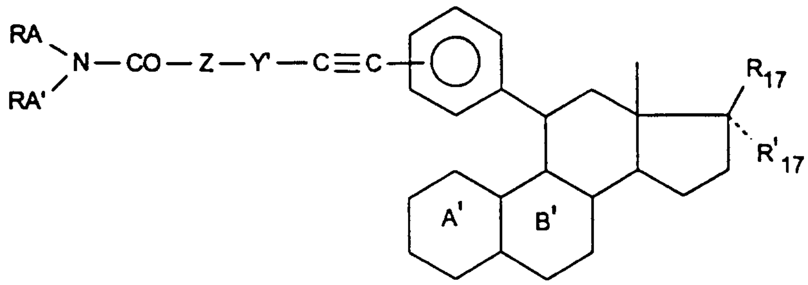

($IX_A$)

which product, if desired, is subjected to an agent giving partial or complete reduction of the triple bond in order to obtain the product of formula ($IX_B$):

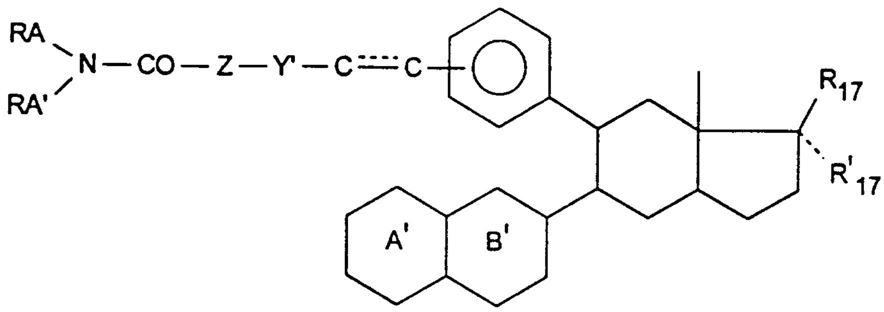

($IX_B$)

**or**, in the case where W represents an -OH radical, a mercapto radical or an activated mercapto radical, to the action of a halogenated derivative of formula (X):

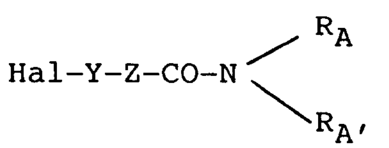

(X)

in which Hal, Y, Z, $R_A$ and $R_{A'}$ have the same meaning as indicated above, in the presence of an alkaline agent, then if appropriate, to the action of a deprotection agent in order to obtain a product of formula ($IX_C$):

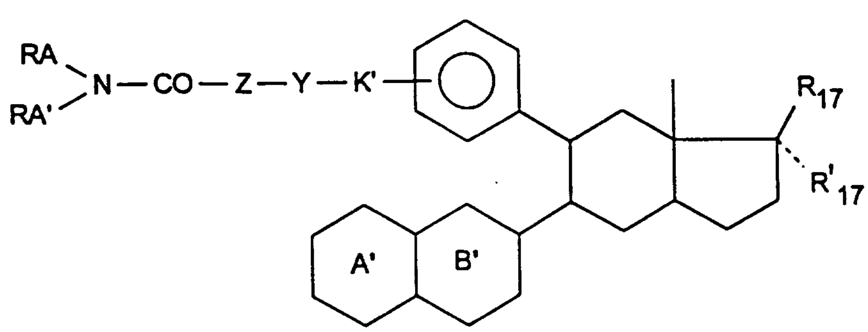

($IX_C$)

in which K' represents either an oxygen atom, or a sulphur atom, which products of formula ($IX_A$), ($IX_B$) or ($IX_C$) are, if appropriate, subjected to an aromatization agent of ring A, then to a controlled saponification in order to

obtain the products of formula (I') which, if appropriate, are treated as indicated above for the products of formula (I).

2. Process according to claim 1 for the preparation of products of formula (I) as defined in claim 1, a compound of formula (II):

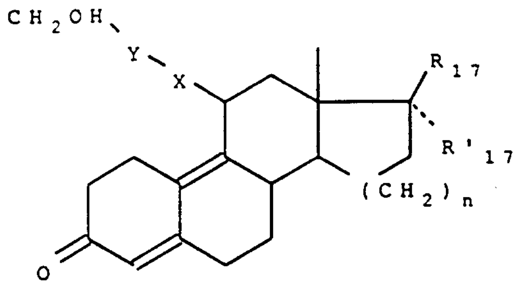

(II)

in which X, Y, n, $R_{17}$ and $R'_{17}$ have the same meaning as in claim 1, it being understood that when $R_{17}$ represents a hydroxyl radical, this radical is protected, is subjected to the action of an oxidation agent in order to obtain the product of formula (III):

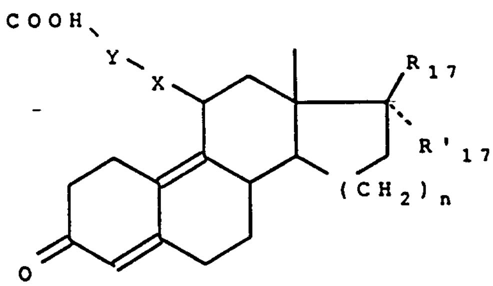

(III)

which is subjected to the action of an agent which allows the carboxylic function to be activated, then to the action of a compound of formula (IV):

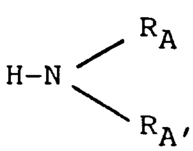

(IV)

in which $R_A$ and $R_{A'}$ have the same meaning as in claim 1, in order to obtain the product of formula (V):

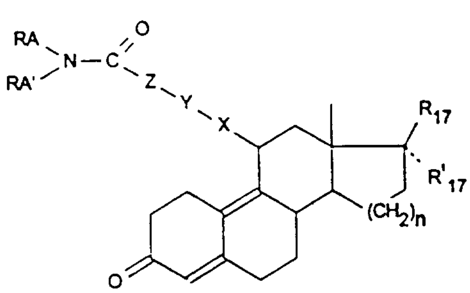

(V)

in which Z is a single bond, which products (V), if necessary, are subjected to a deprotection reaction of the hydroxy function and, if desired,

- to a reducing agent when $R_{17}$ and $R'_{17}$ together form a ketone function, then if appropriate, the hydroxylated derivative in position 17 thus obtained is subjected to an acylation agent,
- or to a saponification agent when $R_{17}$ represents an acyloxy function,

which compound of formula (V) is subjected to an aromatization agent of the A ring, then to a controlled saponification agent so as to obtain the products of formula (I) which, if desired, are subjected to one or more of the following reactions:

either, when $R_{17}$ and $R'_{17}$ together form a ketone function, they are subjected to a reducing agent or to a metal complex of formula (VI):

$$M\text{-}R'_{17} \qquad \text{(VI)}$$

in which M represents a metal atom and $R'_{17}$ has the same meaning as in claim 1, with the exception of hydrogen,
or , when $R_{17}$ is a hydroxyl radical, they are subjected to a selective acylation agent in position 17,
or, when $R_A$ or $R_{A'}$ is a hydrogen atom, they are subjected to an appropriate alkylation agent,
or, when Y represents an unsaturated aliphatic chain, they are subjected to a hydrogenation agent,
in order to obtain the products of formula (I) in which K is an oxygen atom which, if desired, are subjected to a suiphuration agent in order to obtain the products of formula (I) in which K is a sulphur atom.

**3.** Process according to claim 1 for the preparation of products of formula (I) in which X represents an arylene radical and Y represents an aliphatic chain optionally linked to the arylene group by a double or a triple bond and containing at least 3 carbon atoms or linked to the arylene group by an oxygen or sulphur atom, a compound of formula (VII):

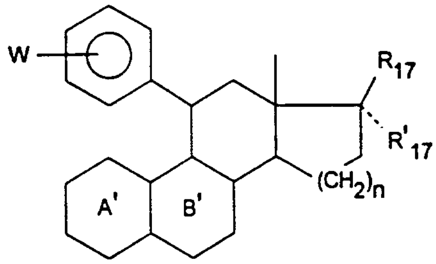

(VII)

in which W represents either an -OH radical, or an optionally activated mercapto radical, or a -C≡CH radical, n, $R_{17}$ and R' having the same meaning as indicated in claim 1, it being understood that when $R_{17}$ represents an -OH radical, it is optionally protected, the A' and B' rings are:

- either

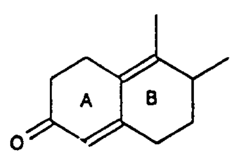

- or

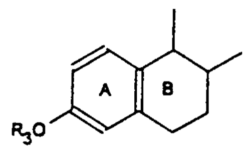

where $R_3$ represents a hydrogen atom or a protective group of the hydroxyl function, is subjected

**either**, in the case where W represents a C≡CH radical, to the action of a halogenated derivative of formula (VIII):

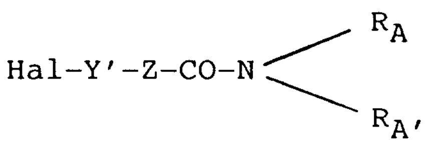

(VIII)

in which Hal is a halogen atom, Z, $R_A$ and $R_{A'}$ have the same meaning as indicated in claim 1 and Y' represents the above aliphatic chain Y comprising at least 2 carbon atoms, in the presence of a strong base and if appropriate, is subjected to the action of a deprotection agent in position 3 and/or position 17, in order to obtain the product of formula ($IX_A$):

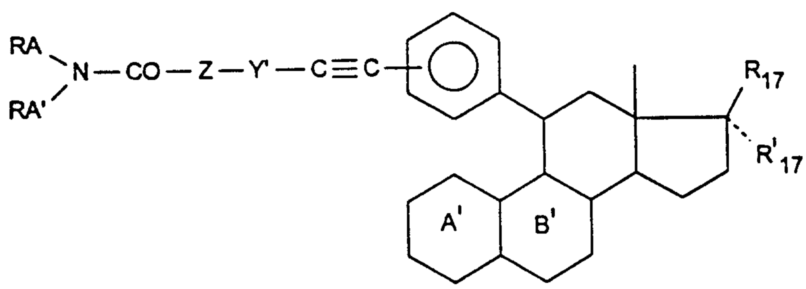

($IX_A$)

which product, if desired, is subjected to an agent giving partial or complete reduction of the triple bond in order to obtain the product of formula ($IX_B$):

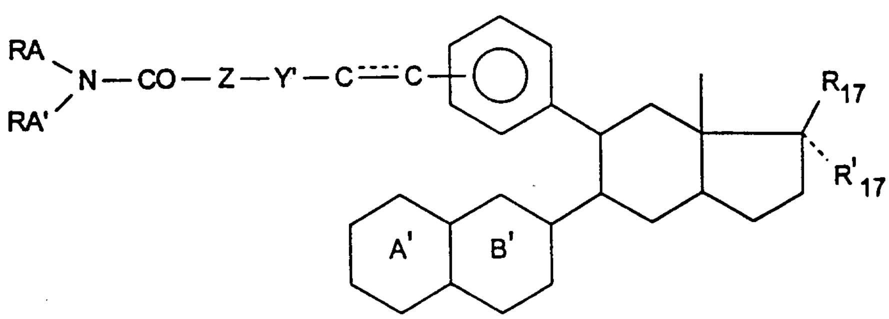

($IX_B$)

**or**, in the case where W represents an -OH radical, a mercapto radical or an activated mercapto radical, to the action of a halogenated derivative of formula (X):

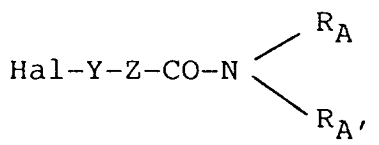

(X)

in which Hal, Y, Z, $R_A$ and $R_{A'}$ have the same meaning as indicated in claim 1, in the presence of an alkaline agent, then if appropriate, to the action of a deprotection agent in order to obtain a product of formula ($IX_C$):

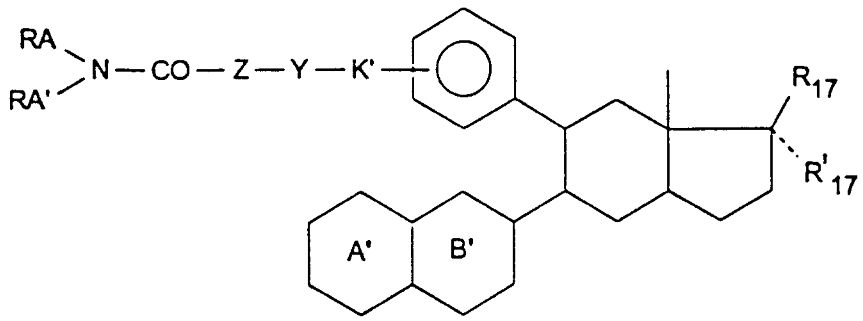

($IX_C$)

in which K' represents either an oxygen atom, or a sulphur atom, which products of formula ($IX_A$), ($IX_B$) or ($IX_C$) are, if appropriate, subjected to an aromatization agent of ring A, then to a controlled saponification in order to obtain the products of formula (I') which, if appropriate, are treated as indicated in claim 1 for the products of formula (I).

**4.** Process according to any one of claim 1 to 3, characterized in that a product of formula (II) or (VII) in which n = 1 is used at the start.

**5.** Process according to claim 4, characterized in that a product of formula (II) in which X is an aryleneoxy radical and Y represents a saturated linear chain containing 5 to 10 carbon atoms, optionally interupted by an oxygen atom or a product of formula (VII) in which W represents an OH radical and a product of formula (X) in which Y has the meaning indicated above are used at the start.

**6.** Process according to claim 4, characterized in that a product of formula (II) in which X is an arylenethio radical and Y represents a saturated linear chain containing 5 to 10 carbon atoms or a product of formula (VII) in which W represents an optionally activiated mercapto radical and a product of formula (X) in which Y has the meaning indicated above are used at the start.

**7.** Process according to claim 4, characterized in that a product of formula (II) in which X is an arylene radical, Y represents an unsaturated linear chain containing 5 to 10 carbon atoms and a product of formula (VII) in which $R_A$ and $R_{A'}$ form with the atom to which they are linked a piperidine radical N-substituted by an aralkyl radical or a product of formula (VII) in which W represents a -C=CH radical and a product of formula (VII) in which $R_A$ and $R_{A'}$ have the meaning indicated above are used at the start.

**Claims for the following Contracting State : GR**

**1.** Preparation process for the compounds of formula (I):

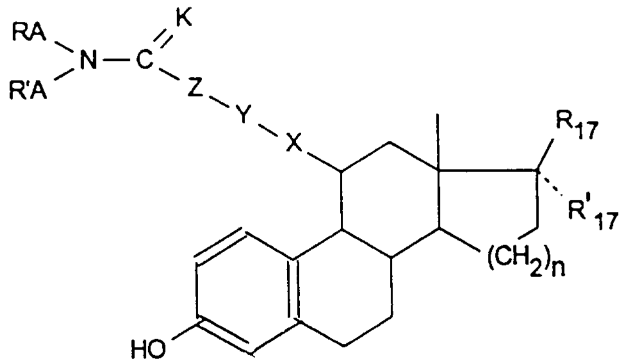

(I)

in which:

<u>either</u>

n = 1, K represents an oxygen atom,

$R_{17}$ represents a hydroxyl radical or a radical of formula

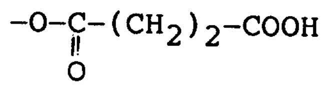

optionally salified,

$R'_{17}$ represents a hydrogen atom or an ethynyl radical,

$R_A$ represents a methyl radical,

$R'_A$ represents an isopropyl or linear butyl radical

X represents a methylene, phenylene or phenyloxy radical linked to the steroid by a carbon atom,

Y represents one of the radicals chosen from the group constituted by $-(CH_2)_7$; $-(CH_2)_8-$; $(CH_2)_5-C{\equiv}C-$; $-(CH_2)q-O-CH_2-$ with q = 5 to 7 and $-(CH_2)_5-S-CH_2-$ in which the sulphur atom is optionally oxidized in the form of the sulphoxide or sulphone,

the names of which follow:

- 11beta-(4-((7-(butylmethylamino) carbonyl) heptyl) oxy) phenyl)-3-hydroxy-estra-1,3,5(10)-trien-17beta-yl monobutanedioate,
- 11beta-(4-((7-((butylmethylamino) carbonyl) heptyl) oxy) phenyl)-hydroxy-estra-1,3,5(10)-trien-17beta-yl and sodium butanedloate,
- N-butyl-2-(6-(4-(3,17beta-dihydroxy-estra-1,3,5(10)-trien-11beta-yl) phenoxy) hexyloxy-N-methyl acetamide,
- N-butyl-8-(4-(3,17beta-dihydroxy-estra-1,3,5(10)-trien-11beta-yl) phenoxy)-N-methyl-2-octynamide,
- N-butyl-2-((5-(4-(3,17beta-dihydroxy-estra-1,3,5(10)-trien-11beta-yl) phenyl) pentyl) thio) N-methyl acetamide,
- N-butyl-4-(3,17beta-dihydroxy-estra-1,3,5(10)-trien-11beta-yl)-N-methyl benzenenonamide,
- N-butyl-2-((5-(4-(3,17beta-dihydroxy-estra-1,3,5(10)-trien-11beta-yl) phenyl) pentyl) oxy)-N-methyl acetamide,
- 2-((8-(3,17beta-dihydroxy 19-nor-17alpha-pregna-1,3,5(10)-(trien-20-yn-11beta-yl) octyl) oxy)-N-methyl-N-(1-methylethyl) acetamide,

Z represents a single bond,

<u>or</u>

n = 1 or 2

K represents an oxygen or sulphur atom, $R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms,

X, Y and Z are such that:

- X represents a methylene radical, an arylene group, a $CH_2$-O radical, an arylenoxy or arylenethio radical linked to the steroid by a carbon atom,
- Y represents a single bond or a saturated or unsaturated, linear or branched aliphatic chain, containing 1 to 18 carbon atoms, optionally interrupted by one or more radicals chosen from arylene, oxygen or sulphur radicals optionally oxidized in the form of the sulphoxide or sulphone and optionally terminated by an arylene radical,
- Z represents a single bond, it being understood that when Y and Z are a single bond, X cannot be a methylene or $CH_2$-O radical,

$R_A$ or $R_{A'}$, identical or different, represent a hydrogen atom, a linear or branched alkyl radical containing 1 to 8 carbon atoms, optionally substituted by an aryl, alkyl or dialkylamino, hydroxy, halogen or an esterified carboxyl radical or $R_A$ and $R_{A'}$ form together with the nitrogen atom to which they are linked a heterocycle with 5 or 6 members, saturated or not, optionally containing one or more heteroatoms chosen from the group constituted by oxygen, nitrogen and sulphur atoms and optionally substituted by an alkyl radical having 1 to 4 carbon atoms, an aryl or aralkyl radical having 6 to 10 carbon atoms, it being understood that at least one of the $R_A$ or $R_{A'}$ radicals is not a hydrogen atom and that at least any one of the following conditions is fulfilled:

- either n = 2,
- or K is a sulphur atom,
- or X is an arylenethio radical linked to the steroid by a carbon atom,
- or $R_A$ and $R_{A'}$ form together with the carbon atom to which they are linked a heterocycle with 5 or 6 members substituted by an aryl or aralkyl radical, characterized in that:

a) to prepare the products of formula (I) as defined above, a compound of formula (II):

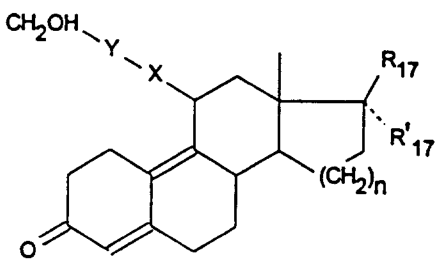

(II)

in which X, Y, n, $R_{17}$ and $R'_{17}$ have the same meaning as above, it being understood that when $R_{17}$ represents a hydroxyl radical, this radical is protected, is subjected to the action of an oxidation agent in order to obtain the product of formula (III):

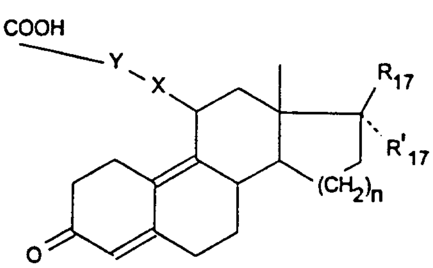

(III)

which is subjected to the action of an agent which allows the carboxylic function to be activated, then to the action of a compound of formula (IV):

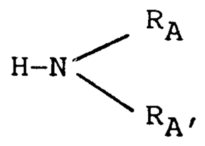

(IV)

in which $R_A$ and $R_{A'}$ have the same meaning as above, in order to obtain the product of formula (V):

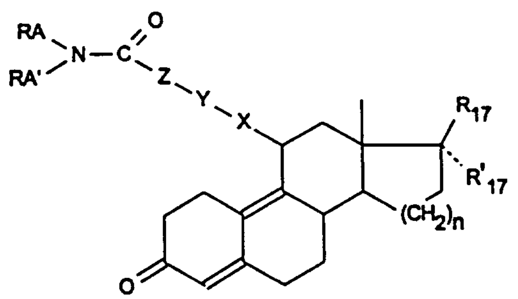

(V)

in which Z is a single bond, which products (V), if necessary, are subjected to a deprotection reaction of the hydroxy function and, if desired,

- to a reducing agent when $R_{17}$ and $R'_{17}$ together form a ketone function, then if appropriate, the hydroxylated derivative in position 17 thus obtained is subjected to an acylation agent,
- or to a saponification agent when $R_{17}$ represents an acyloxy function,

which compound of formula (V) is subjected to an aromatization agent of the A ring, then to a controlled saponification agent so as to obtain the products of formula (I) which, if desired, are subjected to one or more of the following reactions:

either, when $R_{17}$ and $R'_{17}$ together form a ketone function, they are subjected to a reducing agent or to a metal complex of formula (VI):

$$M\text{-}R'_{17} \qquad \text{(VI)}$$

in which M represents a metal atom and $R'_{17}$ has the same meaning as above, with the exception of hydrogen,
or , when $R_{17}$ is a hydroxyl radical, they are subjected to a selective acylation agent in position 17,
or, when $R_A$ or $R_{A'}$ is a hydrogen atom, they are subjected to an appropriate alkylation agent,
or, when Y represents an unsaturated aliphatic chain, they are subjected to a hydrogenation agent,
in order to obtain the products of formula (I) in which K is an oxygen atom which, if desired, are subjected to a sulphuration agent in order to obtain the products of formula (I) in which K is a sulphur atom,

b) to prepare the products of formula (I) in which X represents an arylene radical and Y represents an aliphatic chain optionally linked to the arylene group by a double or a triple bond and containing at least 3 carbon atoms or linked to the arylene group by an oxygen or sulphur atom, a compound of formula (VII):

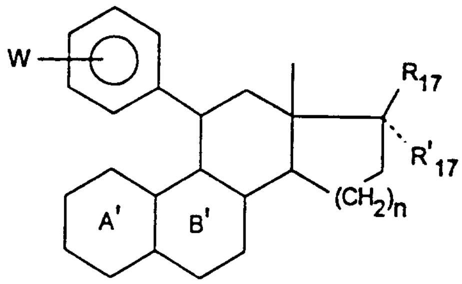

(VII)

in which W represents either an -OH radical, or an optionally activated mercapto radical, or a -C≡CH radical, n, $R_{17}$ and R' having the same meaning as indicated above, it being understood that when $R_{17}$ represents an -OH radical, it is optionally protected, the A' and B' rings are:

- either

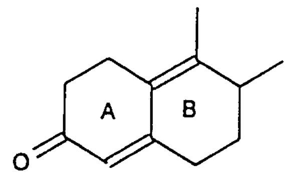

- or

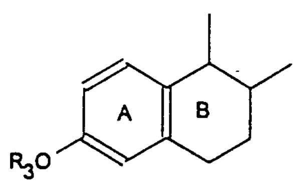

where $R_3$ represents a hydrogen atom or a protective group of the hydroxyl function,

is subjected

**<u>either</u>**, in the case where W represents a C≡CH radical, to the action of a halogenated derivative of formula (VIII):

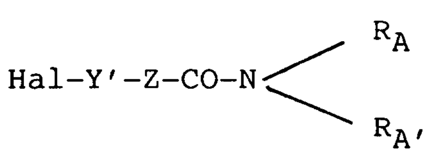

(VIII)

in which Hal is a halogen atom, Z, $R_A$ and $R_{A'}$ have the same meaning as indicated above and Y' represents the above aliphatic chain Y comprising at least 2 carbon atoms, in the presence of a strong base and if appropriate, is subjected to the action of a deprotection agent in position 3 and/or position 17, in order to obtain the product of formula ($IX_A$):

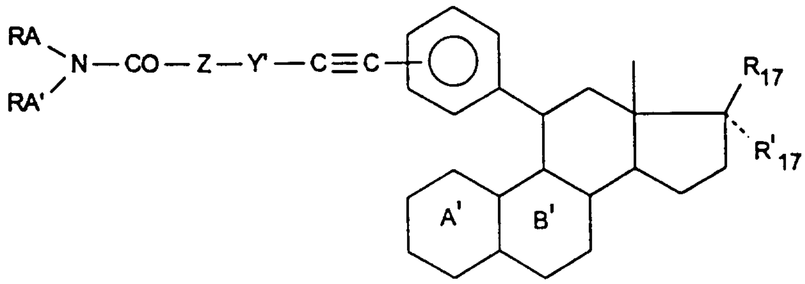

$(IX_A)$

which product, if desired, is subjected to an agent giving partial or complete reduction of the triple bond in order to obtain the product of formula ($IX_B$):

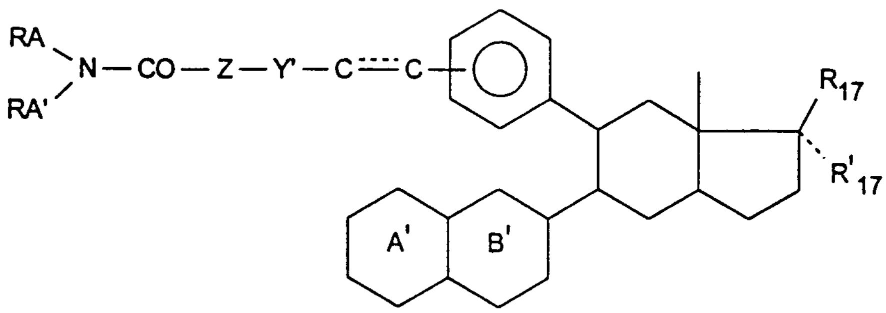

$(IX_B)$

**<u>or</u>**, in the case where W represents an -OH radical, a mercapto radical or an activated mercapto radical, to the action of a halogenated derivative of formula (X):

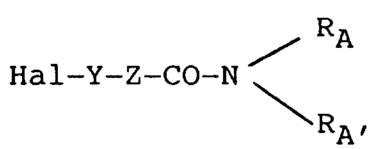

(X)

in which Hal, Y, Z, $R_A$ and $R_{A'}$ have the same meaning as indicated above, in the presence of an alkaline agent, then if appropriate, to the action of a deprotection agent in order to obtain a product of formula ($IX_C$):

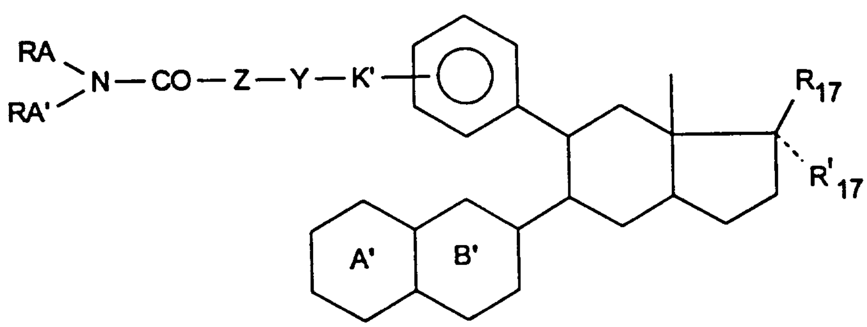

$(IX_C)$

in which K' represents either an oxygen atom, or a sulphur atom, which products of formula ($IX_A$), ($IX_B$) or ($IX_C$) are, if appropriate, subjected to an aromatization agent of ring A, then to a controlled saponification in order to obtain the products of formula (I') which, if appropriate, are treated as indicated above for the products of formula (I).

**2.** Process according to claim 1 for the preparation of products of formula (I) as defined in claim 1, a compound of formula (II):

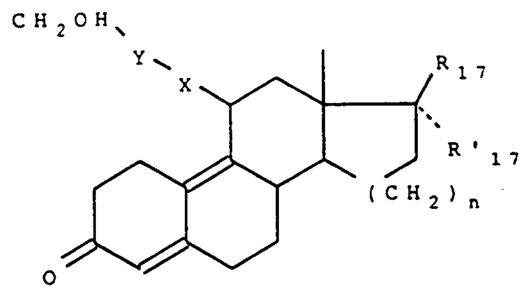

(II)

in which X, Y, n, $R_{17}$ and $R'_{17}$ have the same meaning as in claim 1, it being understood that when $R_{17}$ represents a hydroxyl radical, this radical is protected, is subjected to the action of an oxidation agent in order to obtain the product of formula (III):

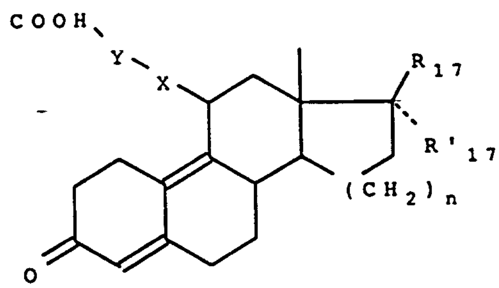

(III)

which is subjected to the action of an agent which allows the carboxylic function to be activated, then to the action of a compound of formula (IV):

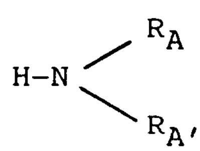

(IV)

in which $R_A$ and $R_{A'}$ have the same meaning as in claim 1, in order to obtain the product of formula (V):

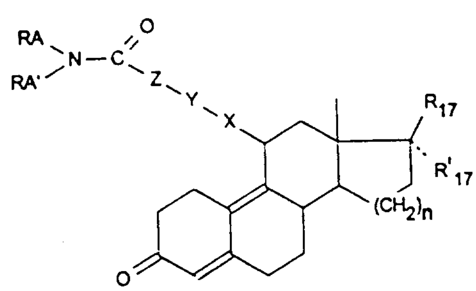

(V)

in which Z is a single bond, which products (V), if necessary, are subjected to a deprotection reaction of the hydroxy function and, if desired,

- to a reducing agent when $R_{17}$ and $R'_{17}$ together form a ketone function, then if appropriate, the hydroxylated derivative in position 17 thus obtained is subjected to an acylation agent,

- or to a saponification agent when $R_{17}$ represents an acyloxy function,

which compound of formula (V) is subjected to an aromatization agent of the A ring, then to a controlled saponification agent so as to obtain the products of formula (I) which, if desired, are subjected to one or more of the following reactions:

either, when $R_{17}$ and $R'_{17}$ together form a ketone function, they are subjected to a reducing agent or to a metal complex of formula (VI):

$$M\text{-}R'_{17} \qquad \text{(VI)}$$

in which M represents a metal atom and $R'_{17}$ has the same meaning as in claim 1, with the exception of hydrogen,
or , when $R_{17}$ is a hydroxyl radical, they are subjected to a selective acylation agent in position 17,
or, when $R_A$ or $R_{A'}$ is a hydrogen atom, they are subjected to an appropriate alkylation agent,
or, when Y represents an unsaturated aliphatic chain, they are subjected to a hydrogenation agent,
in order to obtain the products of formula (I) in which K is an oxygen atom which, if desired, are subjected to a suiphuration agent in order to obtain the products of formula (I) in which K is a sulphur atom.

**3.** Process according to claim 1 for the preparation of products of formula (I) in which X represents an arylene radical and Y represents an aliphatic chain optionally linked to the arylene group by a double or a triple bond and containing at least 3 carbon atoms or linked to the arylene group by an oxygen or sulphur atom, a compound of formula (VII):

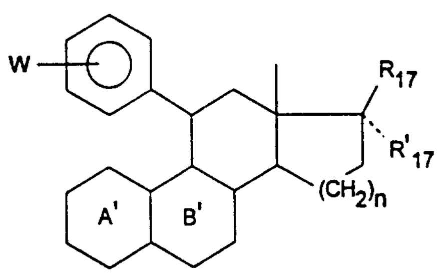

(VII)

in which W represents either an -OH radical, or an optionally activated mercapto radical, or a -C≡CH radical, n, $R_{17}$ and R' having the same meaning as indicated in claim 1, it being understood that when $R_{17}$ represents an -OH radical, it is optionally protected, the A' and B' rings are:

- either

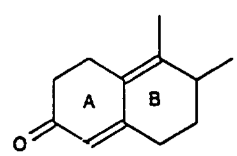

- or

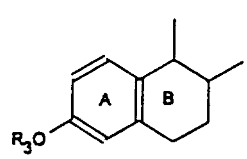

where $R_3$ represents a hydrogen atom or a protective group of the hydroxyl function, is subjected

**either**, in the case where W represents a C≡CH radical, to the action of a halogenated derivative of formula (VIII):

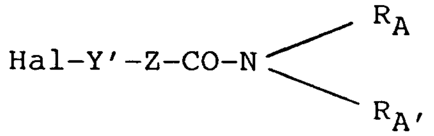

(VIII)

in which Hal is a halogen atom, Z, $R_A$ and $R_{A'}$ have the same meaning as indicated in claim 1 and Y' represents the above aliphatic chain Y comprising at least 2 carbon atoms, in the presence of a strong base and if appropriate, is subjected to the action of a deprotection agent in position 3 and/or position 17, in order to obtain the product of formula ($IX_A$):

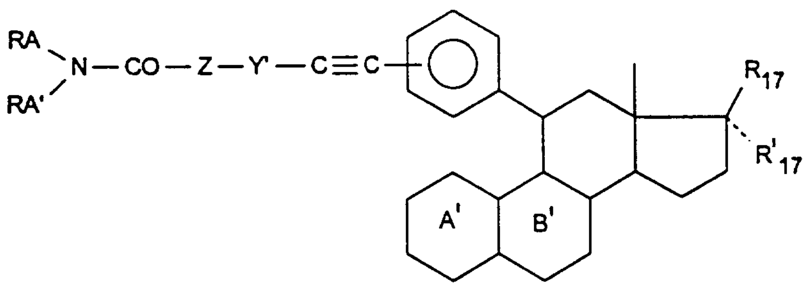

($IX_A$)

which product, if desired, is subjected to an agent giving partial or complete reduction of the triple bond in order to obtain the product of formula ($IX_B$):

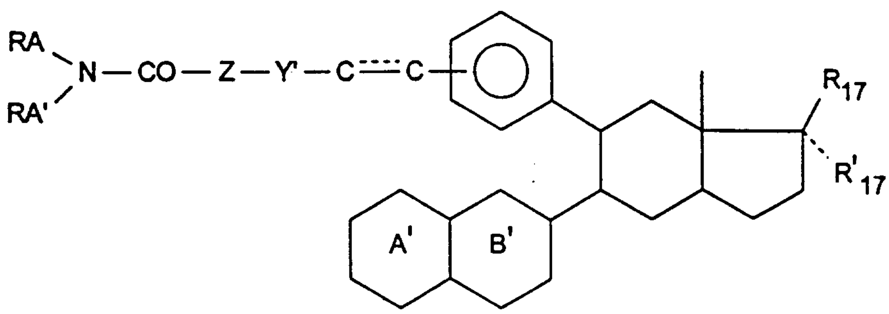

($IX_B$)

**or**, in the case where W represents an -OH radical, a mercapto radical or an activated mercapto radical, to the action of a halogenated derivative of formula (X):

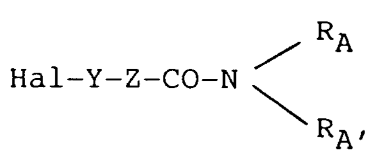

(X)

in which Hal, Y, Z, $R_A$ and $R_{A'}$ have the same meaning as indicated in claim 1, in the presence of an alkaline agent, then if appropriate, to the action of a deprotection agent in order to obtain a product of formula ($IX_C$):

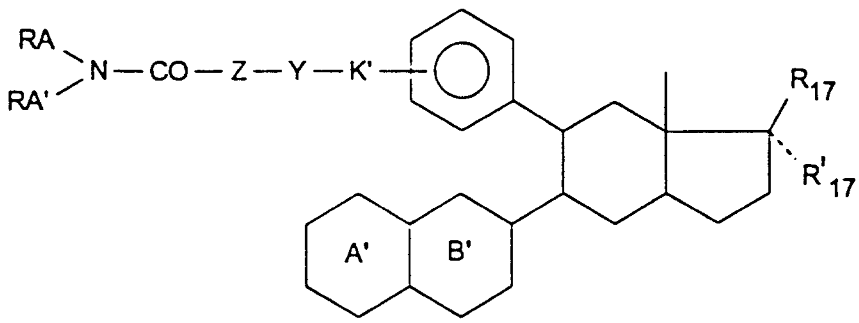

(IX$_C$)

in which K' represents either an oxygen atom, or a sulphur atom, which products of formula (IX$_A$), (IX$_B$) or (IX$_C$) are, if appropriate, subjected to an aromatization agent of ring A, then to a controlled saponification in order to obtain the products of formula (I') which, if appropriate, are treated as indicated in claim 1 for the products of formula (I).

**4.** Process according to any one of claim 1 to 3, characterized in that a product of formula (II) or (VII) in which n = 1 is used at the start.

**5.** Process according to claim 4, characterized in that a product of formula (II) in which X is an aryleneoxy radical and Y represents a saturated linear chain containing 5 to 10 carbon atoms, optionally interupted by an oxygen atom or a product of formula (VII) in which W represents an OH radical and a product of formula (X) in which Y has the meaning indicated above are used at the start.

**6.** Process according to claim 4, characterized in that a product of formula (II) in which X is an arylenethio radical and Y represents a saturated linear chain containing 5 to 10 carbon atoms or a product of formula (VII) in which W represents an optionally activiated mercapto radical and a product of formula (X) in which Y has the meaning indicated above are used at the start.

**7.** Process according to claim 4, characterized in that a product of formula (II) in which X is an arylene radical, Y represents an unsaturated linear chain containing 5 to 10 carbon atoms and a product of formula (VII) in which $R_A$ and $R_{A'}$ form with the atom to which they are linked a piperidine radical N-substituted by an aralkyl radical or a product of formula (VII) in which W represents a -C≡CH radical and a product of formula (VII) in which $R_A$ and $R_{A'}$ have the meaning indicated above are used at the start.

**8.** Process according to claim 1, characterized in that the starting products are chosen in such a way that any one of the products of formula (I) the names of which follow are prepared:

- N-butyl-5-(4-(3,17beta-dihydroxy-estra-1,3,5(10)-trien-11beta-yl) phenoxy) pentyloxy)-N-methyl-ethanethioamide,
- N-butyl-8-(4-(3,17beta-dihydroxy-estra-1,3,5(10)-trien-11beta-yl) phenoxy)-N-methyl-octanethioamide.

**9.** Process for the preparation of pharmaceutical compositions, characterized in that at least one of the products of formula (I) as defined in claim 1 is used as active ingredient in a form intended for this use.

**10.** Process for the preparation of pharmaceutical compositions, characterized in that at least one of the products of formula (I) as obtained in any one of claims 4 to 8 is used as active ingredient in a form intended for this use.

**11.** As new intermediate products, the products of formula (II) and (III) as defined in claim 1 or 2 in which:

n = 2.

**12.** As new intermediate products, the products of formula (VII) as defined in claim 1 or 3 in which W represents an optionally activated mercapto radical.

## Patentansprüche

### Patentansprüche für folgende Vertragsstaaten : AT, BE, CH, DE, DK, FR, GB, IT, LI, LU, NL, SE

**1.** Verbindungen der Formel (I)

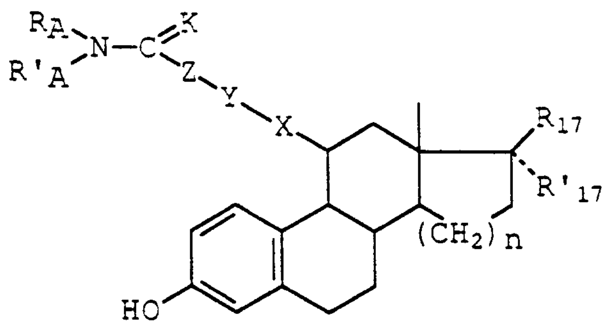

(I)

in der
<u>entweder</u>

n = 1 ist, K ein Sauerstoffatom darstellt, $R_{17}$ einen Rest Hydroxyl oder einen Rest der Formel

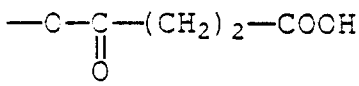

bedeutet, gegebenenfalls in Salz überführt,
$R'_{17}$ ein Wasserstoffatom oder einen Rest Ethinyl darstellt,
$R_A$ ein Rest Methyl ist,
$R'_A$ einen Rest Isopropyl oder lineares Butyl darstellt,
X einen Rest Methylen, Phenylen oder Phenyloxy bedeutet, verbunden mit dem Steroid durch ein Kohlenstoffatom,
Y einen der Reste darstellt, gewählt aus der Gruppe, die gebildet wird durch $-(CH_2)_7-$; $(CH_2)_8-$; $-(CH_2)_5-C{\equiv}C-$; $-(CH_2)_q-O-CH_2-$ mit q = 5 bis 7 und $-(CH_2)_5-S-CH_2-$, worin das Schwefelatom gegebenenfalls in Form von Sulfoxid oder Sulfon oxidiert ist,
von denen die Namen folgen:

- Monobutandioat von 11beta-{4-[(7-((Butylmethylamino)-carbonyl)-heptyl)-oxy]-phenyl}-3-hydroxy-estra-1,3,5(10)-trien-17beta-yl,
- Butandioat von 11beta-{4-[(7-((Butylmethylamino)-carbonyl)-heptyl)-oxy]-phenyl}-hydroxy-estra-1,3,5(10)-trien-17beta-yl und Natrium,
- N-Butyl-2-{6-[4-(3,17beta-dihydroxy-estra-1,3,5(10)-trien-11beta-yl)-phenoxy]-hexyloxy}-N-methyl-acetamid,
- N-Butyl-8-[4-(3,17beta-dihydroxy-estra-1,3,5(10)-trien-11beta-yl)-phenoxy]-N-methyl-2-octinamid,
- N-Butyl-2-{[5-(4-(3,17beta-dihydroxy-estra-1,3,5(10)-trien-11beta-yl)-phenyl)-pentyl]-thio}-N-methyl-acetamid,
- N-Butyl-4-(3,17beta-dihydroxy-estra-1,3,5(10)-trien-11beta-yl)-N-methyl-phenylen-nonamid,
- N-Butyl-2-{[5-(4-(3,17beta-dihydroxy-estra-1,3,5(10)-trien-11beta-yl)-phenyl)-pentyl]-oxy}-N-methyl-acetamid,
- 2-{[8-(3,17beta-dihydroxy-19-nor-17alpha-pregna-1,3,5(10)-trien-20-in-11beta-yl)-octyl]-oxy}-N-methyl-N-(1-methylethyl)-acetamid;

und Z eine einfache Bindung darstellt,

<u>oder</u>

n = 1 oder 2 ist,
K ein Sauerstoffatom oder ein Schwefelatom darstellt, $R_{17}$ und $R'_{17}$ bedeuten:

- entweder bilden $R_{17}$ und $R'_{17}$ zusammen eine Ketonfunktion
- oder $R_{17}$ ist ein Rest Hydroxyl oder ein Rest Acyloxy und $R'_{17}$ bedeutet ein Wasserstoffatom, einen Rest Alkyl, Alkenyl oder Alkinyl mit höchstens 8 Kohlenstoffatomen, gegebenenfalls substituiert,

X, Y und Z bedeuten:

- X stellt einen Rest Methylen, eine Gruppe Arylen, einen Rest $CH_2$-O, Arylenoxy oder Arylenthio dar, verbunden mit dem Steroid durch ein Kohlenstoffatom,
- Y stellt eine einfache Bindung oder eine lineare oder verzweigte, gesättigte oder ungesättigte aliphatische Kette mit 1 bis 18 Kohlenstoffatomen dar, gegebenenfalls unterbrochen durch einen oder mehrere Reste, ausgewählt unter den Resten Arylen, Sauerstoff oder Schwefel, gegebenenfalls oxidiert in Form von Sulfoxid oder Sulfon und gegebenenfalls abgeschlossen durch einen Rest Arylen,
- Z stellt eine einfache Bindung dar mit der Maßgabe, daß in dem Fall, wo Y und Z eine einfache Bindung bedeuten, X nicht ein Rest Methylen oder $CH_2$-O sein kann,

$R_A$ oder $R'_{A'}$ gleich oder verschieden, bedeuteten ein Wasserstoffatom, einen linearen oder verzweigten Rest Alkyl mit 1 bis 8 Kohlenstoffatomen, gegebenenfalls substituiert durch einen Rest Aryl, Alkyl oder Dialkylamino, Hydroxy, Halogen oder verestertes Carboxyl, oder $R_A$ und $R'_A$ bilden zusammen mit dem Stickstoffatom, an das sie gebunden sind, einen gesättigten oder ungesättigten Heterocyclus mit 5 oder 6 Ringgliedern, der gegebenenfalls ein oder mehrere Heteroatome umfassen kann, gewählt aus der durch die Atome von Sauerstoff, Stickstoff und Schwefel gebildeten Gruppe und gegebenenfalls substituiert durch einen Rest Alkyl mit 1 bis 4 Kohlenstoffatomen, einen Rest Aryl oder Aralkyl mit 6 bis 10 Kohlenstoffatomen, mit der Maßgabe, daß mindestens einer der Reste $R_A$ oder $R'_A$ kein Wasserstoffatom ist und daß mindestens irgendeine der folgenden Bedingungen erfüllt ist:

- entweder ist n = 2,
- oder K ist ein Schwefelatom,
- oder X ist ein Rest Arylenthio, verbunden mit dem Steroid durch ein Kohlenstoffatom,
- oder $R_A$ und $R'_A$ bilden mit dem Kohlenstoffatom, an das sie gebunden sind, einen Heterocyclus mit 5 oder 6 Ringgliedern, substituiert durch einen Rest Aryl oder Aralkyl.

**2.** Produkte der Formel ($I_A$), die der wie in Anspruch 1 definierten Formel (I) entsprechen, worin

n = 1 oder 2 ist,
K ein Sauerstoffatom oder ein Schwefelatom darstellt, $R_{17}$ und $R'_{17}$ bedeuten:

- entweder bilden $R_{17}$ und $R'_{17}$ zusammen eine Ketonfunktion
- oder $R_{17}$ ist ein Rest Hydroxyl oder ein Rest Acyloxy und $R'_{17}$ bedeutet ein Wasserstoffatom, einen Rest Alkyl, Alkenyl oder Alkinyl mit höchstens 8 Kohlenstoffatomen, gegebenenfalls substituiert,

X, Y und Z bedeuten:

- X stellt einen Rest Methylen, eine Gruppe Arylen, einen Rest $CH_2$-O, Arylenoxy oder Arylenthio dar, verbunden mit dem Steroid durch ein Kohlenstoffatom,
- Y stellt eine einfache Bindung oder eine lineare oder verzweigte, gesättigte oder ungesättigte aliphatische Kette mit 1 bis 18 Kohlenstoffatomen dar, gegebenenfalls unterbrochen durch einen oder mehrere Reste, ausgewählt unter den Resten Arylen, Sauerstoff oder Schwefel, gegebenenfalls oxidiert in Form von Sulfoxid oder Sulfon und gegebenenfalls abgeschlossen durch einen Rest Arylen,
- Z stellt eine einfache Bindung dar mit der Maßgabe, daß in dem Fall, wo Y und Z eine einfache Bindung bedeuten, X nicht ein Rest Methylen oder $CH_2$-O sein kann,

$R_A$ oder $R'_{A'}$ gleich oder verschieden, bedeuteten ein Wasserstoffatom, einen linearen oder verzweigten Rest Alkyl mit 1 bis 8 Kohlenstoffatomen, gegebenenfalls substituiert durch einen Rest Aryl, Alkyl oder Dialkylamino, Hydroxy, Halogen oder verestertes Carboxyl, oder $R_A$ und $R'_A$ bilden zusammen mit dem Stickstoffatom, an das sie gebunden sind, einen gesättigten oder ungesättigten Heterocyclus mit 5 oder 6 Ringgliedern, der

gegebenenfalls ein oder mehrere Heteroatome umfassen kann, ausgewählt aus der durch die Atome von Sauerstoff, Stickstoff und Schwefel gebildeten Gruppe und gegebenenfalls substituiert durch einen Rest Alkyl mit 1 bis 4 Kohlenstoffatomen, einen Rest Aryl oder Aralkyl mit 6 bis 10 Kohlenstoffatomen, mit der Maßgabe, daß mindestens einer der Reste $R_A$ oder $R'_A$ kein Wasserstoffatom ist und daß mindestens irgendeine der folgenden Bedingungen erfüllt ist:

- entweder ist n = 2,
- oder K ist ein Schwefelatom,
- oder X ist ein Rest Arylenthio, verbunden mit dem Steroid durch ein Kohlenstoffatom,
- oder $R_A$ und $R'_A$ bilden mit dem Kohlenstoffatom, an das sie gebunden sind, einen Heterocyclus mit 5 oder 6 Ringgliedern, substituiert durch einen Rest Aryl oder Aralkyl.

**3.** Produkte der Formel ($I_A$) wie in Anspruch 2 definiert, worin n = 1 ist.

**4.** Produkte nach Anspruch 3, worin K ein Schwefelatom darstellt, X ein Rest Arylenoxy ist und Y eine lineare gesättigte Kette mit 5 bis 10 Kohlenstoffatomen bedeutet, gegebenenfalls unterbrochen durch ein Sauerstoffatom.

**5.** Produkte nach Anspruch 3, worin X ein Rest Arylenthio ist und Y eine lineare gesättigte Kette mit 5 bis 10 Kohlenstoffatomen bedeutet.

**6.** Produkte nach Anspruch 3, worin X ein Rest Arylen ist, Y eine lineare ungesättigte Kette mit 5 bis 10 Kohlenstoffatomen bedeutet und $R_A$ und $R'_A$ mit dem Atom, an das sie gebunden sind, ein Piperidin bilden, N-substituiert durch einen Rest Aralkyl.

**7.** Produkte der Formel (I) nach Anspruch 1 mit den folgenden Namen:

- N-Butyl-5-{[4-(3,17beta-dihydroxy-estra-1,3,5(10)-trien-11beta-yl)-phenoxy]-pentyloxy}-N-methyl-ethanthioamid,
- N-Butyl-8-{4-[3,17beta-dihydroxy-estra-1,3,5(10)-trien-11beta-yl]-phenoxy}-N-methyl-octanthioamid.

**8.** Verfahren zur Herstellung der Produkte der Formel (I) wie in Anspruch 1 definiert, dadurch gekennzeichnet, daß man eine Verbindung der Formel (II)

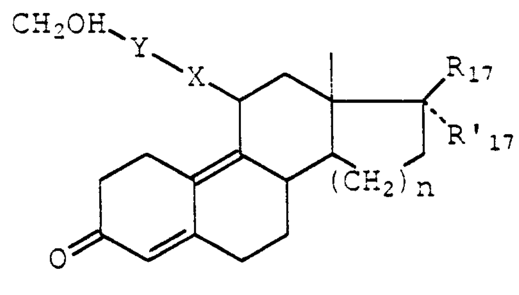

(II)

in der X, Y, n, $R_{17}$ und $R'_{17}$ die gleiche Bedeutung wie in Anspruch 1 besitzen, mit der Maßgabe, daß in dem Fall, wo $R_{17}$ einen Rest Hydroxyl darstellt, dieser Rest geschützt ist, der Einwirkung eines Oxidationsmittels unterzieht, um das Produkt der Formel (III)

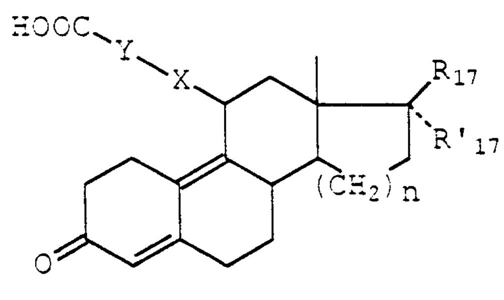

(III)

zu erhalten, das man der Einwirkung eines Mittels unterzieht, das ermöglicht, die Carboxyfunction zu aktivieren, und man anschließend eine Verbindung der Formel (IV)

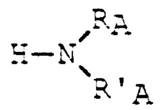

(IV)

einwirken läßt, in der $R_A$ und $R'_A$ die gleiche Bedeutung wie in Formel (I) besitzen, um das Produkt der Formel (V)

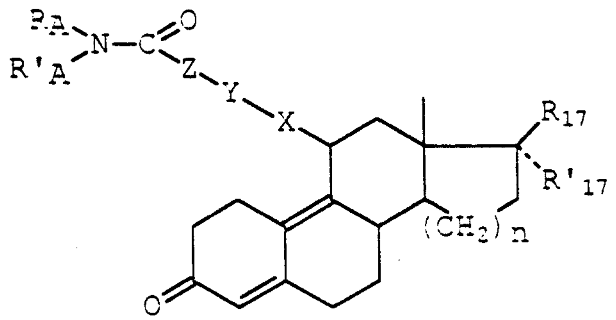

(V)

zu erhalten, in der Z eine einfache Bindung ist, wobei man das Produkt (V), wenn erforderlich, einer Reaktion zur Abspaltung der Schutzgruppe von der Hydroxyfunktion unterzient und, wenn gewünscht

- einem Reduktionsmittel unterzieht, wenn $R_{17}$ und $R'_{17}$ zusammen eine Ketonfunktion bilden, und man anschließend gegebenenfalls das auf diese Weise erhaltene, in 17 hydroxylierte Derivat der Einwirkung eines Acylierungsmittels unterwirft,
- oder einem Mittel zur Verseifung unterzieht, wenn $R_{17}$ eine Acyloxyfunktion darstellt,

oder die Verbindung der Formel (V) einem Mittel zur Aromatisierung des Ringes A und anschließend einem Mittel zur schonenden Verseifung unterzieht, um die Produkte der Formel (I) zu erhalten, die man, wenn gewünscht, einer oder mehreren der folgenden Reaktionen unterwirft:

entweder, wenn $R_{17}$ und $R'_{17}$ zusammen eine Ketonfunktion bilden, mit einem Reduktionsmittel oder einem Metallkomplex der Formel (VI)

$$M\text{-}R'_{17} \qquad (VI)$$

in der M ein Metallatom darstellt und $R'_{17}$ die gleiche Bedeutung wie in Anspruch 1 mit Ausnahme von Wasserstoff besitzt,
oder, wenn $R_{17}$ ein Rest Hydroxyl ist, mit einem selektiven Acylierungsmittel für die Position 17,
oder, wenn $R_A$ oder $R'_A$ ein Wasserstoffatom sind, mit einem geeigneten Mittel zur Alkylierung,
oder, wenn Y eine ungesättigte aliphatische Kette darstellt, mit einem Mittel zur Hydrierung,
um die Produkte der Formel (I) zu erhalten, in der K ein Sauerstoffatom ist, die man, wenn gewünscht, einem Mittel zur Sulfurierung unterzieht, um die Produkte der Formel (I) zu erhalten, in der K ein Schwefelatom ist.

**9.** Verfahren zur Herstellung der Produkte der Formel (I'), die den wie in Anspruch 1 definierten Produkten der Formel (I) entsprechen, in der X einen Rest Arylen darstellt und Y eine aliphatische Kette bedeutet, die gegebenenfalls mit der Arylengruppe durch eine Doppelbindung oder eine Dreifachbindung verbunden ist und die mindestens 3 Kohlenstoffatome umfaßt, oder die mit der Arylengruppe durch ein Sauerstoffatom oder durch ein Schwefelatom verbunden ist, dadurch gekennzeichnet, daß man eine Verbindung der Formel (VII)

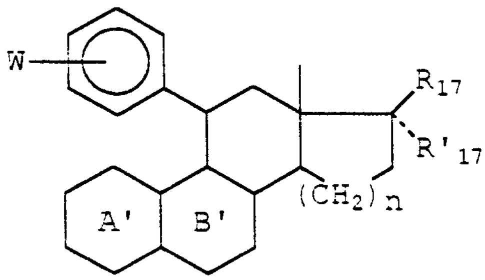

(VII)

in der W entweder einen Rest -OH oder einen gegebenenfalls aktivierten Rest Mercapto oder einen Rest -C≡CH bedeutet sowie n, $R_{17}$ und R' die gleiche Bedeutung wie in Anspruch 1 angegeben besitzen, mit der Maßgabe, daß in dem Fall, wo $R_{17}$ einen Rest -OH darstellt, dieser gegebenenfalls geschützt ist,
die Ringe A' und B'

entweder

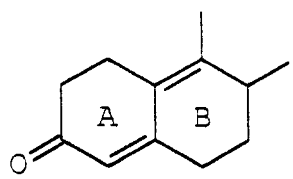

oder

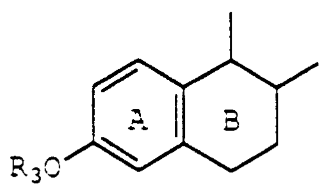

bedeuten, worin $R_3$ ein Wasserstoffatom oder eine Schutzgruppe für die Hydroxyfunktion darstellt,

oder in dem Fall, wo W einen Rest -C≡CH bedeutet, der Einwirkung eines Halogenderivates der Formel (VIII)

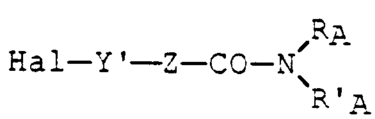

(VIII)

in der Hal ein Halogenatom ist, Z, $R_A$ und $R'_A$ die gleiche Bedeutung wie in Anspruch 1 angegeben besitzen und Y' die oben genannte aliphatische Kette Y darstellt, die mindestens 2 Kohlenstoffatome umfaßt, in Anwesenheit einer starken Base und gegebenenfalls der Einwirkung eines Mittels zum Abspalten der Schutzgruppe in 3 und/oder 17 unterzieht, um das Produkt der Formel ($IX_A$)

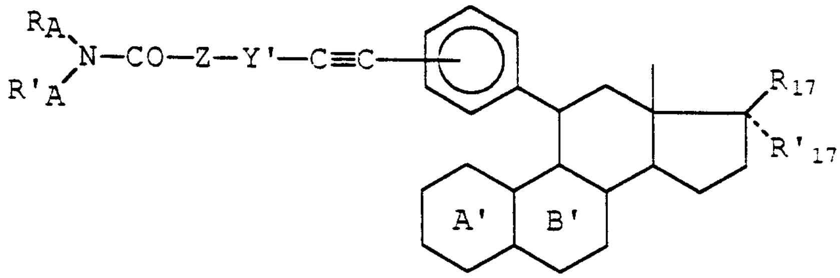

($IX_A$)

zu erhalten, das man, wenn gewünscht, einem Mittel zur teilweisen oder vollständigen Reduktion der Dreifachbindung unterwirft, um das Produkt der Formel ($IX_B$)

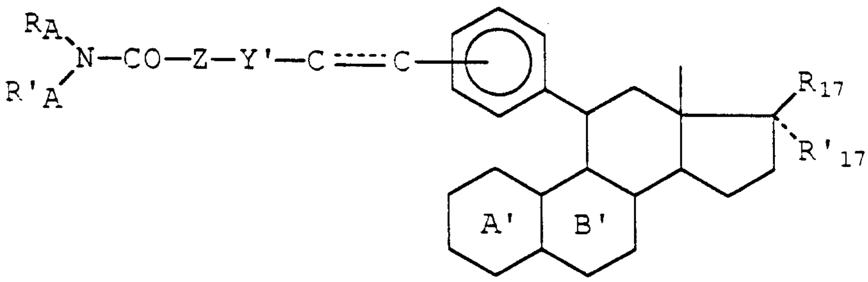

($IX_B$)

zu erhalten

oder in dem Fall, wo W einen Rest -OH, einen Rest Mercapto oder einen Rest aktiviertes Mercapto darstellt, der Einwirkung eines Halogenderivates der Formel (X)

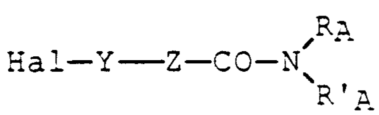

(X)

in der Hal, Y, Z, $R_A$ und $R'_A$ die gleiche Bedeutung wie in Anspruch 1 angegeben besitzen, in Anwesenheit eines alkalischen Mittels und anschließend gegebenenfalls der Einwirkung eines Mittels zum Abspalten der Schutzgruppe unterzieht, um ein Produkt der Formel ($IX_C$)

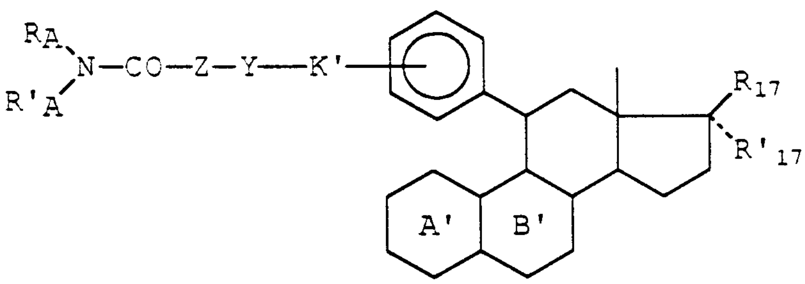

($IX_C$)

zu erhalten, in der K' entweder ein Sauerstoffatom oder ein Schwefelatom darstellt, wobei man die Produkte der Formeln ($IX_A$), ($IX_B$) oder ($IX_C$) gegebenenfalls einem Mittel zur Aromatisierung des Ringes A und anschließend einem Mittel zur schonenden Verseifung unterzieht, um die Produkte der Formel (I') zu erhalten, die man gegebenenfalls wie in Anspruch 8 bei den Produkten der Formel (I) angegeben behandelt.

**10.** Als Medikament die Produkte der Formel (I) wie in irgendeinem der Ansprüche 1 bis 7 definiert.

**11.** Pharmazeutische Zusammensetzungen, die als Wirkstoff mindestens eines der in Anspruch 10 definierten Medikamente umfassen.

**12.** Als neue Zwischenprodukte die Produkte der Formeln (II) und (III) wie in Anspruch 8 definiert, worin n = 2 ist.

**13.** Als neue Zwischenprodukte die Produkte der Formel (VII) wie in Anspruch 9 definiert, worin W einen gegebenenfalls aktivierten Rest Mercapto darstellt.

**Patentansprüche für folgenden Vertragsstaat : ES**

**1.** Verfahren zur Herstellung von Verbindungen der Formel (I)

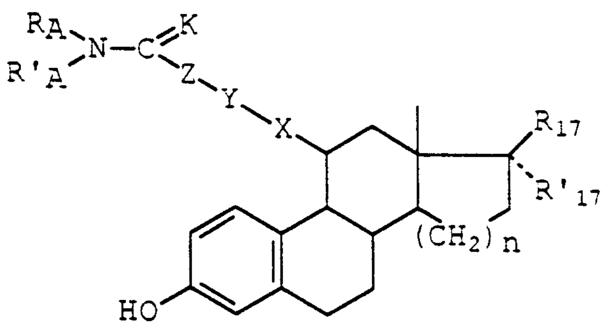

(I)

in der
<u>entweder</u>

n = 1 ist, K ein Sauerstoffatom darstellt, $R_{17}$ einen Rest Hydroxyl oder einen Rest der Formel

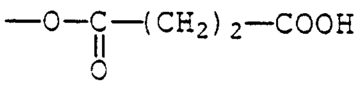

bedeutet, gegebenenfalls in Salz überführt,
$R'_{17}$ ein Wasserstoffatom oder einen Rest Ethinyl darstellt,
$R_A$ ein Rest Methyl ist,
$R'_A$ einen Rest Isopropyl oder lineares Butyl darstellt,
X einen Rest Methylen, Phenylen oder Phenyloxy bedeutet, verbunden mit dem Steroid durch ein Kohlenstoffatom,
Y einen der Reste darstellt, gewählt aus der Gruppe, die gebildet wird durch $-(CH_2)_7-$; $-(CH_2)_8-$; $(CH_2)_5-C{\equiv}C-$; $(CH_2)_q-O-CH_2$ mit q = 5 bis 7 und $-(CH_2)_5-S-CH_2-$, worin das Schwefelatom gegebenenfalls in Form von Sulfoxid oder Sulfon oxidiert ist,
von denen die Namen folgen:

- Monobutandioat von 11beta-{4-[(7-((Butylmethylamino)-carbonyl)-heptyl)-oxy]-phenyl}-3-hydroxy-estra-1,3,5(10)-trien-17beta-yl,
- Butandioat von 11beta-{4-[(7-((Butylmethylamino)-carbonyl)-heptyl)-oxy]-phenyl}-hydroxy-estra-1,3,5(10)-trien-17beta-yl und Natrium,
- N-Butyl-2-{6-[4-(3,17beta-dihydroxy-estra-1,3,5(10)-trien-11beta-yl)-phenoxy]-hexyloxy}-N-methyl-acetamid,
- N-Butyl-8-[4-(3,17beta-dihydroxy-estra-1,3,5(10)-trien-11beta-yl)-phenoxy]-N-methyl-2-octinamid,
- N-Butyl-2-{[5-(4-(3,17beta-dihydroxy-estra-1,3,5(10)-trien-11beta-yl)-phenyl)-pentyl]-thio}-N-methyl-acetamid,
- N-Butyl-4-(3,17beta-dihydroxy-estra-1,3,5(10)-trien-11beta-yl)-N-methyl- phenylen-nonamid,
- N-Butyl-2-{[5-(4-(3,17beta-dihydroxy-estra-1,3,5(10)-trien-11beta-yl)-phenyl)-pentyl]-oxy}-N-methyl-acetamid,
- 2-{[8-(3,17beta-dihydroxy-19-nor-17alpha-pregna-1,3,5(10)-trien-20-in-11beta-yl)-octyl]-oxy}-N-methyl-N-(1-methylethyl)-acetamid;

und Z eine einfache Bindung darstellt,

oder

n = 1 oder 2 ist,
K ein Sauerstoffatom oder ein Schwefelatom darstellt, $R_{17}$ und $R'_{17}$ bedeuten:

- entweder bilden $R_{17}$ und $R'_{17}$ zusammen eine Ketonfunktion
- oder $R_{17}$ ist ein Rest Hydroxyl oder ein Rest Acyloxy und $R'_{17}$ bedeutet ein Wasserstoffatom, einen Rest Alkyl, Alkenyl oder Alkinyl mit höchstens 8 Kohlenstoffatomen, gegebenenfalls substituiert,

X, Y und Z bedeuten:

- X stellt einen Rest Methylen, eine Gruppe Arylen, einen Rest $CH_2$-O, Arylenoxy oder Arylenthio dar, verbunden mit dem Steroid durch ein Kohlenstoffatom,
- Y stellt eine einfache Bindung oder eine lineare oder verzweigte, gesättigte oder ungesättigte aliphatische Kette mit 1 bis 18 Kohlenstoffatomen dar, gegebenenfalls unterbrochen durch einen oder mehrere Reste, ausgewählt unter den Resten Arylen, Sauerstoff oder Schwefel, gegebenenfalls oxidiert in Form von Sulfoxid oder Sulfon und gegebenenfalls abgeschlossen durch einen Rest Arylen,
- Z stellt eine einfache Bindung dar mit der Maßgabe, daß in dem Fall, wo Y und Z eine einfache Bindung bedeuten, X nicht ein Rest Methylen oder $CH_2$-O sein kann,

$R_A$ oder $R'_{A'}$ gleich oder verschieden, bedeuteten ein Wasserstoffatom, einen linearen oder verzweigten Rest Alkyl mit 1 bis 8 Kohlenstoffatomen, gegebenenfalls substituiert durch einen Rest Aryl, Alkyl oder Dialkylamino, Hydroxy, Halogen oder verestertes Carboxyl, oder $R_A$ und $R'_A$ bilden zusammen mit dem Stickstoffatom, an das sie gebunden sind, einen gesättigten oder ungesättigten Heterocyclus mit 5 oder 6 Ringgliedern, der gegebenenfalls ein oder mehrere Heteroatome umfassen kann, gewählt aus der durch die Atome von Sauerstoff, Stickstoff und Schwefel gebildeten Gruppe und gegebenenfalls substituiert durch einen Rest Alkyl mit 1 bis 4 Kohlenstoffatomen, einen Rest Aryl oder Aralkyl mit 6 bis 10 Kohlenstoffatomen, mit der Maßgabe, daß mindestens einer der Reste $R_A$ oder $R'_A$ kein Wasserstoffatom ist und daß mindestens irgendeine der folgenden Bedingungen erfüllt ist:

- entweder ist n = 2,
- oder K ist ein Schwefelatom,
- oder X ist ein Rest Arylenthio, verbunden mit dem Steroid durch ein Kohlenstoffatom,
- oder $R_A$ und $R'_A$ bilden mit dem Kohlenstoffatom, an das sie gebunden sind, einen Heterocyclus mit 5 oder 6 Ringgliedern, substituiert durch einen Rest Aryl oder Aralkyl,

dadurch gekennzeichnet, daß man

a) zur Herstellung der wie oben definierten Produkte der Formel (I) eine Verbindung der Formel (II)

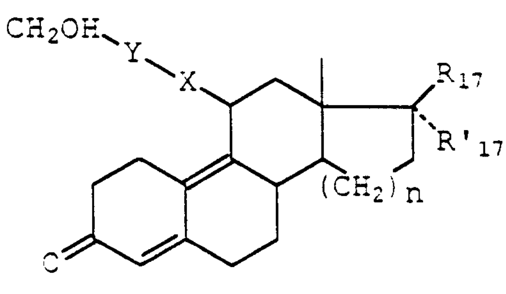

(II)

in der X, Y, n, $R_{17}$ und $R'_{17}$ die gleiche Bedeutung wie oben besitzen, mit der Maßgabe, daß in dem Fall, wo $R_{17}$ einen Rest Hydroxyl darstellt, dieser Rest geschützt ist,
der Einwirkung eines Oxidationsmittels unterzieht, um das Produkt der Formel (III)

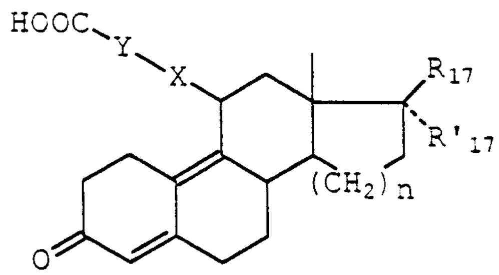

(III)

zu erhalten, das man der Einwirkung eines Mittels unterzieht, das ermöglicht, die Carboxylfunktion zu aktivieren, und man anschließend eine Verbindung der Formel (IV)

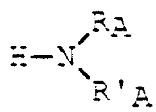

(IV)

einwirken läßt, in der $R_A$ und $R'_A$ die gleiche Bedeutung wie oben besitzen, um das Produkt der Formel (V)

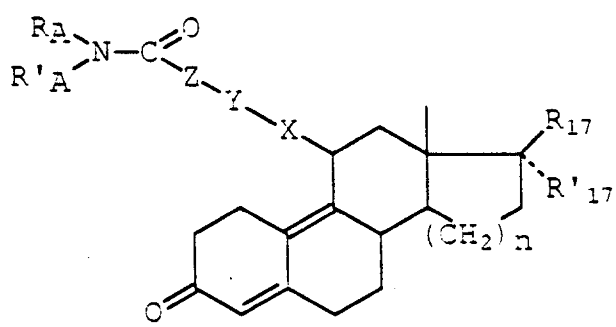

(V)

zu erhalten, in der Z eine einfache Bindung ist, wobei man das Produkt (V), wenn erforderlich, einer Reaktion zur Abspaltung der Schutzgruppe von der Hydroxyfunktion unterzieht und, wenn gewünscht

- einem Reduktionsmittel unterzieht, wenn $R_{17}$ und $R'_{17}$ zusammen eine Ketonfunktion bilden, und man anschließend gegebenenfalls das auf diese Weise erhaltene, in 17 hydroxylierte Derivat der Einwirkung eines Acylierungsmittels unterwirft,
- oder einem Mittel zur Verseifung unterzieht, wenn $R_{17}$ eine Acyloxyfunktion darstellt,

oder die Verbindung der Formel (V) einem Mittel zur Aromatisierung des Ringes A und anschließend einem Mittel zur schonenden Verseifung unterzieht, um die Produkte der Formel (I) zu erhalten, die man, wenn gewünscht, einer oder mehreren der folgenden Reaktionen unterwirft:

entweder, wenn $R_{17}$ und $R'_{17}$ zusammen eine Ketonfunktion bilden, mit einem Reduktionsmittel oder einem Metallkomplex der Formel (VI)

$$M\text{-}R'_{17} \qquad \text{(VI)}$$

in der M ein Metallatom darstellt und $R'_{17}$ die gleiche Bedeutung wie oben mit Ausnahme von Wasserstoff besitzt, oder, wenn $R_{17}$ ein Rest Hydroxyl ist, mit einem selektiven Acylierungsmittel für die Position 17,
oder, wenn $R_A$ oder $R'_A$ ein Wasserstoffatom sind, mit einem geeigneten Mittel zur Alkylierung,
oder, wenn Y eine ungesättigte aliphatische Kette darstellt, mit einem Mittel zur Hydrierung umsetzt,
um die Produkte der Formel (I) zu erhalten, in der K ein Sauerstoffatom ist, die man, wenn gewünscht, einem Mittel zur Sulfurierung unterzieht, um die Produkte der Formel (I) zu erhalten, in der K ein Schwefelatom ist;

b) zur Herstellung der Produkte der Formel (I), in der X einen Rest Arylen darstellt und Y eine aliphatische Kette bedeutet, die gegebenenfalls mit der Arylengruppe durch eine Doppelbindung oder eine Dreifachbindung verbunden ist und die mindestens 3 Kohlenstoffatome umfaßt, oder die mit der Arylengruppe durch ein Sauerstoffatom oder durch ein Schwefelatom verbunden ist, eine Verbindung der Formel (VII)

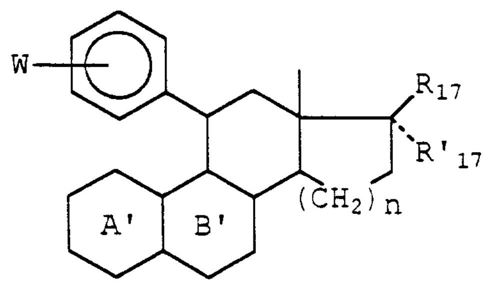

(VII)

in der W entweder einen Rest -OH oder einen gegebenenfalls aktivierten Rest Mercapto oder einen Rest -C≡CH bedeutet sowie n, $R_{17}$ und R' die gleiche Bedeutung wie oben angegeben besitzen, mit der Maßgabe, daß in dem Fall, wo $R_{17}$ einen Rest -OH darstellt, dieser gegebenenfalls geschützt ist,
die Ringe A' und B'

entweder

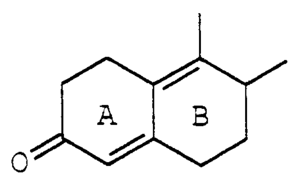

oder

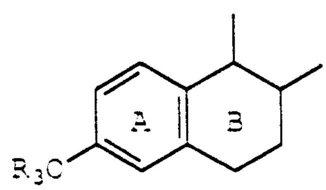

bedeuten, worin $R_3$ ein Wasserstoffatom oder eine Schutzgruppe für die Hydroxyfunktion darstellt,

<u>oder</u> in dem Fall, wo W einen Rest -C≡CH bedeutet, der Einwirkung eines Halogenderivates der Formel (VIII)

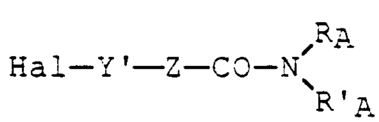

(VIII)

in der Hai ein Halogenatom ist, Z, $R_A$ und $R'_A$ die gleiche Bedeutung wie oben angegeben besitzen und Y' die oben genannte aliphatische Kette Y darstellt, die mindestens 2 Kohlenstoffatome umfaßt, in Anwesenheit einer starken Base und gegebenenfalls der Einwirkung eines Mittels zum Abspalten der Schutzgruppe in 3 und/oder 17 unterzieht, um das Produkt der Formel ($IX_A$)

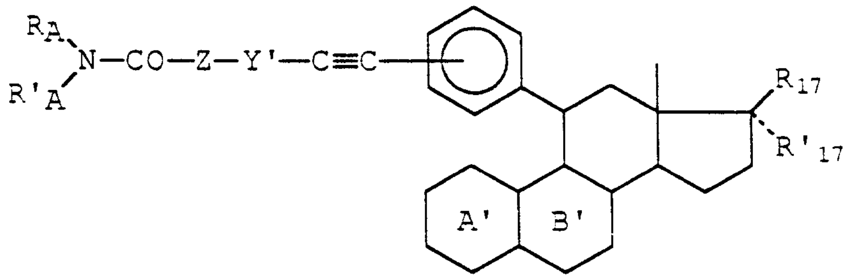

($IX_A$)

zu erhalten, das man, wenn gewünscht, einem Mittel zur teilweisen oder vollständigen Reduktion der Dreifachbindung unterwirft, um das Produkt der Formel ($IX_B$)

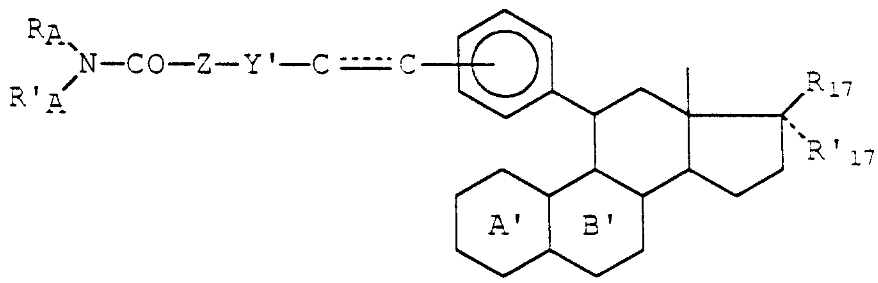

($IX_B$)

zu erhalten

<u>oder</u>

in dem Fall, wo W einen Rest -OH, einen Rest Mercapto oder einen Rest aktiviertes Mercapto darstellt, der Einwirkung eines Halogenderivates der Formel (X)

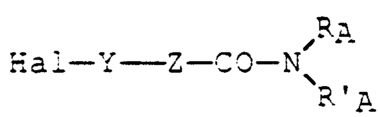

(X)

in der Hal, Y, Z, $R_A$ und $R'_A$ die gleiche Bedeutung wie oben angegeben besitzen, in Anwesenheit eines alkalischen Mittels und anschließend gegebenenfalls der Einwirkung eines Mittels zum Abspalten der Schutzgruppe unterzieht, um ein Produkt der Formel ($IX_C$)

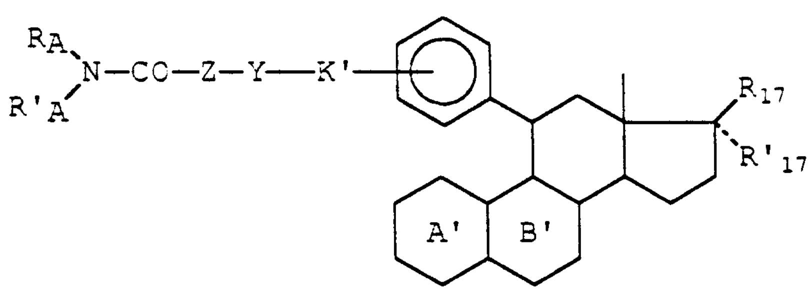

($IX_C$)

zu erhalten, in der K' entweder ein Sauerstoffatom oder ein Schwefelatom darstellt, wobei man die Produkte der Formeln ($IX_A$), ($IX_B$) oder ($IX_C$) gegebenenfalls einem Mittel zur Aromatisierung des Ringes A und anschließend einem Mittel zur schonenden Verseifung unterzieht, um die Produkte der Formel (I') zu erhalten, die man gegebenenfalls wie oben bei den Produkten der Formel (I) angegeben behandelt.

**2.** Verfahren nach Anspruch 1, dadurch gekennzeichnet, daß man zur Herstellung der wie in Anspruch 1 definierten Produkte der Formel (I) eine Verbindung der Formel (II)

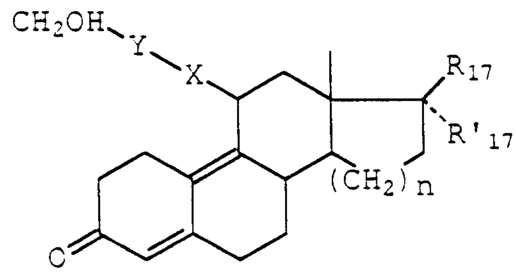

(II)

in der X, Y, n, $R_{17}$ und $R'_{17}$ die gleiche Bedeutung wie in Anspruch 1 besitzen, mit der Maßgabe, daß in dem Fall, wo $R_{17}$ einen Rest Hydroxyl darstellt, dieser Rest geschützt ist, der Einwirkung eines Oxidationsmittels unterzieht, um das Produkt der Formel (III)

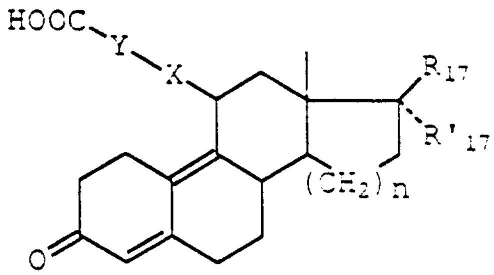

(III)

zu erhalten, das man der Einwirkung eines Mittels unterzieht, das ermöglicht, die Carboxylfunktion zu aktivieren, und man anschließend eine Verbindung der Formel (IV)

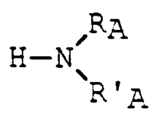

(IV)

einwirken läßt, in der $R_A$ und $R'_A$ die gleiche Bedeutung wie in Anspruch 1 besitzen, um das Produkt der Formel (V)

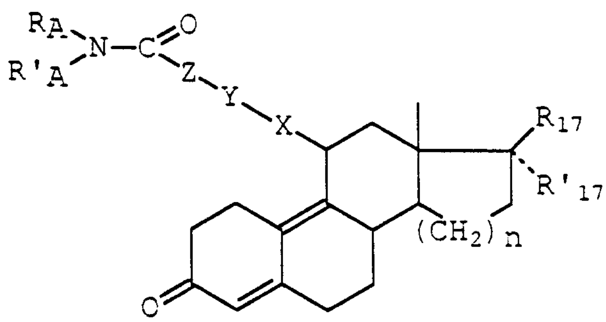

(V)

zu erhalten, in der Z eine einfache Bindung ist, wobei man das Produkt (V), wenn erforderlich, einer Reaktion zur Abspaltung der Schutzgruppe von der Hydroxyfunktion unterzieht und, wenn gewünscht

- einem Reduktionsmittel unterzieht, wenn $R_{17}$ und $R'_{17}$ zusammen eine Ketonfunktion bilden, und man anschließend gegebenenfalls das auf diese Weise erhaltene, in 17 hydroxylierte Derivat der Einwirkung eines Acylierungsmittels unterwirft,
- oder einem Mittel zur Verseifung unterzieht, wenn $R_{17}$ eine Acyloxyfunktion darstellt,

oder die Verbindung der Formel (V) einem Mittel zur Aromatisierung des Ringes A und anschließend einem Mittel

zur schonenden Verseifung unterzieht, um die Produkte der Formel (I) zu erhalten, die man, wenn gewünscht, einer oder mehreren der folgenden Reaktionen unterwirft:

entweder, wenn $R_{17}$ und $R'_{17}$ zusammen eine Ketonfunktion bilden, mit einem Reduktionsmittel oder einem Metallkomplex der Formel (VI)

$$M\text{-}R'_{17} \qquad \text{(VI)}$$

in der M ein Metallatom darstellt und $R'_{17}$ die gleiche Bedeutung wie in Anspruch 1 mit Ausnahme von Wasserstoff besitzt, oder, wenn $R_{17}$ ein Rest Hydroxyl ist, mit einem selektiven Acylierungsmittel für die Position 17,
oder, wenn $R_A$ oder $R'_A$ ein Wasserstoffatom sind, mit einem geeigneten Mittel zur Alkylierung umsetzt,
oder, wenn Y eine ungesättigte aliphatische Kette darstellt, mit einem Mittel zur Hydrierung,
um die Produkte der Formel (I) zu erhalten, in der K ein Sauerstoffatom ist, die man, wenn gewünscht, einem Mittel zur Sulfurierung unterzieht, um die Produkte der Formel (I) zu erhalten, in der K ein Schwefelatom ist.

**3.** Verfahren nach Anspruch 1, dadurch gekennzeichnet, daß man zur Herstellung der Produkte der Formel (I), in der X einen Rest Arylen darstellt und Y eine aliphatische Kette bedeutet, die gegebenenfalls mit der Arylengruppe durch eine Doppelbindung oder eine Dreifachbindung verbunden ist und die mindestens 3 Kohlenstoffatome umfaßt, oder die mit der Arylengruppe durch ein Sauerstoffatom oder durch ein Schwefelatom verbunden ist, eine Verbindung der Formel (VII)

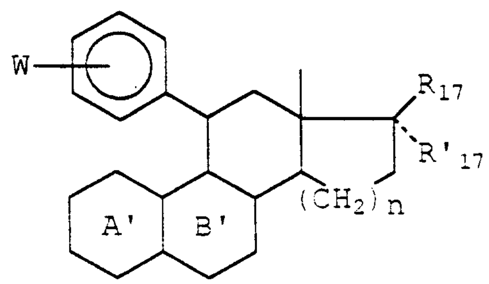

(VII)

in der W entweder einen Rest -OH oder einen gegebenenfalls aktivierten Rest Mercapto oder einen Rest -C≡CH bedeutet sowie n, $R_{17}$ und R' die gleiche Bedeutung wie in Anspruch 1 angegeben besitzen, mit der Maßgabe, daß in dem Fall, wo $R_{17}$ einen Rest -OH darstellt, dieser gegebenenfalls geschützt ist,
die Ringe A' und B'

entweder

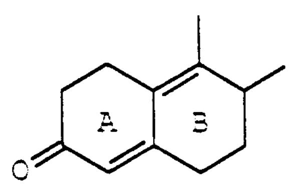

oder

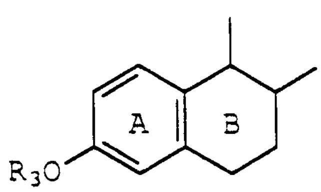

bedeuten, worin $R_3$ ein Wasserstoffatom oder eine Schutzgruppe für die Hydroxyfunktion darstellt,

<u>oder</u> in dem Fall, wo W einen Rest -C≡CH bedeutet, der Einwirkung eines Halogenderivates der Formel (VIII)

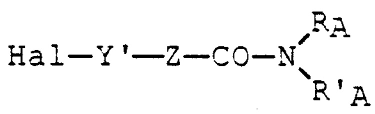

(VIII)

in der Hal ein Halogenatom ist, Z, $R_A$ und $R'_A$ die gleiche Bedeutung wie in Anspruch 1 angegeben besitzen und Y' die oben genannte aliphatische Kette Y darstellt, die mindestens 2 Kohlenstoffatome umfaßt, in Anwesenheit einer starken Base und gegebenenfalls der Einwirkung eines Mittels zum Abspalten der Schutzgruppe in 3 und/oder 17 unterzieht, um das Produkt der Formel ($IX_A$)

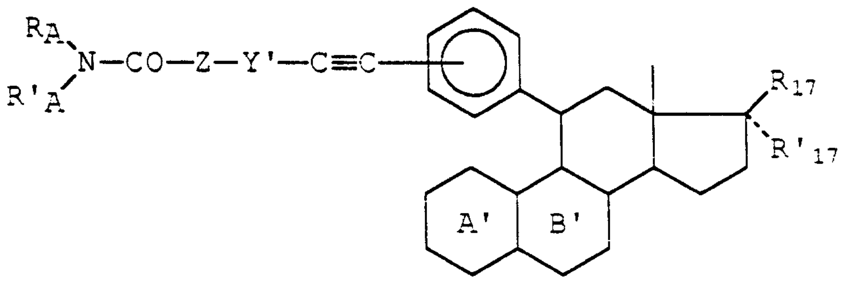

($IX_A$)

zu erhalten, das man, wenn gewünscht, einem Mittel zur teilweisen oder vollständigen Reduktion der Dreifachbindung unterwirft, um das Produkt der Formel ($IX_B$)

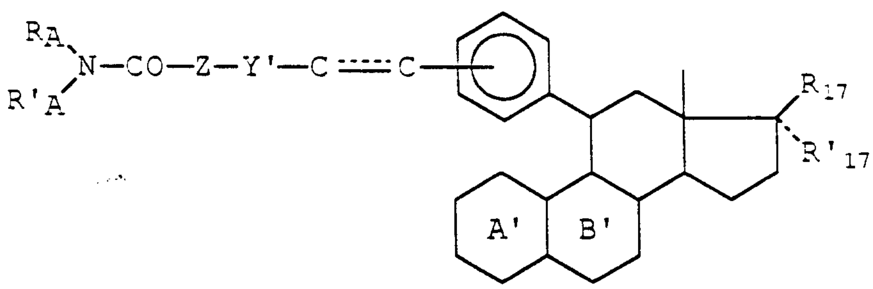

($IX_B$)

zu erhalten
<u>oder</u>
in dem Fall, wo W einen Rest -OH, einen Rest Mercapto oder einen Rest aktiviertes Mercapto darstellt, der Einwirkung eines Halogenderivates der Formel (X)

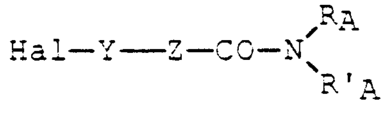

(X)

in der Hal, Y, Z, $R_A$ und $R'_A$ die gleiche Bedeutung wie in Anspruch 1 angegeben besitzen, in Anwesenheit eines alkalischen Mittels und anschließend gegebenenfalls der Einwirkung eines Mittels zum Abspalten der Schutzgruppe unterzieht, um ein Produkt der Formel ($IX_C$)

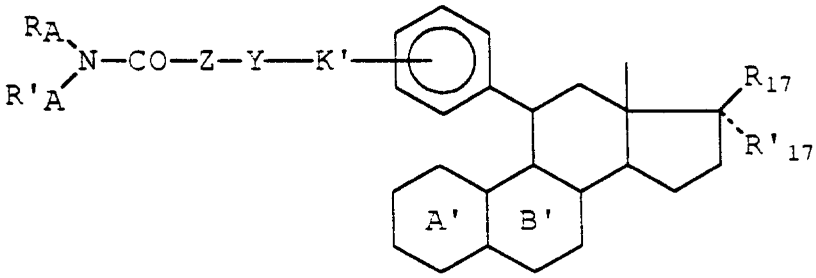

$(IX_C)$

zu erhalten, in der K' entweder ein Sauerstoffatom oder ein Schwefelatom darstellt, wobei man die Produkte der Formeln ($IX_A$), ($IX_B$) oder ($IX_C$) gegebenenfalls einem Mittel zur Aromatisierung des Ringes A und anschließend einem Mittel zur schonenden Verseifung unterzieht, um die Produkte der Formel (I') zu erhalten, die man gegebenenfalls wie in Anspruch 1 bei den Produkten der Formel (I) angegeben behandelt.

**4.** Verfahren nach irgendeinem der Ansprüche 1 bis 31 dadurch gekennzeichnet, daß man ein Ausgangsprodukt der Formeln (II) oder (VII) verwendet, worin n = 1 ist.

**5.** Verfahren nach Anspruch 4, dadurch gekennzeichnet, daß man ein Ausgangsprodukt der Formel (II) verwendet, worin X ein Rest Arylenoxy ist und Y eine lineare gesättigte Kette mit 5 bis 10 Kohlenstoffatomen bedeutet, gegebenenfalls unterbrochen durch ein Sauerstoffatom, oder ein Produkt der Formel (VII), in der W ein Rest -OH ist und ein Produkt der Formel (X) einsetzt, in der Y die oben angegebene Bedeutung besitzt.

**6.** Verfahren nach Anspruch 4, dadurch gekennzeichnet, daß man ein Ausgangsprodukt der Formel (II) verwendet, worin X ein Rest Arylenthio ist und Y eine lineare gesättigte Kette mit 5 bis 10 Kohlenstoffatomen bedeutet, oder ein Produkt der Formel (VII), in der W ein gegebenenfalls aktivierter Rest Mercapto ist und ein Produkt der Formel (X) einsetzt, in der Y die oben angegebene Bedeutung besitzt.

**7.** Verfahren nach Anspruch 4, dadurch gekennzeichnet, daß man ein Ausgangsprodukt der Formel (II), worin X ein Rest Arylen ist und Y eine lineare ungesättigte Kette mit 5 bis 10 Kohlenstoffatomen bedeutet, und ein Produkt der Formel (VII) verwendet, in der $R_A$ und $R'_A$ mit dem Atom, an das sie gebunden sind, ein Piperidin bilden, N-substituiert durch einen Rest Aralkyl, oder ein Produkt der Formel (VII), in der W ein Rest -C≡CH ist, und ein Produkt der Formel (VII) einsetzt, in der $R_A$ und $R'_A$ die oben angegebene Bedeutung besitzen.

**Patentansprüche für folgenden Vertragsstaat : GR**

**1.** Verfahren zur Herstellung von Verbindungen der Formel (I)

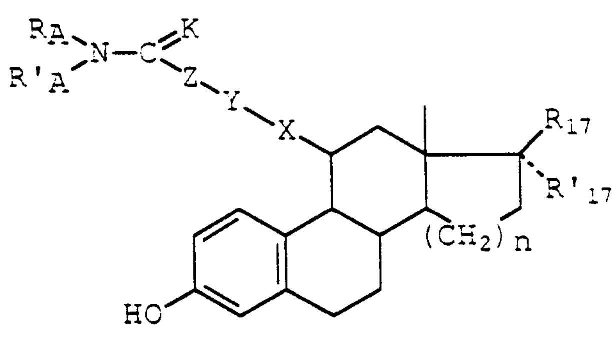

(I)

in der
<u>entweder</u>

n = 1 ist, K ein Sauerstoffatom darstellt,
$R_{17}$ einen Rest Hydroxyl oder einen Rest der Formel

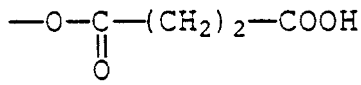

bedeutet, gegebenenfalls in Salz überführt,
$R'_{17}$ ein Wasserstoffatom oder einen Rest Ethinyl darstellt,
$R_A$ ein Rest Methyl ist,
$R'_A$ einen Rest Isopropyl oder lineares Butyl darstellt,
X einen Rest Methylen, Phenylen oder Phenyloxy bedeutet, verbunden mit dem Steroid durch ein Kohlenstoffatom,
Y einen der Reste darstellt, gewählt aus der Gruppe, die gebildet wird durch $-(CH_2)_7-$; $-(CH_2)_8-$; $-(CH_2)_5-C{\equiv}C-$; $-(CH_2)_q-O-CH_2-$ mit q = 5 bis 7 und $-(CH_2)_5-S-CH_2-$, worin das Schwefelatom gegebenenfalls in Form von Sulfoxid oder Sulfon oxidiert ist,
von denen die Namen folgen:

- Monobutandioat von 11beta-{4-[(7-((Butylmethylamlno)-carbonyl)-heptyl)-oxy]-phenyl}-3-hydroxy-estra-1,3,5(10)-trien-17beta-yl,
- Butandioat von 11beta-{4-[(7-((Butylmethylamino)-carbonyl)-heptyl)-oxy]-phenyl}-hydroxy-estra-1,3,5(10)-trien-17beta-yl und Natrium,
- N-Butyl-2-{6-[4-(3,17beta-dihydroxy-estra-1,3,5(10)-trien-11beta-yl)-phenoxy]-hexyloxy}-N-methyl-acetamid,
- N-Butyl-8-[4-(3,17beta-dihydroxy-estra-1,3,5(10)-trien-11beta-yl)-phenoxy]-N-methyl-2-octinamid,
- N-Butyl-2-{[5-(4-(3,17beta-dihydroxy-estra-1,3,5(10)-trien-11beta-yl)-phenyl)-pentyl]-thio}-N-methyl-acetamid,
- N-Butyl-4-(3,17beta-dihydroxy-estra-1,3,5(10)-trien-11beta-yl)-N-methyl-phenylen-nonamid,
- N-Butyl-2-{[5-(4-(3,17beta-dihydroxy-estra-1,3,5(10)-trien-11beta-yl)-phenyl)-pentyl]-oxy}-N-methyl-acetamid,
- 2-{[8-(3,17beta-dihydroxy-19-nor-17alpha-pregna-1,3,5(10)-trien-20-in-11beta-yl)-octyl]-oxy}-N-methyl-N-(1-methylethyl)-acetamid;

und Z eine einfache Bindung darstellt,

<u>oder</u>

n = 1 oder 2 ist,
K ein Sauerstoffatom oder ein Schwefelatom darstellt, $R_{17}$ und $R'_{17}$ bedeuten:

- entweder bilden $R_{17}$ und $R'_{17}$ zusammen eine Ketonfunktion
- oder $R_{17}$ ist ein Rest Hydroxyl oder ein Rest Acyloxy und $R'_{17}$ bedeutet ein Wasserstoffatom, einen Rest Alkyl, Alkenyl oder Alkinyl mit höchstens 8 Kohlenstoffatomen, gegebenenfalls substituiert,

X, Y und Z bedeuten:

- X stellt einen Rest Methylen, eine Gruppe Arylen, einen Rest $CH_2$-O, Arylenoxy oder Arylenthio dar, verbunden mit dem Steroid durch ein Kohlenstoffatom,
- Y stellt eine einfache Bindung oder eine lineare oder verzweigte, gesättigte oder ungesättigte aliphatische Kette mit 1 bis 18 Kohlenstoffatomen dar, gegebenenfalls unterbrochen durch einen oder mehrere Reste, ausgewählt unter den Resten Arylen, Sauerstoff oder Schwefel, gegebenenfalls oxidiert in Form von Sulfoxid oder Sulfon und gegebenenfalls abgeschlossen durch einen Rest Arylen,
- Z stellt eine einfache Bindung dar mit der Maßgabe, daß in dem Fall, wo Y und Z eine einfache Bindung bedeuten, X nicht ein Rest Methylen oder $CH_2$-O sein kann,

$R_A$ oder $R'_{A'}$ gleich oder verschieden, bedeuteten ein Wasserstoffatom, einen linearen oder verzweigten Rest Alkyl mit 1 bis 8 Kohlenstoffatomen, gegebenenfalls substituiert durch einen Rest Aryl, Alkyl oder Dialkylamino, Hydroxy, Halogen oder verestertes Carboxyl, oder $R_A$ und $R'_A$ bilden zusammen mit dem Stickstoffatom, an das sie gebunden sind, einen gesättigten oder ungesättigten Heterocyclus mit 5 oder 6 Ringgliedern, der gegebenenfalls ein oder mehrere Heteroatome umfassen kann, gewählt aus der durch die Atome von Sauerstoff, Stickstoff und Schwefel gebildeten Gruppe und gegebenenfalls substituiert durch einen Rest Alkyl mit 1 bis 4 Kohlenstoffatomen, einen Rest Aryl oder Aralkyl mit 6 bis 10 Kohlenstoffatomen, mit der Maßgabe, daß mindestens einer der Reste $R_A$ oder $R'_A$ kein Wasserstoffatom ist und daß mindestens irgendeine der folgenden Bedingungen erfüllt ist:

- entweder ist n = 2,
- oder K ist ein Schwefelatom,
- oder X ist ein Rest Arylenthio, verbunden mit dem Steroid durch ein Kohlenstoffatom,
- oder $R_A$ und $R'_A$ bilden mit dem Kohlenstoffatom, an das sie gebunden sind, einen Heterocyclus mit 5 oder 6 Ringgliedern, substituiert durch einen Rest Aryl oder Aralkyl,

dadurch gekennzeichnet, daß man

a) zur Herstellung der wie oben definierten Produkte der Formel (I) eine Verbindung der Formel (II)

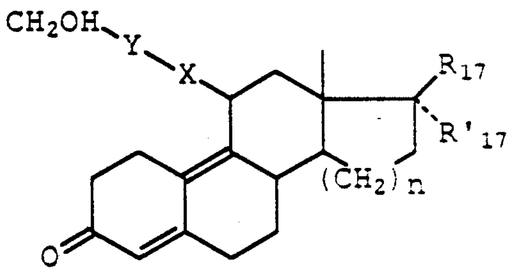

(II)

in der X, Y, n, $R_{17}$ und $R'_{17}$ die gleiche Bedeutung wie oben besitzen, mit der Maßgabe, daß in dem Fall, wo $R_{17}$ einen Rest Hydroxyl darstellt, dieser Rest geschützt ist,
der Einwirkung eines Oxidationsmittels unterzieht, um das Produkt der Formel (III)

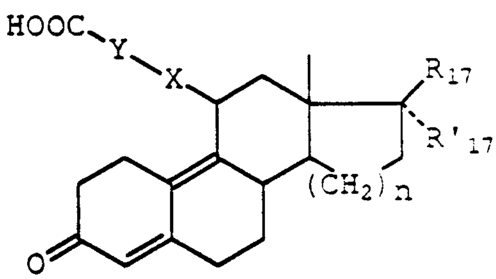

(III)

zu erhalten, das man der Einwirkung eines Mittels unterzieht, das ermöglicht, die Carboxylfunktion zu aktivieren, und man anschließend eine Verbindung der Formel (IV)

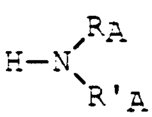

(IV)

einwirken läßt, in der $R_A$ und $R'_A$ die gleiche Bedeutung wie oben besitzen, um das Produkt der Formel (V)

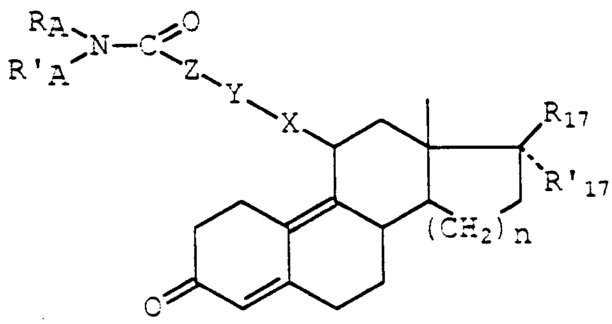

(V)

zu erhalten, in der Z eine einfache Bindung ist, wobei man das Produkt (V), wenn erforderlich, einer Reaktion zur Abspaltung der Schutzgruppe von der Hydroxyfunktion unterzieht und, wenn gewünscht

- einem Reduktionsmittel unterzieht, wenn $R_{17}$ und $R'_{17}$ zusammen eine Ketonfunktion bilden, und man anschließend gegebenenfalls das auf diese Weise erhaltene, in 17 hydroxylierte Derivat der Einwirkung eines Acylierungsmittels unterwirft,
- oder einem Mittel zur Verseifung unterzieht, wenn $R_{17}$ eine Acyloxyfunktion darstellt,

oder die Verbindung der Formel (V) einem Mittel zur Aromatisierung des Ringes A und anschließend einem Mittel zur schonenden Verseifung unterzieht, um die Produkte der Formel (I) zu erhalten, die man, wenn gewünscht, einer oder mehreren der folgenden Reaktionen unterwirft:

entweder, wenn $R_{17}$ und $R'_{17}$ zusammen eine Ketonfunktion bilden, mit einem Reduktionsmittel oder einem Metallkomplex der Formel (VI)

$$M\text{-}R'_{17} \quad \text{(VI)}$$

in der M ein Metallatom darstellt und $R'_{17}$ die gleiche Bedeutung wie oben mit Ausnahme von Wasserstoff besitzt,
oder, wenn $R_{17}$ ein Rest Hydroxyl ist, mit einem selektiven Acylierungsmittel für die Position 17,
oder, wenn $R_A$ oder $R'_A$ ein Wasserstoffatom sind, mit einem geeigneten Mittel zur Alkylierung,
oder, wenn Y eine ungesättigte aliphatische Kette darstellt, mit einem Mittel zur Hydrierung, umsetzt,
um die Produkte der Formel (I) zu erhalten, in der K ein Sauerstoffatom ist, die man, wenn gewünscht, einem Mittel zur Sulfurierung unterzieht, um die Produkte der Formel (I) zu erhalten, in der K ein Schwefelatom ist;

b) zur Herstellung der Produkte der Formel (I), in der X einen Rest Arylen darstellt und Y eine aliphatische Kette bedeutet, die gegebenenfalls mit der Arylengruppe durch eine Doppelbindung oder eine Dreifachbindung verbunden ist und die mindestens 3 Kohlenstoffatome umfaßt, oder die mit der Arylengruppe durch ein Sauerstoffatom oder durch ein Schwefelatom verbunden ist, eine Verbindung der Formel (VII)

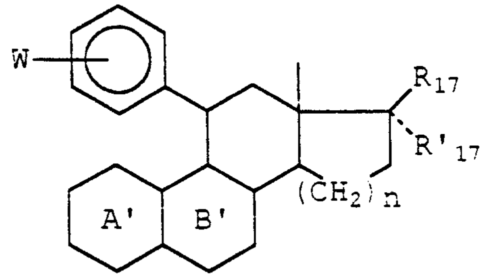

(VII)

in der W entweder einen Rest -OH oder einen gegebenenfalls aktivierten Rest Mercapto oder einen Rest -C≡CH bedeutet sowie n, $R_{17}$ und R' die gleiche Bedeutung wie oben angegeben besitzen, mit der Maßgabe, daß in dem Fall, wo $R_{17}$ einen Rest -OH darstellt, dieser gegebenenfalls geschützt ist,

die Ringe A' und B'

entweder

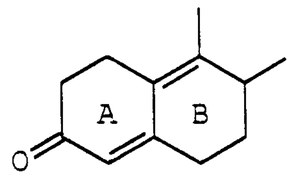

oder

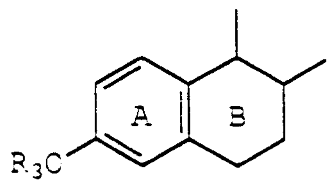

bedeuten, worin $R_3$ ein Wasserstoffatom oder eine Schutzgruppe für die Hydroxyfunktion darstellt,

oder in dem Fall, wo W einen Rest -C≡CH bedeutet, der Einwirkung eines Halogenderivates der Formel (VIII)

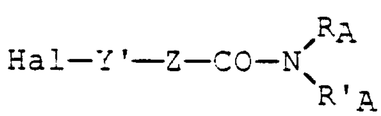

(VIII)

in der Hal ein Halogenatom ist, Z, $R_A$ und $R'_A$ die gleiche Bedeutung wie oben angegeben besitzen und Y' die oben genannte aliphatische Kette Y darstellt, die mindestens 2 Kohlenstoffatome umfaßt, in Anwesenheit einer starken Base und gegebenenfalls der Einwirkung eines Mittels zum Abspalten der Schutzgruppe in 3 und/oder 17 unterzieht, um das Produkt der Formel ($IX_A$)

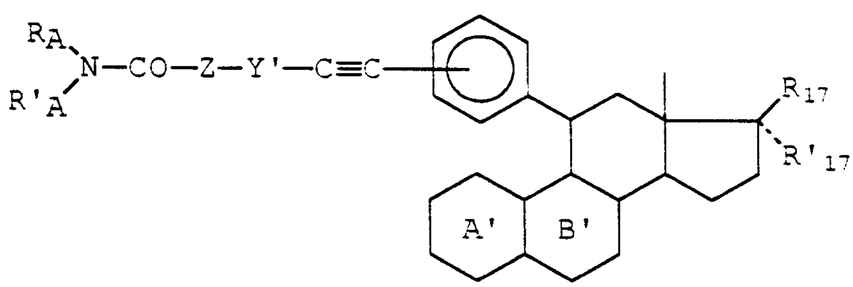

($IX_A$)

zu erhalten, das man, wenn gewünscht, einem Mittel zur teilweisen oder vollständigen Reduktion der Dreifachbindung unterwirft, um das Produkt der Formel ($IX_B$)

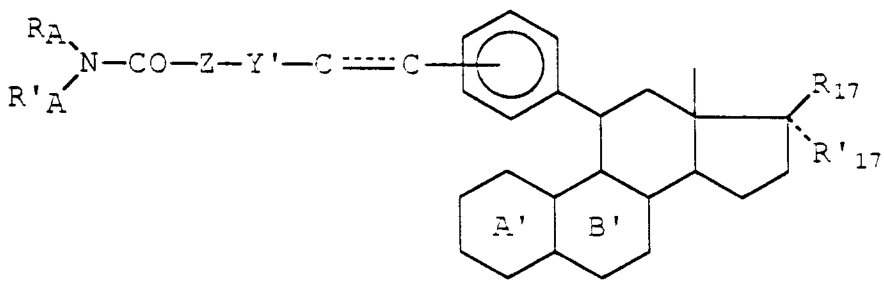

(IX$_B$)

zu erhalten

<u>oder</u>

in dem Fall, wo W einen Rest -OH, einen Rest Mercapto oder einen Rest aktiviertes Mercapto darstellt, der Einwirkung eines Halogenderivates der Formel (X)

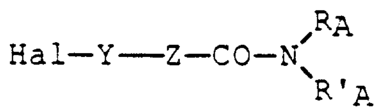

(X)

in der Hai, Y, Z, $R_A$ und $R'_A$ die gleiche Bedeutung wie oben angegeben besitzen, in Anwesenheit eines alkalischen Mittels und anschließend gegebenenfalls der Einwirkung eines Mittels zum Abspalten der Schutzgruppe unterzieht, um ein Produkt der Formel (IX$_C$)

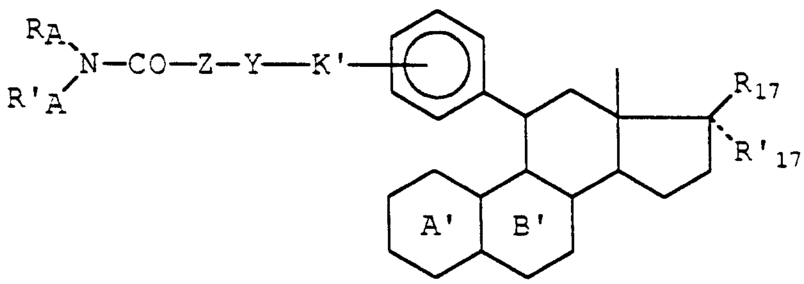

(IX$_C$)

zu erhalten, in der K' entweder ein Sauerstoffatom oder ein Schwefelatom darstellt, wobei man die Produkte der Formeln (IX$_A$), (IX$_B$) oder (IX$_C$) gegebenenfalls einem Mittel zur Aromatisierung des Ringes A und anschließend einem Mittel zur schonenden Verseifung unterzieht, um die Produkte der Formel (I') zu erhalten, die man gegebenenfalls wie oben bei den Produkten der Formel (I) angegeben behandelt.

**2.** Verfahren nach Anspruch 1, dadurch gekennzeichnet, daß man zur Herstellung der wie in Anspruch 1 definierten Produkte der Formel (I) eine Verbindung der Formel (II)

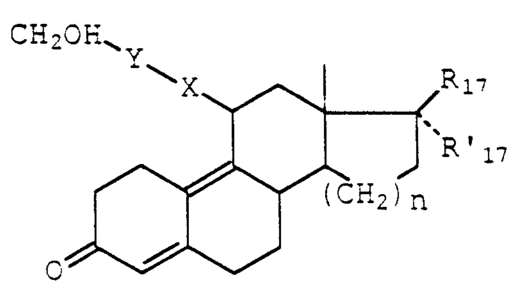

(II)

in der X, Y, n, $R_{17}$ und $R'_{17}$ die gleiche Bedeutung wie in Anspruch 1 besitzen, mit der Maßgabe, daß in dem Fall,

wo $R_{17}$ einen Rest Hydroxyl darstellt, dieser Rest geschützt ist, der Einwirkung eines Oxidationsmittels unterzieht, um das Produkt der Formel (III)

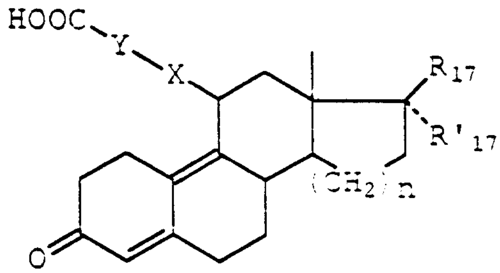

(III)

zu erhalten, das man der Einwirkung eines Mittels unterzieht, das ermöglicht, die Carboxylfunktion zu aktivieren, und man anschließend eine Verbindung der Formel (IV)

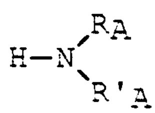

(IV)

einwirken läßt, in der $R_A$ und $R'_A$ die gleiche Bedeutung wie in Anspruch 1 besitzen, um das Produkt der Formel (V)

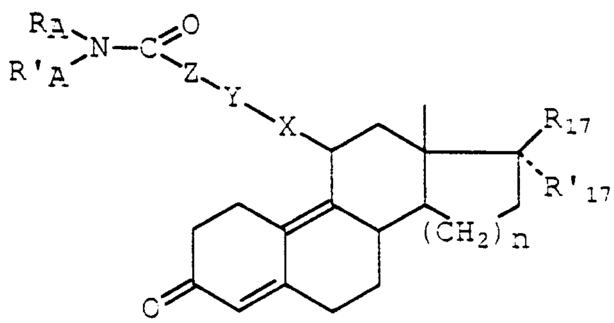

(V)

zu erhalten, in der Z eine einfache Bindung ist, wobei man das Produkt (V), wenn erforderlich, einer Reaktion zur Abspaltung der Schutzgruppe von der Hydroxyfunktion unterzieht und, wenn gewünscht

- einem Reduktionsmittel unterzieht, wenn $R_{17}$ und $R'_{17}$ zusammen eine Ketonfunktion bilden, und man anschließend gegebenenfalls das auf diese Weise enhaltene, in 17 hydroxylierte Derivat der Einwirkung eines Acylierungsmittels unterwirft,
- oder einem Mittel zur Verseifung unterzieht, wenn $R_{17}$ eine Acyloxyfunktion darstellt,

oder die Verbindung der Formel (V) einem Mittel zur Aromatisierung des Ringes A und anschließend einem Mittel zur schonenden Verseifung unterzieht, um die Produkte der Formel (I) zu erhalten, die man, wenn gewünscht, einer oder mehreren der folgenden Reaktionen unterwirft:

entweder, wenn $R_{17}$ und $R'_{17}$ zusammen eine Ketonfunktion bilden, mit einem Reduktionsmittel oder einem Metallkomplex der Formel (VI)

$$M\text{-}R'_{17} \qquad (VI)$$

in der M ein Metallatom darstellt und $R'_{17}$ die gleiche Bedeutung wie in Anspruch 1 mit Ausnahme von Wasserstoff besitzt,
oder, wenn $R_{17}$ ein Rest Hydroxyl ist, mit einem selektiven Acylierungsmittel für die Position 17,
oder, wenn $R_A$ oder $R'_A$ ein Wasserstoffatom sind, mit einem geeigneten Mittel zur Alkylierung,
oder, wenn Y eine ungesättigte aliphatische Kette darstellt, mit einem Mittel zur Hydrierung umsetzt,
um die Produkte der Formel (I) zu erhalten, in der K ein Sauerstoffatom ist, die man, wenn gewünscht, einem

Mittel zur Sulfurierung unterzieht, um die Produkte der Formel (I) zu erhalten, in der K ein Schwefelatom ist.

3. Verfahren nach Anspruch 1, dadurch gekennzeichnet, daß man zur Herstellung der Produkte der Formel (I), in der X einen Rest Arylen darstellt und Y eine aliphatische Kette bedeutet, die gegebenenfalls mit der Arylengruppe durch eine Doppelbindung oder eine Dreifachbindung verbunden ist und die mindestens 3 Kohlenstoffatome umfaßt, oder die mit der Arylengruppe durch ein Sauerstoffatom oder durch ein Schwefelatom verbunden ist, eine Verbindung der Formel (VII)

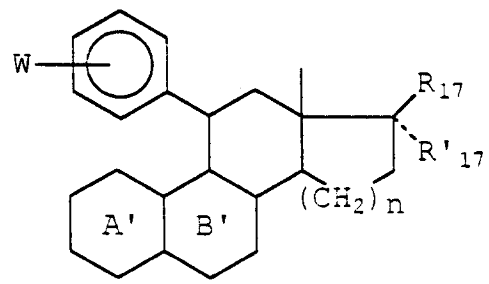

(VII)

in der W entweder einen Rest -OH oder einen gegebenenfalls aktivierten Rest Mercapto oder einen Rest -C≡CH bedeutet sowie n, $R_{17}$ und R' die gleiche Bedeutung wie in Anspruch 1 angegeben besitzen, mit der Maßgabe, daß in dem Fall, wo $R_{17}$ einen Rest -OH darstellt, dieser gegebenenfalls geschützt ist,
die Ringe A' und B'

entweder

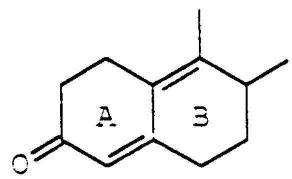

oder

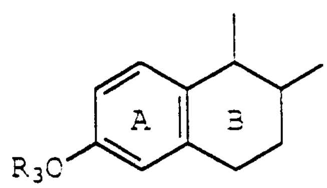

bedeuten, worin $R_3$ ein Wasserstoffatom oder eine Schutzgruppe für die Hydroxyfunktion darstellt,

oder in dem Fall, wo W einen Rest -C≡CH bedeutet, der Einwirkung eines Halogenderivates der Formel (VIII)

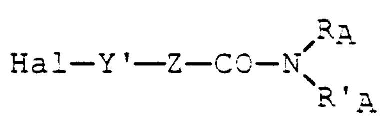

(VIII)

in der Hal ein Halogenatom ist, Z, $R_A$ und $R'_A$ die gleiche Bedeutung wie in Anspruch 1 angegeben besitzen und Y' die oben genannte aliphatische Kette Y darstellt, die mindestens 2 Kohlenstoffatome umfaßt, in Anwesenheit einer starken Base und gegebenenfalls der Einwirkung eines Mittels zum Abspalten der Schutzgruppe in 3 und/oder 17 unterzieht, um das Produkt der Formel ($IX_A$)

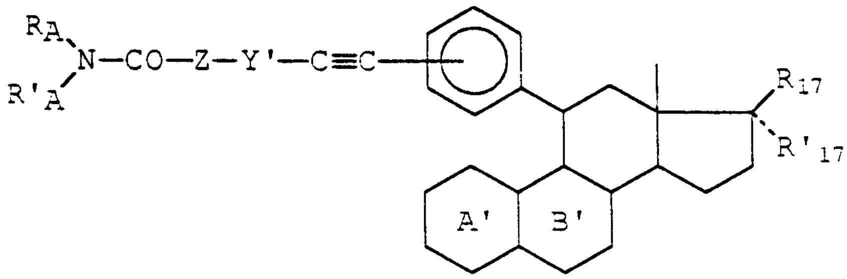

($IX_A$)

zu erhalten, das man, wenn gewünscht, einem Mittel zur teilweisen oder vollständigen Reduktion der Dreifachbindung unterwirft, um das Produkt der Formel ($IX_B$)

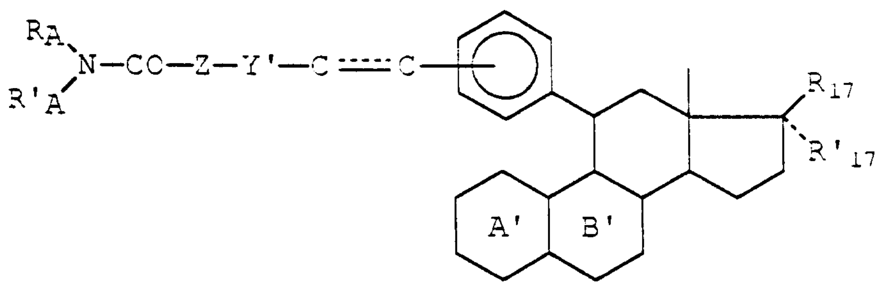

($IX_B$)

zu erhalten

<u>oder</u>

in dem Fall, wo W einen Rest -OH, einen Rest Mercapto oder einen Rest aktiviertes Mercapto darstellt, der Einwirkung eines Halogenderivates der Formel (X)

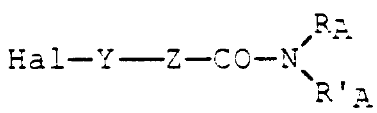

(X)

in der Hal, Y, Z, $R_A$ und $R'_A$ die gleiche Bedeutung wie in Anspruch 1 angegeben besitzen, in Anwesenheit eines alkalischen Mittels und anschließend gegebenenfalls der Einwirkung eines Mittels zum Abspalten der Schutzgruppe unterzieht, um ein Produkt der Formel ($IX_C$)

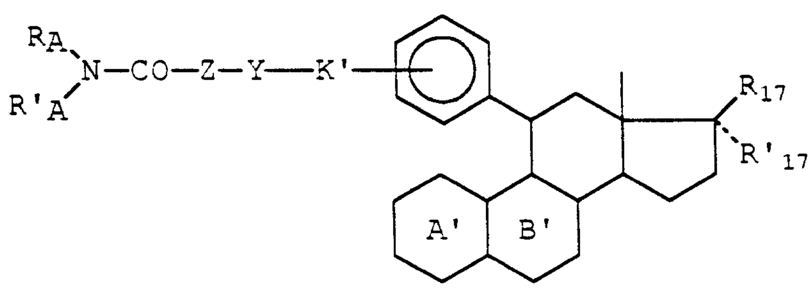

($IX_C$)

zu erhalten, in der K' entweder ein Sauerstoffatom oder ein Schwefelatom darstellt, wobei man die Produkte der Formeln ($IX_A$), ($IX_B$) oder ($IX_C$) gegebenenfalls einem Mittel zur Aromatisierung des Ringes A und anschließend einem Mittel zur schonenden Verseifung unterzieht, um die Produkte der Formel (I') zu erhalten, die man gegebenenfalls wie in Anspruch 1 bei den Produkten der Formel (I) angegeben behandelt.

4. Verfahren nach irgendeinem der Ansprüche 1 bis 3, dadurch gekennzeichnet, daß man ein Ausgangsprodukt der Formeln (II) oder (VII) verwendet, worin n = 1 ist.

5. Verfahren nach Anspruch 4, dadurch gekennzeichnet, daß man ein Ausgangsprodukt der Formel (II) verwendet, worin X ein Rest Arylenoxy ist und Y eine lineare gesättigte Kette mit 5 bis 10 Kohlenstoffatomen bedeutet, gegebenenfalls unterbrochen durch ein Sauerstoffatom, oder ein Produkt der Formel (VII), in der W ein Rest -OH ist und ein Produkt der Formel (X) einsetzt, in der Y die oben angegebene Bedeutung besitzt.

6. Verfahren nach Anspruch 4, dadurch gekennzeichnet, daß man ein Ausgangsprodukt der Formel (II) verwendet, worin X ein Rest Arylenthio ist und Y eine lineare gesättigte Kette mit 5 bis 10 Kohlenstoffatomen bedeutet, oder ein Produkt der Formel (VII), in der W ein gegebenenfalls aktivierter Rest Mercapto ist und ein Produkt der Formel (X) einsetzt, in der Y die oben angegebene Bedeutung besitzt.

7. Verfahren nach Anspruch 4, dadurch gekennzeichnet, daß man ein Ausgangsprodukt der Formel (II), worin X ein Rest Arylen ist und Y eine lineare ungesättigte Kette mit 5 bis 10 Kohlenstoffatomen bedeutet, und ein Produkt der Formel (VII) verwendet, in der $R_A$ und $R'_A$ mit dem Atom, an das sie gebunden sind, ein Piperidin bilden, N-substituiert durch einen Rest Aralkyl, oder ein Produkt der Formel (VII), in der W ein Rest -C≡CH ist, und ein Produkt der Formel (VII) einsetzt, in der $R_A$ und $R'_A$ die oben angegebene Bedeutung besitzen.

8. Verfahren nach Anspruch 1, dadurch gekennzeichnet, daß man die Ausgangsprodukte in der Weise auswählt, daß man irgendeines der Produkte der Formel (I) herstellt, von denen die Namen folgen:

- N-Butyl-5-{[4-(3,17beta-dihydroxy-estra-1,3,5(10)-trien-11beta-yl)-phenoxy]-pentyloxy}-N-methyl-ethanthioamid,
- N-Butyl-8-{4-[3,17beta-dihydroxy-estra-1,3,5(10)-trien-11beta-yl}-phenoxy}-N-methyl-octanthioamid.

9. Verfahren zur Herstellung von pharmazeutischen Zusammensetzungen, dadurch gekennzeichnet, daß man als Wirkstoff mindestens eines der wie in Anspruch 1 definierten Produkte der Formel (I) in eine für diese Verwendung vorgesehene Form bringt.

10. Verfahren zur Herstellung von pharmazeutischen Zusammensetzungen, dadurch gekennzeichnet, daß man als Wirkstoff mindestens eines der nach einem der Ansprüche 4 bis 8 erhaltenen Produkte der Formel (I) in eine für diese Verwendung vorgesehene Form bringt.

11. Als neue Zwischenprodukte die Produkte der Formeln (II) und (III) wie in Anspruch 1 oder 2 definiert, worin n = 2 ist.

12. Als neue Zwischenprodukte die Produkte der Formel (VII) wie in Anspruch 1 oder 3 definiert, worin W einen gegebenenfalls aktivierten Rest Mercapto darstellt.